# EUROPEAN PATENT APPLICATION

(11) **EP 4 119 166 A1**
(43) Date of publication of application: **18.01.2023**
(21) Application number: 21766865.6
(22) Date of filing: 12.03.2021
(51) Int. Cl.: A61K 48/00, A61K 31/7105, A61K 38/46, A61P 35/00

(54) **COMPOSITION FOR INDUCING APOPTOSIS OF CELLS HAVING GENOMIC SEQUENCE VARIATION AND METHOD FOR INDUCING APOPTOSIS OF CELLS BY USING COMPOSITION**

(30) Priority: 12.03.2020 KR 20200030473
(71) Applicant: Institute for Basic Science, Yuseong-gu Daejeon 34126 (KR); Ulsan National Institute of Science and Technology (UNIST), Eonyang-eup Ulju-gun Ulsan 44919 (KR)
(72) Inventor: MYUNG, Kyungjae, Ulsan 44667 (KR); KWON, Taejoon, Ulju-gun Ulsan 44920 (KR); BAEK, In-Joon, Ulju-gun Ulsan 44919 (KR); RA, Jae, Sun, Ulju-gun Ulsan 44928 (KR); PARK, Yong, Hwan, Seoul 04769 (KR); JUNG, Woojae, Seoul 08228 (KR); OTARBAYEV, Daniyar, Ulju-gun Ulsan 44919 (KR); CHO, Seungwoo, Ulju-gun Ulsan 44919 (KR)
(74) Representative: Mewburn Ellis LLP
(86) International application number: PCT/KR2021/003109
(87) International publication number: WO 2021/182917

(57) **Abstract**

The present invention relates to at least one delivery vehicle comprising: either a plurality of cleavaging agents that specifically recognize a plurality of nucleic acid sequences including unique sequences, which do not exist in normal cells, among variant region sequences specific for cells having genomic sequence variation in the cells having genomic sequence variation, or polynucleotides encoding the cleavaging agents; and a nuclease or a polynucleotide encoding the nuclease. The present invention also relates to a composition containing the delivery vehicle, and a method of inducing apoptosis of cells having genomic sequence variation by using the delivery vehicle or the composition.

## Description

### Technical Field

The present invention relates to at least one delivery vehicle comprising: either a plurality of cleavaging agents that specifically recognize a plurality of nucleic acid sequences including unique sequences, which do not exist in normal cells, among cell-specific variant region sequences in cells having genomic sequence variation, or polynucleotides encoding the cleavaging agents; and a nuclease or a polynucleotide encoding the nuclease. The present invention also relates to a composition containing the delivery vehicle, and a method of inducing apoptosis of cells having genomic sequence variation using the delivery vehicle or the composition.

### Background Art

CRISPR/Cas is an RNA-guided gene-editing tool that may use a bacterially derived endonuclease Cas9 (or its mutant nickase) and a guide RNA to introduce a double (or single)-strand break at a specific location within the genome by matching the sequences between the guide RNA and genomic DNA. CRISPR/Cas-mediated gene knockout is expected to be more efficient than RNA interference-mediated gene knockdown, and has provided a useful laboratory tool to study gene function

Studies have reported that the CRISPR-Cas system can work in mammalian cells, and the gene editing technology based on microbial adaptive immunity includes Cas9 (CRISPR associated protein 9: RNA-guided DNA endonuclease enzyme) and a guide RNA (gRNA). The guide RNA includes crRNA (CRISPR RNA) and tracrRNA (trans-activating crRNA). It binds to Cas9 and guides the Cas9 to a desired genomic sequence through base pairing to a target sequence to produce a double strand break (DSB).

Living organisms undergo various types of cell division during life, and this cell division enables the genomic sequence to remain as constant as possible through various repair processes, but variations in the genomic sequence occur in some cases. Cells having variations in the genomic sequence threaten or adversely affect the survival of living organisms. Among these genomic sequence variations, cancer is the most serious issue.

Cancer is caused by the accumulation of mutations, which are either inherited through germline cells or acquired in somatic cells during the cell cycle. Such changes in oncogenes, tumor suppressor genes, and DNA repair genes also cause cells to deviate from the growth and control mechanisms and cause cancer.

Meanwhile, in order to treat cancer, which is a representative disease caused by cells having genomic sequence variation, various anticancer drugs have been continuously developed. Anticancer drugs have been developed as first-generation chemotherapeutic anticancer drugs that were first developed in the 1970s, second-generation targeted anticancer drugs that emerged in the 2000s, and third-generation immunotherapeutic anticancer drugs that have been applied since the 2010s (Nature reviews Cancer. 5(1):65-72.).

First-generation chemotherapeutic anticancer drugs are anticancer compounds, such as Paclitaxel, which attack cancer cells that divide faster than normal cells. However, these chemotherapeutic anticancer drugs have problems in that they destroy the patient's immune system by attacking not only cancer cells but also normal cells, and cause various side effects such as hair loss, vomiting, loss of appetite, fatigue, and severe physical weakness, due to their strong toxicity (Critical reviews in oncology/hematology 89(1):43-61., and Chemistry & Biology Volume 20, Issue 5, 23 May 2013, Pages 648-659).

To solve these problems, targeted anticancer drugs such as rituximab or imatinib, which selectively attack only tumor cells with a specific mutation, have emerged as cancer therapeutic agents. These targeted anticancer drugs have fewer side effects such as hair loss and vomiting and are confirmed to achieve a high treatment response rate, but have problems in that they cannot treat various cancers because they can be used only for patients with specific cancer-causing mutations, and resistance to the targeted anticancer drugs occurs (Adv Pharm Bull. 2017 Sep; 7(3): 339-348.).

Recently, attention has been focused on immunotherapeutic anticancer drugs such as Ipilimumab or Tisagenlecleucel that attack and remove tumor cells through activation of immune cells, unlike other anticancer drugs that attack tumors themselves (Cytotherapy. 2019 Feb;21(2):224-233). These immunotherapeutic anticancer drugs have few side effects and their efficacy can be maintained for a long time in patients who show a therapeutic response. However, it is known that they are effective only for specific patients (a response rate of about 20 to 30%), problems associated with resistance to immune checkpoint inhibitors arise, and immune cell therapy products or the like cause side effects such as cytokine secretion syndrome and neurotoxicity (Nat Rev Drug Discov. 2018 Nov 28;17 (12) :854-855) .

Despite the many anticancer drugs being used for treatment as described above, desired anticancer effects are not sufficiently achieved or the anticancer drugs have side effects. Thus, the development and application of new cancer treatment technology is still required.

Regarding one of new cancer treatment technologies, insertion (IN) of a new DNA sequence, which is not found in normal cells, or deletion (DEL) of a portion of normal cell DNA, is observed in cancer. Since this specific insertion or deletion DNA (indel) that appears in cancer cell DNA is a DNA sequence that does not exist in normal cells, cancer can be treated by setting this indel as an attack target that distinguishes cancer cells from normal cells.

DNA double-strand break is one of the most serious types of damage at the cellular level. Damaged DNA is repaired by non-homologous end joining and homologous recombination, but unrepaired DNA can result in loss or rearrangement of genetic information, leading to apoptosis.

It has been reported that the CRISPR/Cas system can be applied to human cancer treatment (Oncotarget. 2016 Mar 15;7(11):12305-17). However, this merely suggests that, based on mutations correlated with cancer development, modifying or excising one or more genes may increase the likelihood of providing a robust treatment for cancer.

In this regard, U.S. Patent Publication No. 2017/0114413 proposes a CRISPR/Cas system employing a guide RNA that targets cancer-specific sequence variation. It describes that the cancer-specific sequence variation may be a cancer-specific DNA copy number variation (CNV). It is a technology that can treat cancers showing certain CNVs or more by applying the CRISPR/Cas system, and the types of cancers that can be treated by this technology are extremely limited.

Korean Patent No. 2023577 discloses a composition for killing tumor cells containing: a polynucleotide that complementarily binds to a gene having at least 7 CNVs, which is present specifically in cancer cells; and a fusion protein in which Cas9 and RecJ, an exonuclease, are linked together. According to the present invention, ExoI, an exonuclease, is used in addition to Cas9, and thus it is possible to exhibit a desired cancer therapeutic effect by minimizing the number of guide RNAs. However, when RecJ was used as an exonuclease, it was not possible to reduce or minimize the number of administered guide RNAs to a desired extent.

Under this technical background, the present inventors have found that cells having genomic sequence variation can be killed by inducing a double strand break (DSB) in a plurality of variant regions by using a plurality of cleavaging agents that specifically recognize the plurality of variant regions in the cells having genomic sequence variation, thereby completing the present invention.

### Summary of the Invention

An object of the present invention is to provide a composition for inducing apoptosis of cells having genomic sequence variation, the composition containing: a nucleic acid-cleavage enzyme or a polynucleotide encoding the nucleic acid-cleavage enzyme; and a plurality of cleavaging agents that respectively recognize a plurality of variant regions in the cells having genomic acid variation, or polynucleotides encoding the plurality of cleavaging agents.

Another object of the present invention is to provide a vector for inducing apoptosis of cells having genomic sequence variation, the vector containing: a polynucleotide encoding a nucleic acid-cleavage enzyme; and a plurality of cleavaging agents that respectively recognize a plurality of variant regions in the cells having genomic acid variation, or polynucleotides encoding the plurality of cleavaging agents.

Still another object of the present invention is to provide a ribonucleoprotein (RNP) complex for inducing apoptosis of cells having genomic sequence variation, the RNP complex comprising: a nucleic acid-cleavage enzyme; and a plurality of cleavaging agents that respectively recognize a plurality of variant regions in the cells having genomic acid variation, or polynucleotides encoding the plurality of cleavaging agents.

Yet another object of the present invention is to provide a carrier for inducing apoptosis of cells having genomic sequence variation, the carrier comprising: a nucleic acid-cleavage enzyme or a polynucleotide encoding the nucleic acid-cleavage enzyme; and a plurality of cleavaging agents that respectively recognize a plurality of variant regions in the cells having genomic sequence variation, or polynucleotides encoding the plurality of cleavaging agents.

Still yet another object of the present invention is to provide an exosome for inducing apoptosis of cells having genomic sequence variation, the exosome comprising: a nucleic acid-cleavage enzyme or a polynucleotide encoding the nucleic acid-cleavage enzyme; and a plurality of cleavaging agents that respectively recognize a plurality of variant regions in the cells having genomic acid variation, or polynucleotides encoding the plurality of cleavaging agents.

A further object of the present invention is to provide a composition for inducing apoptosis of cells having genomic sequence variation, the composition containing the vector.

Further another object of the present invention is to provide a composition for inducing apoptosis of cells having genomic sequence variation, the composition containing the ribonucleoprotein (RNP) complex.

Further still another object of the present invention is to provide a composition for inducing apoptosis of cells having genomic sequence variation, the composition containing the carrier.

Further yet another object of the present invention is to provide a composition for inducing apoptosis of cells having genomic sequence variation, the composition containing the exosome.

Another object of the present invention is to provide a composition for inducing apoptosis of cells having genomic sequence variation, the composition containing: a nuclease in which endonuclease and exonuclease I are fused together, or a polynucleotide encoding the nuclease; and a plurality of cleavaging agents that respectively recognize a plurality of variant regions in the cells having genomic acid variation, or polynucleotides encoding the plurality of cleavaging agents.

Still another object of the present invention is to provide a vector for inducing apoptosis of cells having genomic sequence variation, the vector comprising: a polynucleotide encoding a nuclease in which endonuclease and exonuclease I are fused together; and a plurality of cleavaging agents that respectively recognize a plurality of variant regions in the cells having genomic sequence variation, or polynucleotides encoding the plurality of cleavaging agents.

Yet another object of the present invention is to provide a ribonucleoprotein (RNP) complex for inducing apoptosis of cells having genomic sequence variation, the RNP complex comprising: a nuclease in which endonuclease and exonuclease I are fused together; and a plurality of cleavaging agents that respectively recognize a plurality of variant regions in the cells having genomic acid variation, or polynucleotides encoding the plurality of cleavaging agents.

Still yet another object of the present invention is to provide a carrier for inducing apoptosis of cells having genomic sequence variation, the carrier containing: a nuclease in which endonuclease and exonuclease I are fused together, or a polynucleotide encoding the nuclease; and a plurality of cleavaging agents that respectively recognize a plurality of variant regions in the cells having genomic sequence variation, or polynucleotides encoding the plurality of cleavaging agents.

A further object of the present invention is to provide an exosome for inducing apoptosis of cells having genomic sequence variation, the exosome containing: a nuclease in which endonuclease and exonuclease I are fused together, or a polynucleotide encoding the nuclease; and a plurality of cleavaging agents that respectively recognize a plurality of variant regions in the cells having genomic sequence variation, or polynucleotides encoding the plurality of cleavaging agents.

Further another object of the present invention is to provide a composition for inducing apoptosis of cells having genomic sequence variation, the composition containing the vector.

Further still another object of the present invention is to provide a composition for inducing apoptosis of cells having genomic sequence variation, the composition containing the ribonucleoprotein (RNP) complex.

Further yet another object of the present invention is to provide a composition for inducing apoptosis of cells having genomic sequence variation, the composition containing the carrier.

Another object of the present invention is to provide a composition for inducing apoptosis of cells having genomic sequence variation, the composition containing the exosome.

Still another object of the present invention is to provide a method for inducing apoptosis of cells having genomic sequence variation, the method comprising a step of providing the composition, vector, ribonucleoprotein (RNP) complex, carrier or exosome for inducing apoptosis of cells having genomic sequence variation.

To achieve the above objects, the present invention provides a composition for inducing apoptosis of cells having genomic sequence variation, the composition containing: (i) a nuclease or a polynucleotide encoding the nuclease; and (ii) cleavaging agents that respectively recognize at least 4 variant regions specific for the cells having genomic sequence variation in the cells having genomic sequence variation, or polynucleotides encoding the cleavaging agents.

The present invention is directed to a method for inducing apoptosis of cells having genomic sequence variation, the method comprising treating a subject with: (i) a nuclease or a polynucleotide encoding the nuclease; and (ii) cleavaging agents that respectively recognize at least 4 variant regions specific for the cells having genomic sequence variation in the cells having genomic sequence variation, or polynucleotides encoding the cleavaging agents.

The present invention also provides a vector for inducing apoptosis of cells having genomic sequence variation, the vector comprising: (i) a polynucleotide encoding a nuclease; and (ii) cleavaging agents that respectively recognize at least 4 variant regions specific for the cells having genomic sequence variation in the cells having genomic sequence variation, or polynucleotides encoding the cleavaging agents.

The present invention also provides a ribonucleoprotein complex for inducing apoptosis of cells having genomic sequence variation, the ribonucleoprotein complex comprising: (i) a nuclease; and (ii) cleavaging agents that respectively recognize at least 4 variant regions specific for the cells having genomic sequence variation in the cells having genomic sequence variation, or polynucleotides encoding the cleavaging agents.

The present invention also provides a carrier for inducing apoptosis of cells having genomic sequence variation, the carrier containing: (i) a nuclease or a polynucleotide encoding the nuclease; and (ii) cleavaging agents that respectively recognize at least 4 variant regions specific for the cells having genomic sequence variation in the cells having genomic sequence variation, or polynucleotides encoding the cleavaging agents.

The present invention also provides an exosome for inducing apoptosis of cells having genomic sequence variation, the exosome containing: (i) a nuclease or a polynucleotide encoding the nuclease; and (ii) cleavaging agents that respectively recognize at least 4 variant regions specific for the cells having genomic sequence variation in the cells having genomic sequence variation, or polynucleotides encoding the cleavaging agents.

The present invention also provides a composition for inducing apoptosis of cells having genomic sequence variation, the composition containing: (i) a polynucleotide encoding a nuclease; and (ii) cleavaging agents that respectively recognize at least 4 variant regions specific for the cells having genomic sequence variation in the cells having genomic sequence variation, or polynucleotides encoding the cleavaging agents, wherein (i) and (ii) are contained in the same vector or different vectors.

The present invention also provides a composition for inducing apoptosis of cells having genomic sequence variation, the composition containing: (i) a nuclease or a polynucleotide encoding the nuclease; and (ii) cleavaging agents that respectively recognize at least 4 variant regions specific for the cells having genomic sequence variation in the cells having genomic sequence variation, or polynucleotides encoding the cleavaging agents, wherein (ii) is contained in a vector.

The present invention also provides a composition for inducing apoptosis of cells having genomic sequence variation, the composition containing: (i) a nuclease; and (ii) cleavaging agents that respectively recognize at least 4 variant regions specific for the cells having genomic sequence variation in the cells having genomic sequence variation, or polynucleotides encoding the cleavaging agents, wherein (i) and (ii) form a ribonucleoprotein complex.

The present invention also provides a composition for inducing apoptosis of cells having genomic sequence variation, the composition containing: (i) a nuclease or a polynucleotide encoding the nuclease; and (ii) cleavaging agents that respectively recognize at least 4 variant regions specific for the cells having genomic sequence variation in the cells having genomic sequence variation, or polynucleotides encoding the cleavaging agents, wherein (i) and (ii) are contained in a carrier.

The present invention also provides an exosome-containing composition for inducing apoptosis of cells having genomic sequence variation, the composition containing: (i) a nuclease or a polynucleotide encoding the nuclease; and (ii) cleavaging agents that respectively recognize at least 4 variant regions specific for the cells having genomic sequence variation in the cells having genomic sequence variation, or polynucleotides encoding the cleavaging agents.

The present invention also provides a patient-specific composition for treating disease, the composition containing: (i) a nuclease or a polynucleotide encoding the nuclease; and (ii) cleavaging agents that respectively recognize at least 4 variant regions specific for the cells having genomic sequence variation in the cells having genomic sequence variation, or polynucleotides encoding the cleavaging agents.

The present invention also provides a composition for inducing apoptosis of cells having genomic sequence variation, the composition containing: (i) a nuclease in which endonuclease and exonuclease I are fused together, or a polynucleotide encoding the nuclease; and (ii) cleavaging agents that respectively recognize at least 2 variant regions specific for the cells having genomic sequence variation in the cells having genomic sequence variation, or polynucleotides encoding the cleavaging agents. The present invention also provides a method for inducing apoptosis of cells having genomic sequence variation, the method comprising a step of treating a subject with: (i) a nuclease in which endonuclease and exonuclease I are fused together, or a polynucleotide encoding the nuclease; and (ii) cleavaging agents that respectively recognize at least 2 variant regions specific for the cells having genomic sequence variation in the cells having genomic sequence variation, or polynucleotides encoding the cleavaging agents.

The present invention also provides a vector for inducing apoptosis of cells having genomic sequence variation, the vector comprising: (i) a polynucleotide encoding a nuclease in which endonuclease and exonuclease I are fused together; and (ii) cleavaging agents that respectively recognize at least 2 variant regions specific for the cells having genomic sequence variation in the cells having genomic sequence variation, or polynucleotides encoding the cleavaging agents.

The present invention also provides a ribonucleoprotein complex for inducing apoptosis of cells having genomic sequence variation, the complex comprising: (i) a nuclease in which endonuclease and exonuclease I are fused together; and (ii) cleavaging agents that respectively recognize at least 2 variant regions specific for the cells having genomic sequence variation in the cells having genomic sequence variation, or polynucleotides encoding the cleavaging agents.

The present invention also provides a carrier for inducing apoptosis of cells having genomic sequence variation, the carrier comprising: (i) a nuclease in which endonuclease and exonuclease I are fused together, or a polynucleotide encoding the nuclease; and (ii) cleavaging agents that respectively recognize at least 2 variant regions specific for the cells having genomic sequence variation in the cells having genomic sequence variation, or polynucleotides encoding the cleavaging agents.

The present invention also provides an exosome for inducing apoptosis of cells having genomic sequence variation, the exosome comprising: (i) a nuclease in which endonuclease and exonuclease I are fused together, or a polynucleotide encoding the nuclease; and (ii) cleavaging agents that respectively recognize at least 2 variant regions specific for the cells having genomic sequence variation in the cells having genomic sequence variation, or polynucleotides encoding the cleavaging agents.

The present invention also provides a composition for inducing apoptosis of cells having genomic sequence variation, the composition containing: (i) a polynucleotide encoding a nuclease in which endonuclease and exonuclease I are fused together; and (ii) cleavaging agents that respectively recognize at least 2 variant regions specific for the cells having genomic sequence variation in the cells having genomic sequence variation, or polynucleotides encoding the cleavaging agents, wherein (i) and (ii) are contained in the same vector or different vectors.

The present invention also provides a composition for inducing apoptosis of cells having genomic sequence variation, the composition containing: (i) a nuclease in which endonuclease and exonuclease I are fused together, or a polynucleotide encoding the nuclease; and (ii) cleavaging agents that respectively recognize at least 2 variant regions specific for the cells having genomic sequence variation in the cells having genomic sequence variation, or polynucleotides encoding the cleavaging agents, wherein (ii) is contained in a vector.

The present invention also provides a composition for inducing apoptosis of cells having genomic sequence variation, the composition containing: (i) a nuclease in which endonuclease and exonuclease I are fused together; and (ii) cleavaging agents that respectively recognize at least 2 variant regions specific for the cells having genomic sequence variation in the cells having genomic sequence variation, or polynucleotides encoding the cleavaging agents, wherein (i) and (ii) form a ribonucleoprotein complex.

The present invention also provides a composition for inducing apoptosis of cells having genomic sequence variation, the composition containing: (i) a nuclease in which endonuclease and exonuclease I are fused together, or a polynucleotide encoding the nuclease; and (ii) cleavaging agents that respectively recognize at least 2 variant regions specific for the cells having genomic sequence variation in the cells having genomic sequence variation, or polynucleotides encoding the cleavaging agents, wherein (i) and (ii) are contained in a carrier.

The present invention also provides a composition for inducing apoptosis of cells having genomic sequence variation, the composition containing: (i) a nuclease in which endonuclease and exonuclease I are fused together, or a polynucleotide encoding the nuclease; and (ii) cleavaging agents that respectively recognize at least 2 variant regions specific for the cells having genomic sequence variation in the cells having genomic sequence variation, or polynucleotides encoding the cleavaging agents, wherein (i) and (ii) is contained in an exosome.

The present invention also provides a patient-specific composition for treating disease, the composition containing: (i) a nuclease in which endonuclease and exonuclease I are fused together, or a polynucleotide encoding the nuclease; and (ii) cleavaging agents that respectively recognize at least 2 variant regions specific for the cells having genomic sequence variation in the cells having genomic sequence variation, or polynucleotides encoding the cleavaging agents.

### Brief Description of Drawings

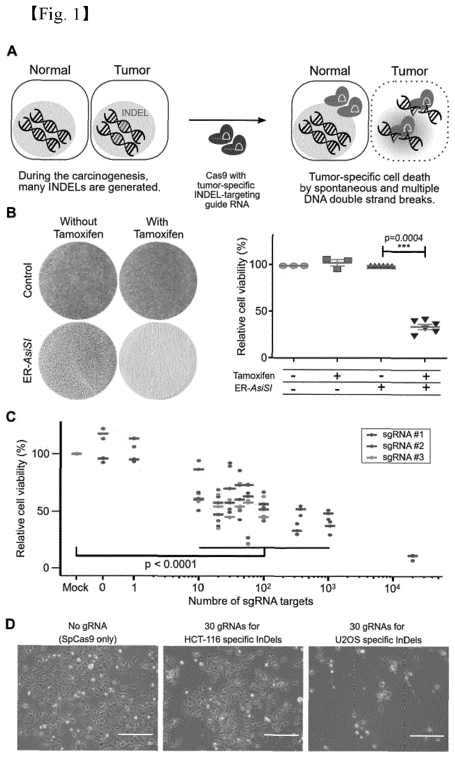

FIG. 1 shows results indicating that CINDELA induces selective apoptosis in cancer cells. (A) A schematic view of CINDELA (cancer specific indel attacker). (B) Simultaneous multiple DNA DSBs (generated with the restriction enzyme AsiSI) induced cell death in osteosarcoma cells (U2OS). (C) DSBs caused by CRISPR-Cas9 induce cell death. Tested sgRNAs targeting 1 to more than 10⁴ sites, and measured the viability of HEK293T cells. (D) CINDELA-induced cancer cell death due to the SpCas9 RNP complex. Scale bar is 300 µm. P-values were calculated using an unpaired two-sided t-test.

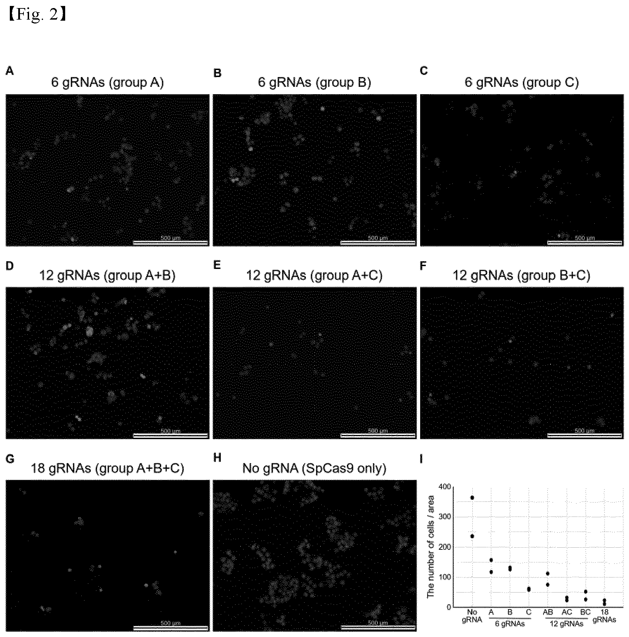

FIG. 2 shows results indicating a stepwise increase in cell death due to simultaneous DNA DSBs induced by CINDELA gRNAs delivered by the RNP complex. Treatment of 18 gRNAs targeting indels specific for HCT116 cells with ATTO-labeled tracer RNA. The successful delivery of the RNP complex was confirmed by detecting the ATTO signal (red). Quantification of live cells in specific regions to confirm the effect of induced DNA DSBs. Delivery of 6 (A to C) 12 (D to F) and 18 (G) gRNAs and tracer RNAs together with SpCas9 to HCT116 cells. (I) Cell viability quantified with each indicated CINDELA treatment.

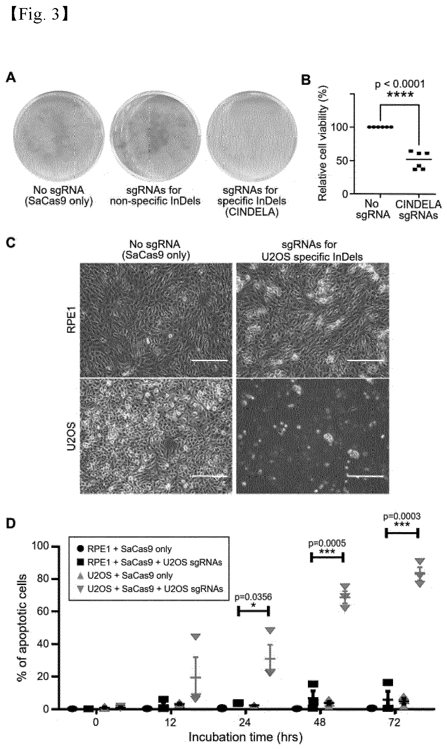

FIG. 3 shows the results for CINDELA including AAV-derived CRISPR/SaCas9. CINDELA involving transfection with the SaCas9 and sgRNA expression plasmids killed U2OS cells. Cells expressing CINDELA sgRNA specific to U2OS indels showed increased cell death compared to cells expressing 30 nonspecific sgRNAs (sgRNA targeting HCT116 specific indels). (B) Relative cell viability was monitored after transfection with the SaCas9 and CINDELA sgRNA plasmids. Significance was assessed using an unpaired two-sided t-test. (C) Cell viability was observed 72 hours after transduction of sgRNA- and SaCas9-expressing AAV into U2OS. As a control, the same AAV was applied to RPE1 cells and AAV expressing only SaCas9 was transduced into U2OS cells. Scale bar is 300 µm. (D) Apoptosis increased in a time-dependent manner after AAV transduction. Cell line-specific targeting significantly increased (p-values were calculated using an unpaired two-sided t-test).

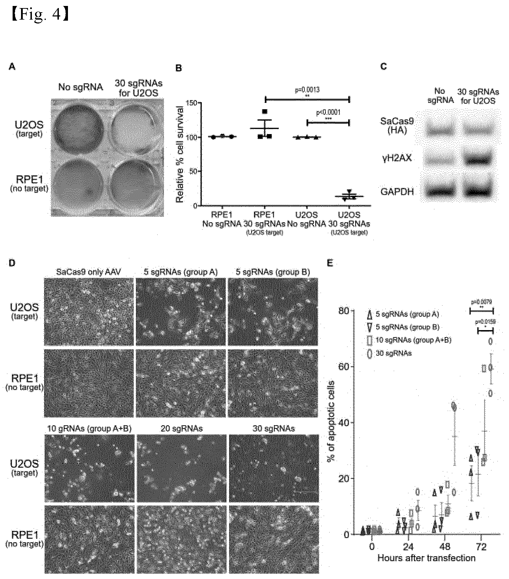

FIG. 4 shows results indicating a stepwise increase in apoptosis caused by the simultaneous DNA DSBs induced by CINDELA sgRNAs delivered by a plasmid. (A) U2OS and RPE1 cells were transduced with AAV containing SaCas9 only (no sgRNA) or AAV expressing one of 30 sgRNA-containing SaCas9 targeting a sequence specific for U2OS cells. (B) Quantification of cell viability observed in (A). 1% bromophenol blue stain in 70% EtOH was extracted from each well with 30% acetate and OD₅₉₅ was measured. (C) Chromatin binding proteins in U2OS cells transduced with AAV expressing SaCas9 only (no sgRNA) or AAV expressing one of 30 sgRNA-containing SaCas9s targeting sequences specific for U2OS cells were immunoblotted with the indicated antibodies. (D) U2OS and RPE1 cell images were captured 3 days after transduction with AAV expressing SaCas9 only or SaCas9 with 5 sgRNAs (groups A and B), 10 sgRNAs, 20 sgRNAs or 30 sgRNAs. (E) Quantification of apoptotic cell death observed in (D).

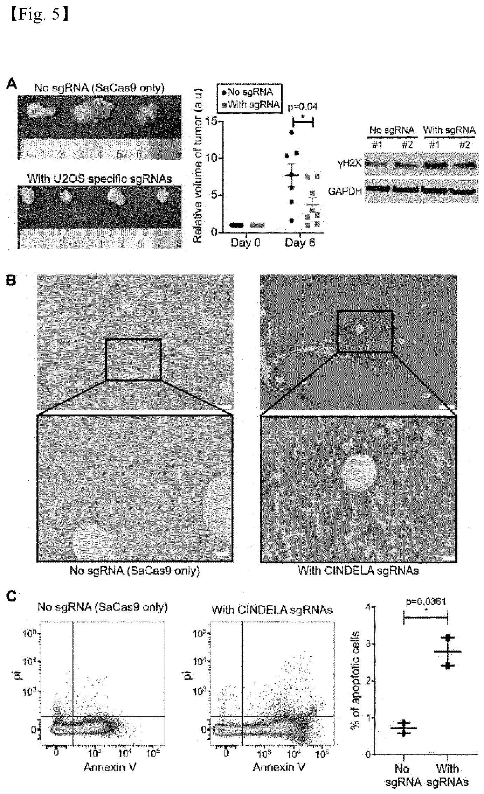

FIG. 5 shows results indicating the effect of CINDELA in a mouse xenograft model. A mouse xenograft model was established with the U2OS osteosarcoma cell line, and 30 CINDELA sgRNAs were delivered together with SaCas9 using AAV. (A) Tumor proliferation was significantly delayed in tumors transduced with AAV expressing U2OS-specific CINDELA sgRNA and SaCas9 compared to xenograft model tumors transduced with AAV expressing only SaCas9. Relative tumor volume differences were analyzed using an unpaired two-sided t-test. (B) Apoptosis increased in xenograft model tumor samples treated with CINDELA sgRNA and SaCas9 compared to tumors treated with SaCas9 alone. 6 days after AAV injection, xenograft model tissue was collected and apoptosis was measured through TUNEL analysis (scale bar: 100 µm top panel; 20 µm bottom panel). (C) Apoptotic cell death induced by CINDELA was confirmed by measuring Annexin V expression through FACS analysis (p-values were calculated using unpaired two-sided t-test).

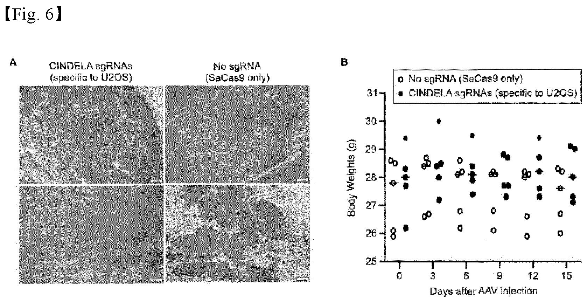

FIG. 6 shows the effect of CINDELA in U2OS-derived xenograft model tissue. (A) Expression of SaCas9 in CINDELA tumors was detected by immunostaining using an anti-HA (tagged to SaCas9) antibody. (B) The body weight of the mice was measured during AAV injection. No sgRNA (containing SaCas9 only) and CINDELA sgRNA (U2OS specific) indicate injection of either SaCas9 alone or AAV expressing one of 30 sgRNA-containing SaCas9 targeting sequences specific for U2OS cells.

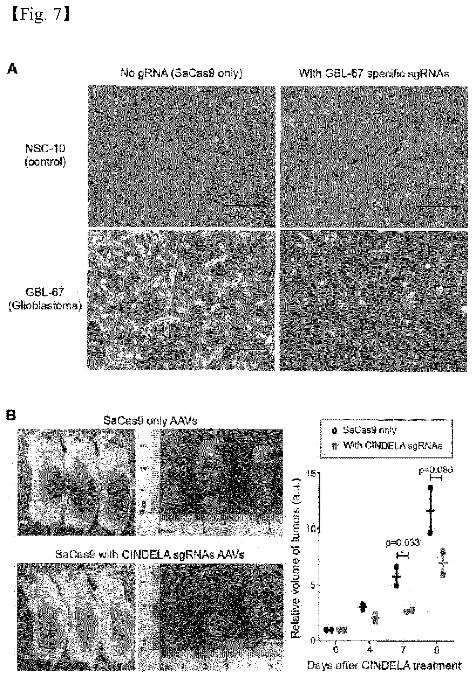

FIG. 7 shows the effect of CINDELA in patient-derived tumor cells and PDX (patient-derived xenograft) model. (A) Tumor cells from a glioblastoma patient (GBL-67) were treated with 26 CINDELA sgRNAs and SaCas9 using AAV transduction. Compared to the neural stem cell line (NSC-10), CINDELA treatment specifically killed GBL-67 cells. Scale bar is 300 µm. (B) Lung cancer PDX model was treated with CINDELA sgRNAs and SaCas9. Interestingly, tumor growth was significantly delayed in treatment with CINDELA sgRNA and SaCas9 compared with a control containing only SaCas9 (significance was assessed using a two-sided unpaired t-test).

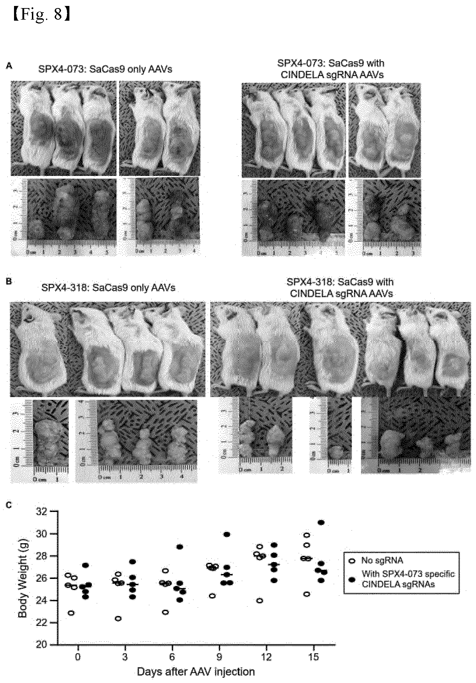

FIG. 8 shows that the growth of lung cancer PDX is inhibited by CINDELA treatment. (A) AAV expressing either SaCas9 only or one of SaCas9 containing 29 sgRNAs targeting sequences specific for PDX-073 cancer was injected 5 times at 3-day intervals for 2 weeks. Photographs of tumor-bearing mice (top panel) and tumors extracted from mice (bottom panel). (B) AAV expressing either SaCas9 alone or one of SaCas9 containing 22 sgRNAs targeting sequences specific for PDX-318 cancer was injected 5 times at 3-day intervals for 2 weeks. Photographs of tumor-bearing mice (top panel) and tumors extracted from mice (bottom panel). (C) The body weight of the mice was measured during AAV injection.

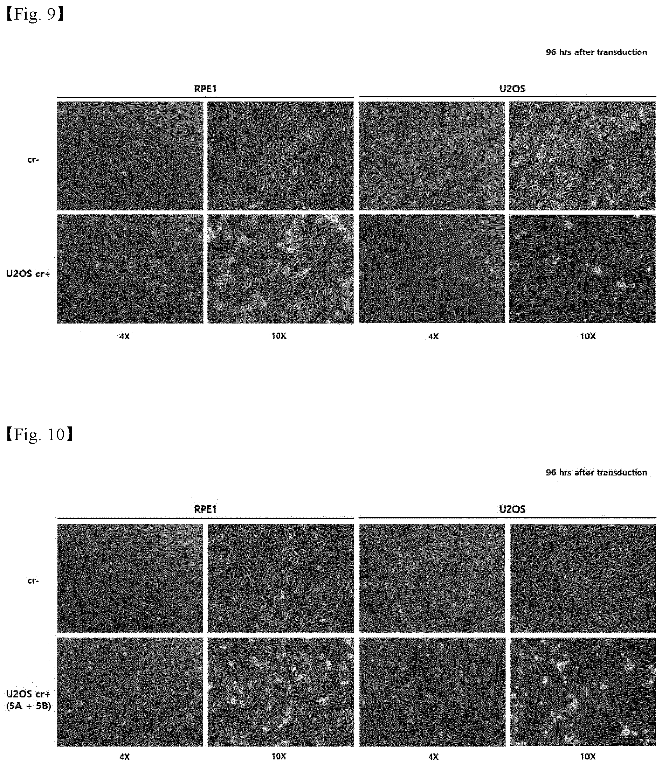

FIG. 9 shows the results of confirming whether it is possible to induce apoptosis by targeting 30 DNA sequences in the U2OS cell line.
FIG. 10 shows the results of confirming whether it is possible to induce apoptosis by targeting 10 DNA sequences in the U2OS cell line.

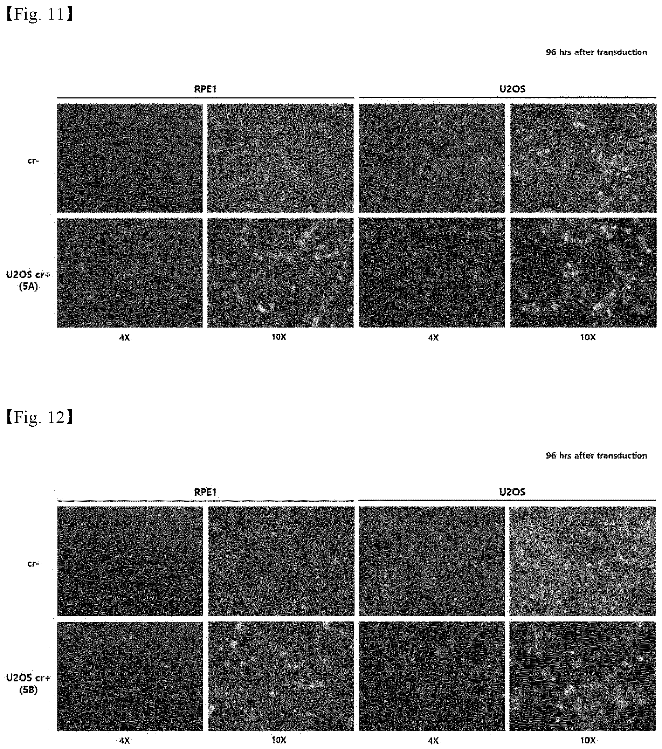

FIG. 11 shows the results of confirming whether it is possible to induce apoptosis by targeting 5 DNA sequences in the U2OS cell line.
FIG. 12 shows the results of confirming whether it is possible to induce apoptosis by targeting 5 DNA sequences (different from those in FIG. 11) in the U2OS cell line.

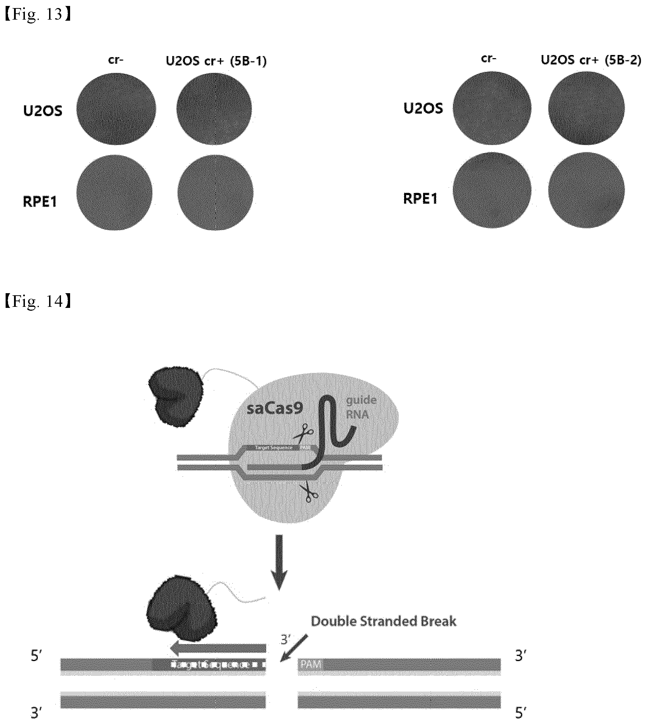

FIG. 13 shows the results of confirming whether it is possible to induce apoptosis by targeting 3 DNA sequences in the U2OS cell line.
FIG. 14 is a schematic view showing Exo-saCas9 and a mechanism of inducing DSB using the same.

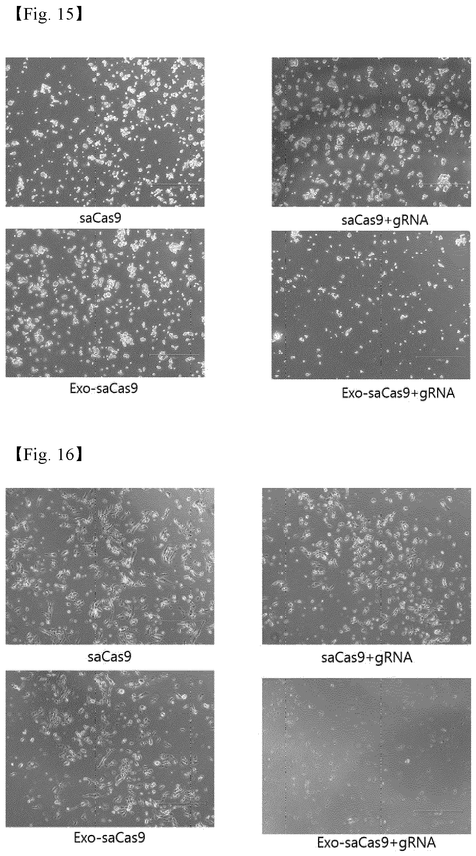

FIG. 15 shows the results obtained by transfection with 5 guide RNAs targeting chromosome 3 and evaluating the effect of the guide RNAs on the apoptosis of HCT116 cells. It was confirmed that, when saCas9 was used alone, apoptosis was insignificant with 5 guide RNAs, but when Exo-saCas9 was used, apoptosis was sufficiently caused by 5 guide RNAs.
FIG. 16 shows the results of confirming that apoptosis in the U2OS cell line can be sufficiently caused by 5 guide RNAs using Exo-saCas9.

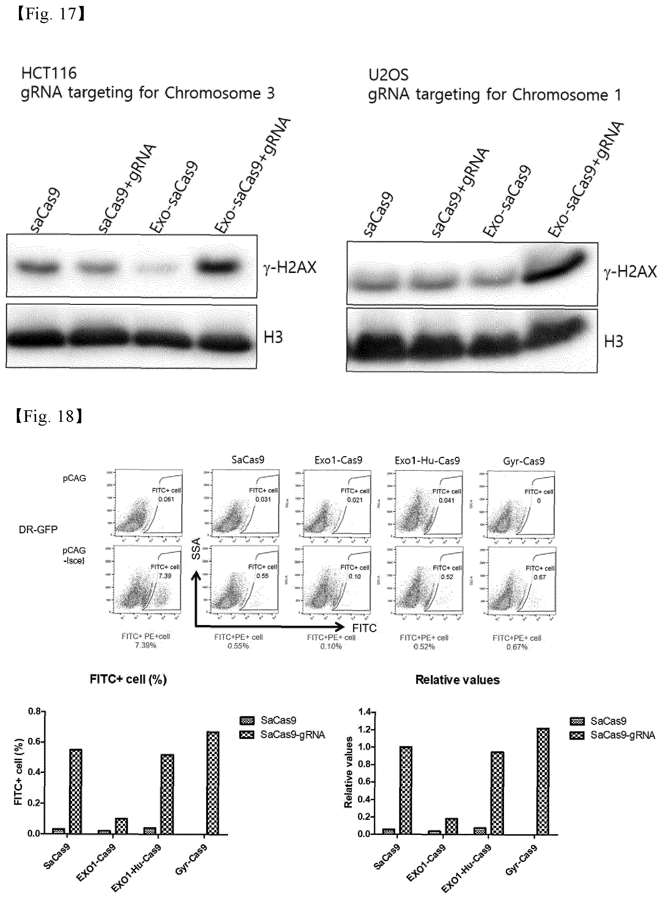

FIG. 17 shows the results of confirming through the DNA damage marker g-H2AX that the reason why Exo-Cas9 induces apoptosis better is that it inhibits damage repair after DNA DSB induction by saCas9.
FIG. 18 shows the results of examining the degree of homologous recombination (HR) when Exo-Cas9 was used, by DR-GFP assay. As a result, it was confirmed through that HR was significantly reduced when Exo-Cas9 was used. This proves that Exo-Cas9 inhibits HR.

### Detailed Description and Preferred Embodiments of the Invention

Unless otherwise defined, all technical and scientific terms used in the present specification have the same meanings as commonly understood by those skilled in the art to which the present disclosure pertains. In general, the nomenclature used in the present specification is well known and commonly used in the art.

In one aspect, the present invention is directed to a composition for inducing apoptosis of cells having genomic sequence variation, the composition containing: (i) a nuclease or a polynucleotide encoding the nuclease; and (ii) cleavaging agents that respectively recognize at least 4 variant regions specific for the cells having genomic sequence variation in the cells having genomic sequence variation, or polynucleotides encoding the cleavaging agents.

### 1. Cells having genomic sequence variation

Even during normal somatic cell replication, about one error occurs while DNA polymerase synthesizes 10⁷ nucleotide pairs. 99% of these mistakes are corrected by the DNA repair mechanism, resulting in one error in replication of 10⁹ nucleotides in total. These mutations accumulate and cause genomic instability.

This genomic instability can be accelerated by extracellular environmental factors, such as exposure to X-rays, ultraviolet rays or radiation, reactive oxygen species (ROS), and the like.

Genomic instability results in the occurrence of genome-damaged cells, that is, "cells having genomic sequence variation," and the mutated genetic information is inherited by the next-generation daughter cells which proliferate.

That is, the "cells having genomic sequence variation" are cells in which a change in the genomic nucleotide sequence has occurred due to the accumulation of mutations. Due to this change in the genomic sequence, the activity and function of the cells become different from those of normal cells, which causes a problem in living or function of an organism or organism's organ, that is, a disease.

For example, diseased cells resulting from "cells having genomic sequence variation" specifically include senescent cells, benign tumor cells, malignant tumor cells, that is, cancer cells, etc. without being limited thereto.

Variant regions specific for these cells having genomic sequence variation are created by insertion (In) of a new DNA sequence, which is not found in normal cells, or deletion (Del) of a portion of normal cell DNA. Usually, such variation sequences are called "indels". Genomic sequence variations also include substitution of a specific sequence with another sequence, in addition to "indels".

A so-called point mutation is a mutation caused by substitution of one base pair in the nucleotide sequences of DNA and RNA. In the present invention, "indel" refers to sequences inserted or deleted relative to the normal cell DNA sequence, as well as substituted sequences and a combination of inserted, deleted and substituted sequences, which are not present in normal cells.

Since these specific "indel" sequences appearing in the DNA of genetically mutated cells are nucleotide sequences that do not exist in normal cells, they can be set as attack targets that distinguish disease-causing cells from for normal cells.

In the present invention, the indel sequences do not mean only sequences associated with disease-causing genes. In addition, sequences having genetic information encoding specific proteins, as well as so-called junk DNA sequences, may be indel sequences of the present invention. That is, indel, which is the main target in the present invention, is 1) a sequence that does not exist in normal cells, and 2) a nucleotide sequence that is commonly present in diseased cells.

The cells having genomic sequence variation refer to cells whose activity is different from that of normal cells due to genetic modification or damage. For example, the cells may refer to diseased cells caused by genetic modification or damage.

For example, diseases such as cancer, aging, immune disease, and cardiovascular disease may be caused by the cells having genomic sequence variation.

Cancer and cellular senescence are typical diseases or phenomena that occur due to genomic instability. In the case of cellular senescence, senescent cells accumulate in body tissues and organs, which easily damage the environment where chronic inflammatory reactions occur, as well as surrounding tissues and cells. Eventually, the regenerative ability of *in vivo* tissues is reduced, causing degenerative diseases such as dementia, diabetes mellitus, and degenerative arthritis. The present invention provides a method for selectively killing "cells having genomic sequence variation". Therefore, it is possible to effectively treat diseases caused by "cells having genomic sequence variation".

Cancer cells are caused by the accumulation of mutations. In this process, many indels are created in DNA, and if cells with indels are provided with the following two genetic features by this genetic mutation, they become cancer cells: (1) the cells proliferate while deviating from normal regulation, and (2) invade and occupy areas normally prepared for other cells. Cells having the first feature but lacking the second feature proliferate excessively but remain only as a single mass to form a tumor. The tumor in this case is called benign, and this tumor can be removed surgically. However, when a tumor has the ability to invade surrounding tissues, it becomes a cancer called malignant. Malignant tumor cells with invasion ability can break away from the primary tumor, enter the bloodstream or lymphatic vessels, and metastasize to other parts of the body to form secondary tumors.

The cancer is, for example, melanoma, small cell lung cancer, non-small cell lung cancer, glioma, liver cancer, thyroid tumor, stomach cancer, prostate cancer, breast cancer, ovarian cancer, bladder cancer, lung cancer, colorectal cancer, breast cancer, prostate cancer, glioblastoma, endometrial cancer, kidney cancer, colon cancer, pancreatic cancer, esophageal carcinoma, head and neck cancer, mesothelioma, sarcoma, osteosarcoma, cholangiocarcinoma, or epidermal cancer, without being limited thereto.

In all types of cancer cells, insertion (IN) of a new DNA sequence, which is not found in normal cells, or deletion (DEL) of a portion of normal cell DNA, is observed, and in each type of cancer cell, a specifically inserted/deleted DNA sequence may exist.

Cells have a DNA damage repair mechanism that repairs DNA double-strand breaks. However, this damage repair mechanism effectively repairs a small number of DNA double-strand breaks, but when the number of DNA double-strand breaks increases, the cell is killed. It is known that bacterial cells can be killed by a single DNA double-strand break, but an increased number of DSBs are required to kill animal cells.

According to the present invention, based on the above facts, it is possible to identify indels in cells having genomic sequence variation, for example, cancer cells, prepare cleavaging agents capable of recognizing the indels, and induce specific apoptosis of cells having genomic sequence variation, for example, cancer cells, by using the cleavaging agents and a nuclease.

In the case of senescence, senescent cells accumulate a lot of DNA damage and have reduced DNA repair activity, and repair must be actively carried out to treat this damage. However, in particular, double-strand break repair is reduced and DNA mutations are frequently observed in senescent cells and tissues.

In the case of cellular senescence, genomic instability due to DNA damage is a major senescence pathway. When DNA damage occurs, important genes can be damaged or transcriptional regulation processes can change, resulting in problems in the production and function of proteins involved in specific biochemical pathways and disruption of normal cell functions. If these cells accumulate without being eliminated, they may endanger the homeostasis of the whole organism without ending with the breakdown of single cells.

In senescent cells, insertion (IN) of a new DNA sequence, which is not found in normal cells, or deletion (DEL) of a portion of normal cell DNA, is observed, and in each type of senescent cells, a specifically inserted/deleted DNA sequence may exist.

According to the present invention, based on the above facts, it is possible to identify indels in cells having genomic sequence variation, for example, senescent cells, prepare cleavaging agents capable of recognizing the indels, and induce specific apoptosis of cells having genomic sequence variation, for example, senescent cells, by using the cleavaging agents and a nuclease.

By selectively removing senescent cells, senescence-related features can be alleviated. When senescent cells were selectively killed by expressing an apoptotic gene together with the p16 promoter serving as a marker of cellular senescence, and the function of the tissue was examined, and it was confirmed that alleviation of senescence-related features occurred. Research results revealed that, when senescent cells were removed from a mouse model at an early stage, cataracts and muscle loss, which are typical senescence phenotypes, did not occur, and exercise performance also increased (Baker, D. J., T, (2011) Nature 479:232-236).

In the case of immune diseases, B-cell receptors and T-cell receptors exist in mammalian lymphocyte cells, and can recognize various types of antigens while variable (V), diversity (D) and joining (J) genes are combined together through the V(D)J recombination process. In this process, double-strand breaks occur, and when the proteins that control these double-strand breaks are lacking, the V(D)J recombination process cannot function properly, leading to various diseases such as immunodeficiency and neurological defects.

In immune disease-causing cells, insertion (IN) of a new DNA sequence, which is not found in normal cells, or deletion (DEL) of a portion of normal cell DNA, is observed, and in each type of immune disease-causing cells, a specifically inserted/deleted DNA sequence may exist.

According to the present invention, based on the above facts, it is possible to identify indels in cells having genomic sequence variation, for example, immune disease-causing cells, prepare cleavaging agents capable of recognizing the indels, and induce specific apoptosis of cells having genomic sequence variation, for example, immune disease-causing cells, by using the cleavaging agents and a nuclease.

Regarding cardiovascular diseases, for example, heart failure is a disease that occurs when the pumping function of the heart is weakened, and it is known that there is a relationship between heart failure, diabetes, and insulin resistance in heart failure patients. It is also known that, in heart failure, DNA damage accumulates in the heart tissue and visceral fat due to the inflammatory response and insulin resistance of adipose tissue. In fact, it has been found that, when a high-calorie diet was given to mice, a lot of fatty acids were introduced into adipose tissue, causing excessive production of ROS, which resulted in DNA damage accumulation, and that p53 was involved in the inflammatory response and insulin resistance of adipose tissue. This excessive fat breakdown induces oxidative stress in the nervous system, which leads to DNA damage and a p53-dependent inflammatory response in adipose tissue. In addition, it has been reported that DNA damage accumulates in the early stages of atherosclerosis. In addition, it has been reported that DNA damage increases the level of plaque, and this increase in the level of plaque leads to atherosclerosis. This suggests that DNA damage may also play an important role in atherosclerosis.

Most of the diseases caused by "cells having genomic sequence variation" are caused by deficiency in DNA repair or DNA damage response. Typical diseases include Aicardi-Goutières syndrome (brain atrophy and muscle abnormalities), amyotrophic lateral sclerosis 4 (ALS4, degeneration of nerve cells); Meier-Gorlin Syndrome (microencephaly, abnormality of the kneecap), Seckel syndrome (a disease in which the blood count decreases), Schimke immune-osseous dysplasia (short stature, microencephaly, kidney failure, immunodeficiency, curvature of the back), Ataxia telangiectasia (immune system abnormality, leukemia), Nijmegen breakage syndrome (immune system abnormality, lymphoma development), Bloom syndrome (vasodilation due to skin rash, skin pigmentation abnormality, immune system abnormality, lung disease), Warner syndrome (rapid aging), Rothmund-Thomson syndrome (skin abnormalities, red spots, vasodilation, cataracts), Fanconi anemia (anemia, thrombocytopenia, increased leukemia), Xeroderma pigmentosum (sun sensitivity, skin cancer, pigmentosis), Cockayne syndrome (microphagia, dwarfism, sun sensitivity), Lynch syndrome (colorectal cancer), familial adenomatous polyposis (increased colorectal cancer and rectal cancer), and the like. Patients with these diseases make cells having many genomic sequence variations, thereby causing various diseases in each organ.

In cardiovascular disease-causing cells, insertion (IN) of a new DNA sequence, which is not found in normal cells, or deletion (DEL) of a portion of normal cell DNA, is observed, and in each type of cardiovascular disease-causing cells, a specifically inserted/deleted DNA sequence may exist.

According to the present invention, based on the above facts, it is possible to identify indels in cells having genomic sequence variation, for example, cardiovascular disease-causing cells, prepare cleavaging agents capable of recognizing the indels, and induce specific apoptosis of cells having genomic sequence variation, for example, cardiovascular disease-causing cells, by using the cleavaging agents and a nuclease.

Different indels that are present specifically in genetically mutated cells can be identified by analyzing the genome sequence compared to that of normal cells. For genome sequence analysis, whole genome sequences can be compared, and in some cases, only a specific chromosome can be selected for analysis and comparison of nucleotide sequences.

In some cases, when indels are selected using known whole genome sequencing (WGS) without WGS of cancer cells, only indels that does not exist in normal cells can be identified. In the future, when many indels are found in cancer cells of patients, indels that commonly appear may exist. In this case, it is possible to use a strategy of observing only indels, which appear commonly, in cancer cells by PCR or the like, and incorporating the same in a target composition. In addition, although detection of indels by WGS is currently the most effective method, subtraction hybridization (a method of identifying sites, which do not bind to each other, as indels by denaturing normal cell DNA and cancer cell DNA, followed by hybridization) is also possible. Therefore, it is possible to use a method for identifying indels specific for genetically modified cells by using various methods that can detect indels, including WGS.

### 2. Variant regions

In one embodiment, variant regions specific for the cells having genome sequence variation may be at least four different variant regions corresponding to a target sufficient to induce apoptosis. Variant regions specific for the cells having genome sequence variation may be, for example, 30 or less different variant regions. Variant regions specific for the cells having genome sequence variation may be, for example, 4 to 30 different variant regions.

Variant regions specific for the cells having genome sequence variation may be specifically 4 to 30, more specifically 5 to 30, 5 to 29, 5 to 28, 5 to 27, 5 to 26, 5 to 25, 5 to 24, 5 to 23, 5 to 22, 5 to 21, 5 to 20, 5 to 19, 5 to 18, 5 to 17, 5 to 16, 5 to 15, 5 to 14, 5 to 13, 5 to 12, 5 to 11, 5 to 10, 6 to 30, 6 to 29, 6 to 28, 6 to 27, 6 to 26, 6 to 25, 6 to 24, 6 to 23, 6 to 22, 6 to 21, 6 to 20, 6 to 19, 6 to 18, 6 to 17, 6 to 16, 6 to 15, 6 to 14, 6 to 13, 6 to 12, 6 to 11, 6 to 10, 7 to 30, 7 to 29, 7 to 28, 7 to 27, 7 to 26, 7 to 25, 7 to 24, 7 to 23, 7 to 22, 7 to 21, 7 to 20, 7 to 19, 7 to 18, 7 to 17, 7 to 16, 7 to 15, 7 to 14, 7 to 13, 7 to 12, 7 to 11, 7 to 10, 8 to 30, 8 to 29, 8 to 28, 8 to 27, 8 to 26, 8 to 25, 8 to 24, 8 to 23, 8 to 22, 8 to 21, 8 to 20, 8 to 19, 8 to 18, 8 to 17, 8 to 16, 8 to 15, 8 to 14, 8 to 13, 8 to 12, 8 to 11, 8 to 10, 9 to 30, 9 to 29, 9 to 28, 9 to 27, 9 to 26, 9 to 25, 9 to 24, 9 to 23, 9 to 22, 9 to 21, 9 to 20, 9 to 19, 9 to 18, 9 to 17, 9 to 16, 9 to 15, 9 to 14, 9 to 13, 9 to 12, 9 to 11, 9 to 10, 10 to 30, 10 to 29, 10 to 28, 10 to 27, 10 to 26, 10 to 25, 10 to 24, 10 to 23, 10 to 22, 10 to 21, 10 to 20, 10 to 19, 10 to 18, 10 to 17, 10 to 16, 10 to 15, 10 to 14, 10 to 13, 10 to 12, 10 to 11, 11 to 30, 11 to 29, 11 to 28, 11 to 27, 11 to 26, 11 to 25, 11 to 24, 11 to 23, 11 to 22, 11 to 21, 11 to 20, 11 to 19, 11 to 18, 11 to 17, 11 to 16, 11 to 15, 11 to 14, 11 to 13, 11 to 12, 12 to 30, 12 to 29, 12 to 28, 12 to 27, 12 to 26, 12 to 25, 12 to 24, 12 to 23, 12 to 22, 12 to 21, 12 to 20, 12 to 19, 12 to 18, 12 to 17, 12 to 16, 12 to 15, 12 to 14, 12 to 13, 13 to 30, 13 to 29, 13 to 28, 13 to 27, 13 to 26, 13 to 25, 13 to 24, 13 to 23, 13 to 22, 13 to 21, 13 to 20, 13 to 19, 13 to 18, 13 to 17, 13 to 16, 13 to 15, 13 to 14, 14 to 30, 14 to 29, 14 to 28, 14 to 27, 14 to 26, 14 to 25, 14 to 24, 14 to 23, 14 to 22, 14 to 21, 14 to 20, 14 to 19, 14 to 18, 14 to 17, 14 to 16, 14 to 15, 15 to 30, 15 to 29, 15 to 28, 15 to 27, 15 to 26, 15 to 25, 15 to 24, 15 to 23, 15 to 22, 15 to 21, 15 to 20, 15 to 19, 15 to 18, 15 to 17, or 15 to 16 different variant regions.

The number of different variant regions specific for the cells having genome sequence variation may be specifically 4, 5, 6, 7, 8, 9, 10, 11, 12, 13, 14, 15, 16, 17, 18, 19, 20, 21, 22, 23, 24, 25, 26, 27, 28, 29 or 30 (also including ranges between the numbers).

The genome containing the positions where variation exists may be diploid and/or triploid in the cells. Thus, in consideration of 4, 5, 6, 7, 8, 9, 10, 11, 12, 13, 14, 15, 16, 17, 18, 19, 20, 21, 22, 23, 24, 25, 26, 27, 28, 29 or 30 (also including ranges between the numbers) different variant regions or 4, 5, 6, 7, 8, 9, 10, 11, 12, 13, 14, 15, 16, 17, 18, 19, 20, 21, 22, 23, 24, 25, 26, 27, 28, 29 or 30 pairs of variant regions, the cells may be killed by inducing 4 to 60, for example, 60, 59, 58, 57, 4 to 56, 55, 54, 53, 52, 51, 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5 or 4 (also including ranges between the numbers) different double-stranded breaks (DSBs).

The variant regions specific for the cells having genomic sequence variation may be nucleic acid sequences, for example, coding or non-coding nucleic acid sequences. The nucleic acids of the variant regions specific for the cells having genomic sequence variation may be coding nucleic acids, and may be transcribed from the genome and translated into proteins. The nucleic acids of the variant regions specific for the cells having genomic sequence variation may be non-coding nucleic acids, and are not transcribed from the genome and translated into proteins.

Variation in the cells having genomic sequence variation according to the present invention may not be found in normal cells. This variation may be the sequence of a variant region specific for cells having genomic sequence variation, obtained by WGS (whole genome sequencing) of the cells having genomic sequence variation and normal cells. For example, it may be at least one selected from the group consisting of insertion, deletion and substitution of a sequence.

Mapping of the sequences specific for the cell having genomic sequence variation may be performed through WGS (whole genome sequencing), or subtractive hybridization and sequencing methods. In the case of detected indels, when insertions are found in cancer, guide RNAs may be prepared immediately, and when deletions are found in cancer, break points may be mapped and then guide RNAs including break points may be prepared and used.

WGS (whole genome sequencing) refers to a method of reading the genome in various multiples (10X, 20X and 40X formats) by next generation sequencing. "Next-generation sequencing" refers to technology of fragmenting the whole genome in a chip-based and PCR-based paired end format and performing high-throughput sequencing of the fragments on the basis of chemical reaction (hybridization).

Subtractive hybridization is a method used for cloning a gene whose expression differs between various tissues or cells. A gene specific for the DNA sample in the cell tested can be detected. The DNA in the cell to be tested is denatured into single-stranded DNA and then annealed. By controlling the annealing conditions, the DNA sequence specific for the cell tested can be separated as double-stranded DNA.

The nucleic acid sequence of the variant region specific for the cells having genomic sequence variation refers to a gene region in which DNA DSBs in the nucleic acid sequence are induced by, for example, a nuclease targeting the sequence. Specifically, it may refer to a sequence in the nucleic acid sequence, which is specifically recognized by a nuclease. For example, when the nuclease is Cas9, the nucleic acid sequence may be an about 17-bp to 23-bp nucleic acid sequence located adjacent to the 5' end and/or 3' end of the PAM sequence, which is recognized by the Cas9 protein.

A guide RNA that specifically recognizes the sequence of the variant region specific for cells having genomic sequence variation refers to a nucleotide sequence having a sequence homology of 90% or more, 95% or more, 96% or more, 97% or more, 98% or more, 99% or more, or 100% to the nucleotide sequence of the complementary strand of the DNA strand in which the PAM sequence is located. The guide RNA may bind to the nucleotide sequence of the complementary strand.

The sequence of the variant region specific for the cells having genomic sequence variation is represented by the nucleic acid sequence of the strand in which the PAM sequence is located, among the two DNA strands of the corresponding sequence region. In this case, since the DNA strand to which the guide RNA actually binds is the complementary strand of the strand in which the PAM sequence is located, the targeting sequence included in the guide RNA has the same nucleic acid sequence as the nucleic acid sequence comprising an indel, except for the change from T to U due to the nature of the RNA.

When the Cas9 protein is from *Streptococcus pyogenes,* the PAM sequence may be 5'-NGG-3' (where N is A, T, G, or C), and the nucleic acid sequence comprising the variation sequence specific for cells having genomic sequence variation may be a gene region located adjacent to the 5' end and/or 3' end of the 5'-NGG-3' sequence. For example, the nucleic acid sequence may be a gene region having a maximum length of about 50 bp or about 40 bp.

When the Cas9 protein is from *Streptococcus thermophilus,* the PAM sequence may be 5'-NNAGAAW-3' (where N is A, T, G, or C), and the variation sequence specific for cells having genomic sequence variation may be a gene region located adjacent to the 5' end and/or 3' end of the 5'-NNAGAAW-3' sequence. For example, the variation sequence may be a gene region having a maximum length of about 50 bp or about 40 bp.

When the Cas9 protein is from *Staphylococcus aureus,* the PAM sequence may be 5'-NNGRRT-3' (where N is A, T, G, or C, and R is A or G), and the variation sequence specific for cells having genomic sequence variation may be a gene region located adjacent to the 5' end and/or 3' end of the 5'-NNGRRT-3' sequence. For example, the variation sequence may be a gene region having a maximum length of about 50 bp or about 40 bp.

When the Cas9 protein is from *Campylobacter jejuni,* the PAM sequence may be 5'-NNNNRYAC-3' (where N is A, T, G, or C, R is A or G, and Y is C or T), and the variation sequence specific for cells having genomic sequence variation may be a gene region located adjacent to the 5' end and/or 3' end of the 5'-NNNNRYAC-3' sequence. For example, the variation sequence may be a gene region having a maximum length of about 50 bp or about 40 bp.

### 3. Cleavaging agents

As used herein, the term "cleavaging agent" refers to a nucleotide sequence capable of recognizing a mutated region in cells having genomic sequence variation compared to normal cells, that is, the nucleic acid sequence of a variant region specific for cells having genomic sequence variation, thereby inducing cleavage of the region.

A guide RNA that specifically recognizes the variant region specific for cells having genomic sequence variation refers to a nucleotide sequence having a sequence homology of 90% or more, 95% or more, 96% or more, 97% or more, 98% or more, 99% or more, or 100% to the nucleotide sequence of the complementary strand of the DNA strand in which the PAM sequence is located. The guide RNA may bind to the nucleotide sequence of the complementary strand.

The cleavaging agent that specifically recognizes a nucleic acid sequence including an indel in cells having genomic sequence variation may be, for example, a guide RNA. The guide RNA may be, for example, at least one selected from the group consisting of CRISPR RNA (crRNA), trans-activating crRNA (tracrRNA), and single-stranded guide RNA (sgRNA). Specifically, the guide RNA may be a double-stranded crRNA:tracrRNA complex in which crRNA and tracrRNA are linked together, or a single-strand guide RNA (sgRNA) in which crRNA or a portion thereof and tracrRNA or a portion thereof are linked together by an oligonucleotide linker.

In a specific embodiment of the present invention, a double-stranded crRNA: tracrRNA in which crRNA and tracrRNA are linked together may be used as the cleavaging agent. Endonuclease function can be activated by recognizing the binding of crRNA to a target region. In some cases, single-stranded guide RNA (sgRNA) may be used as the cleavaging agent.

In the present invention, a plurality of the cleavaging agents may be used and comprise different sequences.

In the present invention, the number of the plurality of cleavaging agents having different sequences should be sufficient to induce apoptosis compared to the nucleotide sequences of normal cells, and may be 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, 21 or more, 22 or more, 23 or more, 24 or more, 25 or more, 26 or more, 27 or more, 28 or more, 29 or more, or 30 or more. In addition, the number of the cleavaging agents having different sequences may be 30 or less, 29 or less, 28 or less, 27 or less, 26 or less, 25 or less, 24 or less, 23 or less, 22 or less, 21 or less, 20 or less, 19 or less, 18 or less, 17 or less, 16 or less, 15 or less, 14 or less, 13 or less, 12 or less, 11 or less, 10 or less, 9 or less, 8 or less, 7 or less, 6 or less, 5 or less, 4 or less, 3 or less, or 2 or less.

In the present invention, the number of the plurality of cleavaging agents having different sequences should be sufficient to induce apoptosis compared to the nucleotide sequences of normal cells, and may be 4 to 60, for example, 4 to 59, 4 to 58, 4 to 57, 4 to 56, 4 to 55, 4 to 54, 4 to 53, 4 to 52, 4 to 51, 4 to 50, 4 to 49, 4 to 48, 4 to 47, 4 to 46, 4 to 45, 4 to 44, 4 to 43, 4 to 42, 4 to 41, 4 to 40, 4 to 39, 4 to 38, 4 to 37, 4 to 36, 4 to 35, 4 to 34, 4 to 33, 4 to 32, 4 to 31, 4 to 30, 4 to 29, 4 to 28, 4 to 27, 4 to 26, 4 to 25, 4 to 24, 4 to 23, 4 to 22, 4 to 21, 4 to 20, 4 to 19, 4 to 18, 4 to 17, 4 to 16, 4 15, 4 to 14, 4 to 13, 4 to 12, 4 to 11, or 4 to 10.

In the present invention, the number of the plurality of cleavaging agents having different sequences should be sufficient to induce apoptosis compared to the nucleotide sequences of normal cells, and may be, for example, 4 to 30, 4 to 29, 4 to 28, 4 to 27, 4 to 26, 4 to 25, 4 to 24, 4 to 23, 4 to 22, 4 to 21, 4 to 20, 4 to 19, 4 to 8, 4 to 17, 4 to 16, 4 to 15, 4 to 14, 4 to 13, 4 to 12, 4 to 11, 4 to 10, 5 to 30, 5 to 29, 5 to 28, 5 to 27, 5 to 26, 5 to 25, 5 to 24, 5 to 23, 5 to 22, 5 to 21, 5 to 20, 5 to 19, 5 to 18, 5 to 17, 5 to 16, 5 to 15, 5 to 14, 5 to 13, 5 to 12, 5 to 11, 5 to 10, 6 to 30, 6 to 29, 6 to 28, 6 to 27, 6 to 26, 6 to 25, 6 to 24, 6 to 23, 6 to 22, 6 to 21, 6 to 20, 6 to 19, 6 to 18, 6 to 17, 6 to 16, 6 to 15, 6 to 14, 6 to 13, 6 to 12, 6 to 11, 6 to 10, 7 to 30, 7 to 29, 7 to 28, 7 to 27, 7 to 26, 7 to 25, 7 to 24, 7 to 23, 7 to 22, 7 to 21, 7 to 20, 7 to 19, 7 to 18, 7 to 17, 7 to 16, 7 to 15, 7 to 14, 7 to 13, 7 to 12, 7 to 11, 7 to 10, 8 to 30, 8 to 29, 8 to 28, 8 to 27, 8 to 26, 8 to 25, 8 to 24, 8 to 23, 8 to 22, 8 to 21, 8 to 20, 8 to 19, 8 to 18, 8 to 17, 8 to 16, 8 to 15, 8 to 14, 8 to 13, 8 to 12, 8 to 11, 8 to 10, 9 to 30, 9 to 29, 9 to 28, 9 to 27, 9 to 26, 9 to 25, 9 to 24, 9 to 23, 9 to 22, 9 to 21, 9 to 20, 9 to 19, 9 to 18, 9 to 17, 9 to 16, 9 to 15, 9 to 14, 9 to 13, 9 to 12, 9 to 11, 9 to 10, 10 to 30, 10 to 29, 10 to 28, 10 to 27, 10 to 26, 10 to 25, 10 to 24, 10 to 23, 10 to 22, 10 to 21, 10 to 20, 10 to 19, 10 to 18, 10 to 17, 10 to 16, 10 to 15, 10 to 14, 10 to 13, 10 to 12, 10 to 11, 11 to 30, 11 to 29, 11 to 28, 11 to 27, 11 to 26, 11 to 25, 11 to 24, 11 to 23, 11 to 22, 11 to 21, 11 to 20, 11 to 19, 11 to 18, 11 to 17, 11 to 16, 11 to 15, 11 to 14, 11 to 13, 11 to 12, 12 to 30, 12 to 29, 12 to 28, 12 to 27, 12 to 26, 12 to 25, 12 to 24, 12 to 23, 12 to 22, 12 to 21, 12 to 20, 12 to 19, 12 to 18, 12 to 17, 12 to 16, 12 to 15, 12 to 14, 12 to 13, 13 to 30, 13 to 29, 13 to 28, 13 to 27, 13 to 26, 13 to 25, 13 to 24, 13 to 23, 13 to 22, 13 to 21, 13 to 20, 13 to 19, 13 to 18, 13 to 17, 13 to 16, 13 to 15, 13 to 14, 14 to 30, 14 to 29, 14 to 28, 14 to 27, 14 to 26, 14 to 25, 14 to 24, 14 to 23, 14 to 22, 14 to 21, 14 to 20, 14 to 19, 14 to 18, 14 to 17, 14 to 16, 14 to 15, 15 to 30, 15 to 29, 15 to 28, 15 to 27, 15 to 26, 15 to 25, 15 to 24, 15 to 23, 15 to 22, 15 to 21, 15 to 20, 15 to 19, 15 to 18, 15 to 17, or 15 to 16.

In the present invention, the number of the cleavaging agents having different sequences may be, for example, 6 to 60, for example, 6 to 59, 6 to 58, 6 to 57, 6 to 56, 6 to 55, 6 to 54, 6 to 53, 6 to 52, 6 to 51, 6 to 50, 6 to 49, 6 to 48, 6 to 47, 6 to 46, 6 to 45, 6 to 44, 6 to 43, 6 to 42, 6 to 41, 6 to 40, 6 to 39, 6 to 38, 6 to 37, 6 to 36, 6 to 35, 6 to 34, 6 to 33, 6 to 32, 6 to 31, 6 to 30, 6 to 29, 6 to 28, 6 to 27, 6 to 26, 6 to 25, 6 to 24, 6 to 23, 6 to 22, 6 to 21, 6 to 20, 6 to 19, 6 to 18, 6 to 17, 6 to 16, 6 to 15, 6 to 14, 6 to 13, 6 to 12, 6 to 11, or 6 to 10.

In the present invention, the number of the cleavaging agents having different sequences may be, for example, 8 to 60, for example, 8 to 59, 8 to 58, 8 to 57, 8 to 56, 8 to 55, 8 to 54, 8 to 53, 8 to 52, 8 to 51, 8 to 50, 8 to 49, 8 to 48, 8 to 47, 8 to 46, 8 to 45, 8 to 44, 8 to 43, 8 to 42, 8 to 41, 8 to 40, 8 to 39, 8 to 38, 8 to 37, 8 to 36, 8 to 35, 8 to 34, 8 to 33, 8 to 32, 8 to 31, 8 to 30, 8 to 29, 8 to 28, 8 to 27, 8 to 26, 8 to 25, 8 to 24, 8 to 23, 8 to 22, 8 to 21, 8 to 20, 8 to 19, 8 to 18, 8 to 17, 8 to 16, 8 to 15, 8 to 14, 8 to 13, 8 to 12, 8 to 11, or 8 to 10.

In the present invention, the number of the cleavaging agents having different sequences may be, for example, 10 to 60, for example, 10 to 59, 10 to 58, 10 to 57, 10 to 56, 10 to 55, 10 to 54, 10 to 53, 10 to 52, 10 to 51, 10 to 50, 10 to 49, 10 to 48, 10 to 47, 10 to 46, 10 to 45, 10 to 44, 10 to 43, 10 to 42, 10 to 41, 10 to 40, 10 to 39, 10 to 38, 10 to 37, 10 to 36, 10 to 35, 10 to 34, 10 to 33, 10 to 32, 10 to 31, 10 to 30, 10 to 29, 10 to 28, 10 to 27, 10 to 26, 10 to 25, 10 to 24, 10 to 23, 10 to 22, 10 to 21, 10 to 20, 10 to 19, 10 to 18, 10 to 17, 10 to 16, 10 to 15, 10 to 14, 10 to 13, or 10 to 12.

In the present invention, the number of the cleavaging agents having different sequences may be, for example, 12 to 60, for example, 12 to 59, 12 to 58, 12 to 57, 12 to 56, 12 to 55, 12 to 54, 12 to 53, 12 to 52, 12 to 51, 12 to 50, 12 to 49, 12 to 48, 12 to 47, 12 to 46, 12 to 45, 12 to 44, 12 to 43, 12 to 42, 12 to 41, 12 to 40, 12 to 39, 12 to 38, 12 to 37, 12 to 36, 12 to 35, 12 to 34, 12 to 33, 12 to 32, 12 to 31, 12 to 30, 12 to 29, 12 to 28, 12 to 27, 12 to 26, 12 to 25, 12 to 24, 12 to 23, 12 to 22, 12 to 21, 12 to 20, 12 to 19, 12 to 18, 12 to 17, 12 to 16, 12 to 15, 12 to 14, or 12 to 13.

In the present invention, the number of the cleavaging agents having different sequences may be, for example, 14 to 60, for example, 14 to 59, 14 to 58, 14 to 57, 14 to 56, 14 to 55, 14 to 54, 14 to 53, 14 to 52, 14 to 51, 14 to 50, 14 to 49, 14 to 48, 14 to 47, 14 to 46, 14 to 45, 14 to 44, 14 to 43, 14 to 42, 14 to 41, 14 to 40, 14 to 39, 14 to 38, 14 to 37, 14 to 36, 14 to 35, 14 to 34, 14 to 33, 14 to 32, 14 to 31, 14 to 30, 14 to 29, 14 to 28, 14 to 27, 14 to 26, 14 to 25, 14 to 24, 14 to 23, 14 to 22, 14 to 21, 14 to 20, 14 to 19, 14 to 18, 14 to 17, 14 to 16, or 14 to 15.

In the present invention, the number of the cleavaging agents having different sequences may be, for example, 16 to 60, for example, 16 to 59, 16 to 58, 16 to 57, 16 to 56, 16 to 55, 16 to 54, 16 to 53, 16 to 52, 16 to 51, 16 to 50, 16 to 49, 16 to 48, 16 to 47, 16 to 46, 16 to 45, 16 to 44, 16 to 43, 16 to 42, 16 to 41, 16 to 40, 16 to 39, 16 to 38, 16 to 37, 16 to 36, 16 to 35, 16 to 34, 16 to 33, 16 to 32, 16 to 31, 16 to 30, 16 to 29, 16 to 28, 16 to 27, 16 to 26, 16 to 25, 16 to 24, 16 to 23, 16 to 22, 16 to 21, 16 to 20, 16 to 19, 16 to 18, or 16 to 17.

In the present invention, the number of the cleavaging agents having different sequences may be, for example, 18 to 60, for example, 18 to 59, 18 to 58, 18 to 57, 18 to 56, 18 to 55, 18 to 54, 18 to 53, 18 to 52, 18 to 51, 18 to 50, 18 to 49, 18 to 48, 18 to 47, 18 to 46, 18 to 45, 18 to 44, 18 to 43, 18 to 42, 18 to 41, 18 to 40, 18 to 39, 18 to 38, 18 to 37, 18 to 36, 18 to 35, 18 to 34, 18 to 33, 18 to 32, 18 to 31, 18 to 30, 18 to 29, 18 to 28, 18 to 27, 18 to 26, 18 to 25, 18 to 24, 18 to 23, 18 to 22, 18 to 21, 18 to 20, or 18 to 19.

In the present invention, the number of the cleavaging agents having different sequences may be, for example, 20 to 60, for example, 20 to 59, 20 to 58, 20 to 57, 20 to 56, 20 to 55, 20 to 54, 20 to 53, 20 to 52, 20 to 51, 20 to 50, 20 to 49, 20 to 48, 20 to 47, 20 to 46, 20 to 45, 20 to 44, 20 to 43, 20 to 42, 20 to 41, 20 to 40, 20 to 39, 20 to 38, 20 to 37, 20 to 36, 20 to 35, 20 to 34, 20 to 33, 20 to 32, 20 to 31, 20 to 30, 20 to 29, 20 to 28, 20 to 27, 20 to 26, 20 to 25, 20 to 24, 20 to 23, 20 to 22, or 20 to 21.

In the present invention, the number of the cleavaging agents having different sequences may be, for example, 22 to 60, for example, 22 to 59, 22 to 58, 22 to 57, 22 to 56, 22 to 55, 22 to 54, 22 to 53, 22 to 52, 22 to 51, 22 to 50, 22 to 49, 22 to 48, 22 to 47, 22 to 46, 22 to 45, 22 to 44, 22 to 43, 22 to 42, 22 to 41, 22 to 40, 22 to 39, 22 to 38, 22 to 37, 22 to 36, 22 to 35, 22 to 34, 22 to 33, 22 to 32, 22 to 31, 22 to 30, 22 to 29, 22 to 28, 22 to 27, 22 to 26, 22 to 25, 22 to 24, or 22 to 23.

In the present invention, the number of the cleavaging agents having different sequences may be, for example, 24 to 60, for example, 24 to 59, 24 to 58, 24 to 57, 24 to 56, 24 to 55, 24 to 54, 24 to 53, 24 to 52, 24 to 51, 24 to 50, 24 to 49, 24 to 48, 24 to 47, 24 to 46, 24 to 45, 24 to 44, 24 to 43, 24 to 42, 24 to 41, 24 to 40, 24 to 39, 24 to 38, 24 to 37, 24 to 36, 24 to 35, 24 to 34, 24 to 33, 24 to 32, 24 to 31, 24 to 30, 24 to 29, 24 to 28, 24 to 27, 24 to 26, or 24 to 25.

In the present invention, the number of the cleavaging agents having different sequences may be, for example, 26 to 60, for example, 26 to 59, 26 to 58, 26 to 57, 26 to 56, 26 to 55, 26 to 54, 26 to 53, 26 to 52, 26 to 51, 26 to 50, 26 to 49, 26 to 48, 26 to 47, 26 to 46, 26 to 45, 26 to 44, 26 to 43, 26 to 42, 26 to 41, 26 to 40, 26 to 39, 26 to 38, 26 to 37, 26 to 36, 26 to 35, 26 to 34, 26 to 33, 26 to 32, 26 to 31, 26 to 30, 26 to 29, 26 to 28, or 26 to 27.

In the present invention, the number of the cleavaging agents having different sequences may be, for example, 28 to 60, for example, 28 to 59, 28 to 58, 28 to 57, 28 to 56, 28 to 55, 28 to 54, 28 to 53, 28 to 52, 28 to 51, 28 to 50, 28 to 49, 28 to 48, 28 to 47, 28 to 46, 28 to 45, 28 to 44, 28 to 43, 28 to 42, 28 to 41, 28 to 40, 28 to 39, 28 to 38, 28 to 37, 28 to 36, 28 to 35, 28 to 34, 28 to 33, 28 to 32, 28 to 31, 28 to 30, or 28 to 29.

In the present invention, the number of the cleavaging agents having different sequences may be, for example, 30 to 60, for example, 30 to 59, 30 to 58, 30 to 57, 30 to 56, 30 to 55, 30 to 54, 30 to 53, 30 to 52, 30 to 51, 30 to 50, 30 to 49, 30 to 48, 30 to 47, 30 to 46, 30 to 45, 30 to 44, 30 to 43, 30 to 42, 30 to 41, 30 to 40, 30 to 39, 30 to 38, 30 to 37, 30 to 36, 30 to 35, 30 to 34, 30 to 33, 30 to 32, or 30 to 31.

In the present invention, the number of the cleavaging agents having different sequences may be, for example, 60, 59, 58, 57, 56, 55, 54, 53, 52, 51, 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, or 4 (also including ranges between the numbers).

The number of the cleavaging agents may be, for example, 5 to 30. For example, the number of the cleavaging agents having different sequences may be 30, 29, 28, 27, 26, 25, 24, 23, 22, 21, 20, 19, 18, 17, 16, 15, 14, 13, 12, 11, 10, 9, 8, 7, 6 or 5. The number of the cleavaging agents having different sequences should be sufficient to induce apoptosis compared to the nucleotide sequences of normal cells and may be 4 to 30. The number of the cleavaging agents having difference sequences may be, for example, 30, 29, 28, 27, 26, 25, 24, 23, 22, 21, 20, 19, 18, 17, 16, 15, 14, 13, 12, 11, 10, 9, 8, 7, 6, 5 or 4.

Depending on whether the chromosome to be targeted by the cleavaging agents is diploid or triploid, it may include 2 or 3 sequences to be targeted by each cleavaging agent. Thus, the number of double-strand breaks (DSBs) induced by the cleavaging agents may be double or triple the number of the cleavaging agents capable of inducing apoptosis. For example, when 5 cleavaging agents having different sequences are contained, 10 to 15 double-strand breaks (DSBs) are required for apoptosis.

The guide RNAs may be prepared through the following steps: By comparing the WGS data of cells having genomic sequence variation with those of normal cells, variant sequences specific for the cells having genomic sequence variation are identified. Based on the variant sequences, guide RNAs specific to the variant sequences specific for the cells having genomic sequence variation, which satisfy guide RNA preparation conditions, are designed. Thereafter, an arbitrary ranking is chosen in consideration of the length of each variant site specific for the cells having genomic sequence variation, and guide RNAs are designed evenly for all chromosomes to obtain the final guide RNA combination.

The guide RNA preparation conditions are as follows. (a) The nucleotide sequence length of each guide RNA excluding the PAM site is 20 base pairs. (b) The proportion of the sum of guanine and cytosine present in the guide RNA is between 40% and 60%. (c) A variant sequence specific for cells having genomic sequence variation exists in the region immediately preceding the PAM site. (d) The maximum homopolymer length is 4 base pairs or less. (e) When mapping allowing one mismatch to the WGS data of normal cells is performed, there should be no mapping result.

After the cell line-specific indels are identified by whole-genome sequencing (WGS), each guide RNA is designed to be included in the PAM site region that strongly binds to the corresponding region. The designed guide RNA is checked using a normal human genome reference sequence to confirm that there is no non-specific reaction. Thereafter, an arbitrary ranking is chosen in consideration of the length of each indel site, and based on this, guide RNAs are designed evenly for all chromosomes to obtain the final guide RNA combination. The number of guide RNAs may be adjusted depending on the type of cell having genomic sequence variation and the experimental method that causes DSBs.

### 4. Nuclease

The nuclease, which is a means for inducing DNA double-strand breaks, may be a restriction enzyme, a zinc finger nuclease (ZNFN), a transcriptional activator-like effector nuclease (TALEN) or a Cas protein, without being limited thereto. The Cas protein may be Cas1, Cas1B, Cas2, Cas3, Cas4, Cas5, Cas6, Cas7, Cas8, Cas9, Cas10, Cas12a, Cas12b, Cas12c, Cas12d, Cas12e, Cas12g, Cas12h, Cas12i, Cas12j, Cas13a, Cas13b, Cas13c, Cas13d, Cas14, Csy1, Csy2, Csy3, Cse1, Cse2, Csc1, Csc2, Csa5, Csn2, CsMT2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3 or Csf4 endonuclease, without being limited thereto.

The Cas protein may originate from a Cas protein ortholog-containing microorganism genus selected from the group consisting of *Corynebacter, Sutterella, Legionella, Treponema, Filifactor, Eubacterium, Streptococcus (Streptococcus pyogenes), Lactobacillus, Mycoplasma, Bacteroides, Flaviivola, Flavobacterium, Azospirillum, Gluconacetobacter, Neisseria, Roseburia, Parvibaculum, Staphylococcus* (*Staphylococcus aureus*), *Nitratifractor, Corynebacterium, and Campylobacter,* and may be a protein isolated from the microorganism genus or a recombinant protein.

### 5. Delivery vehicle

The present invention may include at least one delivery vehicle containing: a plurality of cleavaging agents that specifically recognize nucleic acid sequences comprising specific sequences, which are not present in normal sequences, among variant region sequences specific for cells having genomic sequence variation, or polynucleotides encoding the cleavaging agents; or a nuclease or a polynucleotide encoding the nuclease.

Regarding the delivery vehicle, a plurality of cleavaging agents or polynucleotides encoding the same, and a nuclease or a polynucleotide encoding the same may be located in the same or different delivery vehicle.

A representative side effect that occurs in selectively killing genetically mutated cells is the off-target effect. This side effect may occur when the DNA sequence of a normal cell is incorrectly recognized as indel of a genetically mutated cell and cleaved. In order to reduce such errors, it is preferable to increase the length of the indel sequence. CAS9 may usually be loaded with a guide RNA with a sequence length of 17 to 21 bp.

It is necessary to minimize the dose of the composition for inducing apoptosis in order to lower the off-target effect together with a side effect resulting from a large amount of intracellular transduction, that is, the toxic effect of the composition. Recently, with the development of gene therapy, various methods for introducing genes into cells have been developed. In addition, among the various methods for introducing genes, a method of introducing a gene using a viral vector without being harmful to the human body has been actively studied, and whether the use of the viral vector is toxic has also been actively studied. This study can also be achieved by reducing the number of enzymes to be introduced into cells. This means that, when the composition for induction is an RNP complex, the dose of the RNP complex should be minimized, or when the composition for induction comprises a vector for transformation, the dose of the vector should be minimized.
i) In the former case, if the nuclease of the RNP complex can express multiple cleavaging agents, the number of RNP complexes administered may be reduced. In particular, in the case of CAS12 and CASΦ, a plurality of guide RNAs can be inserted into one line, and thus a plurality of cleavaging agents can be included in one RNP complex.
ii) In the latter case, guide RNAs expressing a plurality of cleavaging agents may be included in the vector.

When the above-described method is used, the effect of reducing the toxic effect by reducing the dose of the composition for inducing apoptosis is obtained. In addition, according to the present invention, the cleavaging agents introduced into each cell are the same, and thus the uniformity of apoptotic action can be increased as described in detail below.

If a total of five cleavaging agents a, b, c, d, e and f are required for apoptosis (cell death), a nuclease (A) corresponding to each cleavaging agent must be introduced or transformed into all cells. Even if 5 nucleases are introduced into a certain cell, not all the cleavaging agents are introduced, which may not result in apoptosis. In this case, the average number of nucleases to be introduced into cells needs to be 5 or more to ensure apoptosis. If nucleases that recognize a, b, c, d, e and f as cleavaging agents are used, apoptosis will occur even if only one of the nucleases is introduced into the cell. Similarly, even if only one vector expressing five cleavaging agents is introduced into a cell without using five different vectors, the cell can be sufficiently killed. Therefore, according to the present invention, it is possible to effectively reduce toxicity by reducing the amount of nuclease introduced and expressed in a cell and to significantly improve the accuracy or uniformity of the apoptotic effect. The use of CAS12 or CASΦ is thought to increase the effects (Science (2020) 369:333).

In addition to viral delivery, a method of delivering RNP complexes using nanoparticles may be used to induce apoptosis. When nanoparticles are used, a peptide that specifically binds to cancer cells may be bound to the outside of the nanoparticles, thereby minimizing side effects that may occur on normal cells. As the peptide, an RPARPAR peptide that specifically targets cancer cells may be used (Sugahara et al., Cancer Cell (2009) 16:510-520).

The delivery vehicle may be, for example, a viral delivery vehicle. The viral delivery vehicle may include a viral vector. The viral vector may include, for example, an adeno-associated viral vector (AAV), an adenoviral vector (AdV), a lentiviral vector (LV) or a retroviral vector (RV)m without being limited thereto.

The delivery vehicle may be, for example, a non-viral delivery vehicle. The non-viral delivery vehicle may include, for example, an episomal vector comprising a viral replicon. Non-viral delivery may include delivery in mRNA form, and delivery by RNP (ribonucleoprotein) or carrier. For delivery in mRNA form, a synthetic modified mRNA or a self-replicating RNA (srRNA) may be included. The carrier may include nanoparticles, a cell-penetrating peptide (CPP) or a polymer.

The delivery vehicle usable in the present invention may include, for example, a vector. The term "vector" refers to a nucleic acid molecule capable of transporting another nucleic acid to which it has been linked. Vectors include, for example, nucleic acid molecules that are single-stranded, double-stranded, or partially double-stranded; nucleic acid molecules that comprise one or more free ends, no free ends (e.g. circular); nucleic acid molecules that comprise DNA, RNA, or both; and other varieties of polynucleotides known in the art.

For example, the vector is a "plasmid," which refers to a circular double stranded DNA loop into which additional DNA segments can be inserted, such as by standard molecular cloning techniques.

The virus delivery vehicle usable in the present invention may include, for example, a viral vector. The viral vector may, for example, be an adeno-associated viral vector (AAV), an adenoviral vector (AdV), a lentiviral vector (LV) or a retroviral vector (RV) (Acta Biochim Pol. 2005, 52(2): 285-291., and Biomolecules. 2020 Jun, 10(6): 839.).

In the case of the viral vector, virally-derived DNA or RNA sequences are present in the vector for packaging into a virus (e.g. retroviruses, replication defective retroviruses, adenoviruses, replication defective adenoviruses, and adeno-associated viruses). Viral vectors also include polynucleotides carried by a virus for transfection into a host cell.

Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome.

Moreover, certain vectors are capable of directing the expression of genes to which they are operatively-linked. Such vectors are referred to herein as "expression vectors." Common expression vectors of utility in recombinant DNA techniques are often in the form of plasmids.

Recombinant expression vectors may comprise a nucleic acid in a form suitable for expression of the nucleic acid in a host cell. This means that the recombinant expression vectors include one or more regulatory elements, which may be selected on the basis of the host cells to be used for expression, that is operatively-linked to the nucleic acid sequence to be expressed.

Within a recombinant expression vector, "operably linked" is intended to mean that the nucleotide sequence of interest is linked to the regulatory element(s) in a manner that allows for expression of the nucleotide sequence (e.g. in an *in vitro* transcription/translation system or in a host cell when the vector is introduced into the host cell).

The term "regulatory element" is intended to include promoters, enhancers, internal ribosomal entry sites (IRES), and other expression control elements (e.g. transcription termination signals, such as polyadenylation signals and poly-U sequences). Regulatory elements include those that direct constitutive expression of a nucleotide sequence in many types of host cell and those that direct expression of the nucleotide sequence only in certain host cells (e.g., tissue-specific regulatory sequences). A tissue-specific promoter may direct expression primarily in a desired tissue of interest, such as muscle, neuron, bone, skin, blood, specific organs (e.g. liver, pancreas), or particular cell types (e.g. lymphocytes). Regulatory elements may also direct expression in a temporal-dependent manner, such as in a cell-cycle dependent or developmental stage-dependent manner, which may or may not also be tissue or cell-type specific.

In some embodiments, a vector may comprise one or more pol III promoters, one or more pol II promoters, one or more pol I promoters, or combinations thereof. Examples of pol III promoters include, but are not limited to, U6 and H1 promoters. Examples of pol II promoters include, but are not limited to, the retroviral Rous sarcoma virus (RSV) LTR promoter (optionally with the RSV enhancer), the cytomegalovirus (CMV) promoter (optionally with the CMV enhancer) [see, e.g., Boshart et al, Cell, 41:521-530 (1985)], the SV40 promoter, the dihydrofolate reductase promoter, the β-actin promoter, the phosphoglycerol kinase (PGK) promoter, and the EF1α promoter.

Also encompassed by the term "regulatory element" are enhancer elements, such as WPRE; CMV enhancers; the R-U5' segment in LTR of HTLV-I; SV40 enhancer; and the intron sequence between exons 2 and 3 of rabbit β-globin. It will be appreciated by those skilled in the art that the design of the expression vector can depend on such factors as the choice of the host cell to be transformed, the level of expression desired, etc. A vector can be introduced into host cells to thereby produce transcripts, proteins, or peptides, including fusion proteins or peptides, encoded by nucleic acids as described herein (e.g., clustered regularly interspersed short palindromic repeats (CRISPR) transcripts, proteins, enzymes, mutant forms thereof, fusion proteins thereof, etc.). Advantageous vectors include lentiviruses and adeno-associated viruses, and types of such vectors can also be selected for targeting particular types of cells.

The terms "polynucleotide", "nucleotide", "nucleotide sequence", "nucleic acid" and "oligonucleotide" are used interchangeably. They refer to a polymeric form of nucleotides of any length, either deoxyribonucleotides or ribonucleotides, or analogs thereof. Polynucleotides may have any three dimensional structure, and may perform any function, known or unknown. A polynucleotide may comprise one or more modified nucleotides, such as methylated nucleotides and nucleotide analogs. If present, modifications to the nucleotide structure may be imparted before or after assembly of the polymer.

Vectors can be designed for expression of the nuclease (e.g., nucleic acid transcripts, proteins, or enzymes) and cleavaging agents according to the present invention in prokaryotic or eukaryotic cells. For example, nuclease and cleavaging agent transcripts can be expressed in bacterial cells such as *Escherichia coli,* insect cells (using baculovirus expression vectors), yeast cells, or mammalian cells. In some embodiments, the recombinant expression vector can be transcribed and translated in vitro, for example using T7 promoter regulatory sequences and T7 polymerase.

Vectors may be introduced and propagated in a prokaryote or prokaryotic cell. In some embodiments, a prokaryote is used to amplify copies of a vector to be introduced into a eukaryotic cell or as an intermediate vector in the production of a vector to be introduced into a eukaryotic cell (e.g. amplifying a plasmid as part of a viral vector packaging system) . A prokaryote may be used to amplify copies of a vector and express one or more nucleic acids, such as to provide a source of one or more proteins for delivery to a host cell or host organism. Expression of proteins in prokaryotes may be carried out in *Escherichia coli* with vectors containing constitutive or inducible promoters.

The vector may be delivered *in vivo* or into a cell through electroporation, lipofection, viral vectors, or nanoparticles, as well as a protein translocation domain (PTD) fusion protein method.

### A. Viral Vector

AAV may be used for *in vivo* gene editing in cells and animal models. ITRs (inverted terminal repeats) may be added. ITRs may be present flanking the AAV genome *and*/*or the transgene of interest* and serve as origins of replication. Rep and/or cap proteins are transcribed to form capsids so that the AAV genome for delivery to the target cell can be encapsulated. Surface receptors on these capsids determine the AAV serotype.

AdV contains double-stranded DNA as genetic material and may be used for *in vivo* gene editing in cells and animal models. LV may be used only *in vitro* and *ex vivo* because it has a high likelihood of developing a tumor when integrated into an oncogene due to random integration and insertional mutagenesis (Popescu N.C., Zimonjic D., DiPaolo J.A. Viral integration, fragile sites, and proto-oncogenes in human neoplasia. Hum. Genet. 1990,84:383-386.). A clinical attempt to treat disease through gene delivery using RV was reported (J. Clin. Investig. 2008,118:3132-3142.).

### (1) AAV

AAV contains ssDNA as genetic material and has a packaging capacity of about 4.7 kb. AAV is the most widely used for *in vivo* gene editing in cells and animal models (Viruses 2019, 11, 28, Current Gene Therapy, 2008, 8, 147-161) .

The genome of AAV is linear single-stranded DNA with a length of about 4.7 kb. At both ends of the AAV genome, there are hairpin structures called inverted terminal repeats (ITRs) . ITRs are involved in replication of the AAV genome and packaging of AAV particles.

AAV-based vectors retain the viral function for transfection into target cells and localization of the genome to the nucleus, but lack the ability of AAV to replicate and produce progeny. AAV is not known to be immunogenic in humans, and thus AAV vectors are less immunogenic than other viral delivery systems. The AAV genome mostly remains in episomal form, and can be partially integrated into mitochondrial DNA and AAVS1 chromosome 19 that do not produce cancer. It can stably express transgenes in non-dividing cells for a long period of time.

Various serotypes suitable for CRISPR editing exist in specific tissues. AAV vectors may comprise a serotype of AAV1, AAV2, AAV3, AAV4, AAV5, AAV6, AAV7, AAV8, or AAV9.

| Tissue | Optimal serotype |
|---|---|
| CNS | AAV1, AAV2, AAV4, AAV5, AAV8, AAV9 |
| Heart | AAV1, AAV8, AAV9 |
| Kidney | AAV2 |
| Liver | AAV7, AAV8, AAV9 |
| Lung | AAV4, AAV5, AAV6, AAV9 |
| Pancreas | AAV8 |

| | |
|---|---|
| Photoreceptor cells | AAV2, AAV5, AAV8 |
| RPE (Retinal Pigment Epithelium) | AAV1, AAV2, AAV4, AAV5, AAV8 |
| Skeletal muscle | AAV1, AAV6, AAV7, AAV8, AAV9 |

Since AAV has a packaging capacity of about 4.7 kb, it is difficult to package transgene-coding genes therein. Attempts have been made to find a method which is applicable when nucleases with large sizes are used, in consideration of the packaging limitations of the delivery system AAV which is most widely used in gene therapy.

Dual or more AAV systems can be applied for efficient delivery of the composition according to the present invention. Methods for manufacturing dual or more AAV systems may include, for example, construction of a fragmented vector, an overlapping vector, a trans-splicing vector or a hybrid vector (Viruses 2019, 11, 28).

A fragmented vector can be constructed by truncating a transgene at a specific point and then packaging each fragment in a separate AAV, and the complete transgene can be produced through HR (homologous recombination) of the overlapping transgenes. An overlapping vector can be produced by a method of inducing HR through transgenes containing a specified overlap, and when dual AAV is transfected into cells by introducing ITRs (inverted terminal repeats) at the end of the transgene, the ITRs may be assembled and HR may happen. A trans-splicing vector contains no overlap, but it can be constructed using transgene fragments separated by a splice donor/splice acceptor site, and the transgene fragments can be linked by the splicing mechanism of the genome. A hybrid vector can be produced by combining overlapping and trans-splicing functions, including an overlap site and a splice donor/splice acceptor site, and the target transgene product may be produced by co-infection with a dual vector, splicing by the splice donor/splice acceptor site, and HR by the overlapping site.

The CRISPR/Cas system can be applied when a nuclease is used for efficient application to the AAV system. The Cas may be, for example, split Cas9, SaCas9, CjCas9, Cas12a or Cas12j.

Split Cas9 targets *Streptococcus* pyogenes-derived Cas9 (SpCas9) having 1,368 amino acids and 4.10 kbp. Two split SpCas9s can be packaged in two AAV vectors, respectively. However, there is a disadvantage that the activity of SpCas9 may be lowered compared to the original activity, and the delivery efficiency of two AAV vectors *in vivo* may be lower than that of a single AAV vector.

SaCas9 is a *Staphylococcus aureus* Cas9 (SaCas9) having 1,053 amino acids and 3.16 kbp. SaCas9 together with sgRNA can be packaged into a single AAV. However, there may be insufficient space for packaging SaCas9 together with two or more sgRNAs. The PAM (protospacer-adjacent motif) sequence 5'-NNGRRT-3' which is recognized by SaCas9 exists at a lower frequency than 5'-NGG-3', which is the PAM sequence of SpCas9, and thus there may be limitations in target site setting.

CjCas9 is *Campylobacter* jejuni-derived Cas9 having 984 amino acids and 2.95 kbp, and may recognize 5'-NNNNACAC-3' as PAM.

Cas12a (type-V Cpf1) may be classified into one from *Lachnospiraceae bacterium* ND2006 (LbCpf1) and one from *Acidaminococcus* sp. BV3L6 (AsCpf1). Cas12a includes a 3.9 kb T nucleotide-rich PAM site (5'-TTTV), self-catalysis of crRNA maturation, and staggered dsDNA cut, and genes can be inserted therein through non-homologous end joining (NHEJ). Cas12a (type-V Cpf1) is known to have a higher targeting specificity than SpCas9 in mammalian cells.

In some embodiments, in order to minimize the transfection rate difference and expression rate difference between vectors, a nuclease-encoding polynucleotide and a plurality of guide RNAs as cleavaging agents may be cloned into one vector and transfected *in vivo* or into a cell. In this case, Cpf1 (Cas12a) may be included as the nuclease. In this case, a single promoter can be used to induce crRNA expression.

Cancer-specific INsertion-DELetion targeting Apoptosis (CINDELA) can be induced by Cas12a. Thereby, the number of vectors can be minimized enough to kill cancer cells. Cas12a can be used to target cancer-specific indels and generate DNA double-strand breaks, thereby inducing apoptosis.

Cas12a is a programmable DNA endonuclease guided by a single guide RNA. For CRISPR-Cas9, each sgRNA is usually expressed in an individual vector or in a multi-cassette vector requiring the promoter of each sgRNA. In contrast, multiplex genome editing via Cas12a can process individual crRNAs by Cas12a itself, allowing the guide RNA to be expressed as one transcript.

For example, multiple guide RNAs can be induced using a pCE048-SiT-Cas12a vector as a single vector. By minimizing the number of vectors, it is possible to improve the efficiency of cancer cell elimination and cancer-specific indel targeting apoptosis.

Cas12j is a single CasΦ of about 70 Kda, and the molecular weight thereof is half that of Cas9/Cas12a. Cas12j may recognize 5' TBN 3' PAM (where B is G, T or C), and can cleave crRNA-complementary DNA without tracrRNA. Based on these characteristics, Cas12j may provide a compact system.

International Patent Publication No. WO2016/176191 discloses a dual vector system for the treatment of genetic diseases. The vector system is an AAV vector, and may include an AAV vector including a Cas9 gene and an AAV vector including at least one sgRNA. In this case, with respect to the content ratio of the AAV vector including the Cas9 gene and the AAV vector including at least one sgRNA, the content of the AAV vector including the sgRNA may exceed the content of the AAV vector including the Cas9 gene. For example, the ratio of the AAV vector including the Cas9 gene to the AAV vector including at least one sgRNA may be about 1:3 to about 1:100, specifically about 1:10. It is described that the ratio of the nuclease (e.g., Cas9 or Cpf) to the donor template can be maintained even when the nuclease is additionally or alternatively supplied by a source other than the AAV vector, and this description may also be applied to the present invention.

In relation to the application of AAV, U.S. Patent No. 10577630 discloses a method of modifying mammals by editing a target sequence at a target genomic locus in mammalian hepatocytes through AAV particles containing one or more polynucleotides encoding a guide RNA and a CRISPR enzyme. U.S. Patent No. 10577630 discloses that mammals can be transformed by editing the *in vivo* target sequence, including a method for delivering AAV particles to target cells. U.S. Patent No. 10577630 discloses that, considering the packaging size of AAV, the CRISPR enzyme is SaCas9 so that the guide RNA and the CRISPR enzyme can be loaded at once. The disclosure is also applicable to the compositions according to the present invention and delivery thereof, and may be incorporated herein by reference.

### (2) AdV

AdV contains dsDNA as genetic material and has a packaging capacity of about 35 kbp. It has been verified that AdV can also be used for gene editing in cells and animal models. AdV-based vectors retain the viral function for transfection into target cells and localization of the genome to the nucleus, but lack the ability of AdV to replicate and produce AdV (Genes & Diseases Volume 4, Issue 2, June 2017, Pages 43-63).

AdV can be transfected into both dividing cells and non-dividing cells. Since AdV is not integrated into the cell genome and exists outside the chromosome, it can reduce the off-target effect in which unwanted variations other than the desired target site occur when the vector is delivered to a cell together with the composition according to the present invention.

Optimization attempts have been made to use AdV as a gene delivery vector in order to increase transduction efficiency and minimize side effects.

In the case of first-generation AdV, a replication-defective vector was produced by removing the viral gene E1. However, acute or chronic immune responses may still occur due to viral capsid and/or viral gene expression.

In the case of second-generation AdV, viral genes E2 and E4 were removed to control chronic immune response. Packaging capacity can be increased to about 8 kb by removing E2 and E4.

In the case of third-generation AdV, all viral genes can be removed and only ITR and encapsidation (ψ) related genes can be included. Packaging capacity may be increased to about 35 kb, and thus all of the nuclease and the plurality of cleavaging agents may be included in a single vector and delivered. In addition, the third-generation AdV vector may not induce chronic immune response.

It has been reported that CRISPR/Cas9 can be delivered using the third-generation Adv, high-capacity adenoviral vectors (HCAdVs) (Nature Scientific Reports volume 7, Article number: 17113 (2017)). Unlike the case of AAV, all components of the CRISPR/Cas9 system can be contained in a single vector. Constitutive or inducible expression of Cas9 may be induced and multiple gRNAs may be included. A CRISPR/Cas9-HCAdV targeting HPV 18 (human papillomavirus 18) oncogene E6, DMD (dystrophin gene causing Duchenne muscular dystrophy) and CCR5 (HIV co-receptor C-C chemokine receptor type 5) can be produced, a CRISPR/Cas9-HCAdV expression unit may be delivered to a target, and DNA DSBs may be induced at a desired target location. The above description of CRISPR/Cas9 is applicable to the composition of the present invention and delivery thereof and may be incorporated herein by reference.

### (3) LV

LV contains retrovirus ssRNA as genetic material and has a packaging capacity of about 8 kb. LV allows transfection of foreign genes into dividing cells without dilution (Cancer Gene Therapy pp 379-391).

Attempts have been made to remove the integrase activity of the LV in order to prevent the viral genome from being integrated and to maintain the ability to transport the transcription and pre-integration complex to the vicinity of the nucleus without being affected (IDLV (Integrase-deficient lentiviral vectors), Cell Rep. 2017; 20: 2980-2991). In addition, in relation to the CRISPR/Cas system delivery, there was an attempt to inactivate the Cas9 open reading frame (ORF) by co-expression with Cas9-targeting sgRNA after integration of functional CRISPR/Cas9 sequences into the host genome through two-lentivirus CRISPR/Cas9 delivery (Nat. Commun. 2017; 8: 15334). These attempts are also applicable to the composition according to the present invention and delivery thereof and may be incorporated herein by reference.

LV can be transfected into both dividing cells and non-dividing cells. Viral particles can be produced by transfection of three vectors, including a transfer vector, a packaging vector and an envelope vector, and then quantified/purified, thereby selecting cells to which a desired gene has been well delivered.

The transfer vector may include 5' LTR and 3' LTR, which are Tat (transactivator of transcription) binding sites, a packaging signal (ψ) and a transgene. The packaging vector may include a viral structural gene such as gag and/or pol from which the packaging signal (ψ) has been removed and in which enzymes such as capsid, reverse transcriptase, protease, and integrase are expressed. The vector may contain viral regulatory genes (tat and/or rev) such as Tat for inducing transcription and/or Rev for transporting mRNA. The envelope vector may include a viral env that is a viral envelope expression gene.

### (4) RV

Replication is initiated through provirus DNA, in which RNA carrying genetic information is converted into double-stranded DNA by reverse transcriptase. It is possible to introduce a foreign gene of about 8 kb. Viral particles can be prepared by introducing a plasmid into which a therapeutic gene including LTR has been introduced into a virus particle-forming cell line. The cell line supplies gag, pol, and env proteins (Genetic Vaccines and Therapy 2004, 2:9).

Viral particles can be produced by transfection of three vectors, including a transfer vector, a packaging vector and an envelope vector, and then quantified/purified, thereby selecting cells to which a desired gene has been well delivered.

The transfer vector may include 5' LTR and 3' LTR, which are Tat (transactivator of transcription) binding sites, and a transgene. 5' LTR and 3' LTR induce viral replication, host insertion, and viral gene expression. The packaging vector may include a viral structural gene such as gag and/or pol from which the packaging signal (ψ) has been removed and in which enzymes such as capsid, reverse transcriptase, protease, and integrase are expressed. The envelope vector may include a viral env that is a viral envelope expression gene.

### (5) Others

In addition, for example, as described in WO2017/223330, CRISPR/Cas may be delivered using the genome of a single-stranded RNA (ssRNA) virus.

LV and AAV have been used for cell transformation ex *vivo* and *in vivo,* but viral DNA-based replication carries the risk of undesirable genotoxicity or carcinogenesis of the host genome, and thus ssRNA viral vectors in a form different from conventional vectors are presented. Mononegavirales comprising a linear, single-stranded, negative, non-infectious RNA chain can be exemplified. Mononegavirales is replicated by producing 5 to 10 different mRNAs through polar sequential transcription and synthesizing the complete antigenome. For example, Mononegavirales may include Bornaviridae, Filoviridae, Nyamiviridae, Paramyxodiridae, or Rhabdoviridae. Bornaviridae includes bornavirus. Filovindae includes cuevavirus, ebolavirus and marburgvirus. Nyamiviridae includes nyavirus. Paramyxoviridae includes aquaparamyxovirus, avulavirus, feravirus, heniparvirus, morbillivirus, respirovirus, rubulavirus, pneumovirus, metapneumovirus and sendai virus. Rhabdoviridae includes alemndravirus, baiavirus, curiovirus, cytorhabdovirus, dichorhavirus, ephemerovirus, hapavirus, ledantevirus, lyssavirus, novirhabdovirus, nuclearhabdovirus, perhabdovirus, sawgravirus, sigmavirus, sprivivirus, tibrovirus, tupavirus, and vesiculovirus. In particular, in WO2017/223330, a CRISPR/Cas delivery system was constructed through the sendai virus. In this case, a self-cleaving ribozyme is applied to the gRNA of the CRISPR/Cas system so that the gRNA can be effectively cleaved/released when introduced into a host cell. Thereby, it was confirmed that the RNA transfection efficiency was increased. The disclosure of WO2017/223330 is also applicable to the composition according to the present invention and delivery thereof and may be incorporated herein by reference.

The vector dose to achieve a therapeutic effect, e.g., the dose in vector genomes/per kilogram of body weight (vg/kg), will vary based on several factors, such as (a) the route of administration, (b) the level of therapeutic gene expression required to achieve a therapeutic effect, (c) any host immune response to the vector, and (d) the stability of the protein expressed.

### B. Non-Viral Delivery vehicle

Non-viral delivery vehicle include, for example, episomal vectors, including Simian virus 40 (SV40) ori, bovine papilloma virus (BPV) ori or Epstein-Barr nuclear antigen (EBV) ori, a method for delivery in mRNA form, delivery by ribonucleoprotein (RNP), or delivery by carriers such as nanoparticles, CPP or polymers (Nature Reviews Genetics volume 15, pages 541-555(2014)).

### (1) Episomal Vector

An episomal vector containing a viral plasmid replicon may be constructed to deliver the composition according to the present invention. Available viral plasmid replicons may include, for example, Simian virus 40 (SV40), bovine papilloma virus (BPV) or Epstein-Barr nuclear antigen (EBV) replication origin (Ori). A plasmid vector including the replication origin (Ori) may be constructed. As the plasmid vector, an *Escherichia* coli-derived plasmid, an Actinomyces-derived plasmid, a *Bacillus subtilis-derived* plasmid or a yeast-derived plasmid may be used. The *Escherichia* coli-derived plasmid may include, for example, pBR322, pUC18, pUC19, pUC118, pUC119 or pBluescript. The Actinomyces-derived plasmid may include, for example, pIJ486, etc. The *Bacillus* subtilis-derived plasmid may include, for example, pUB110, pSH19, etc. The yeast-derived plasmid may include, for example, YEp13, YEp 24, Ycp50, etc.

For example, commercially available pCMV6-XL3 (OriGene Technologies Inc.), EGFP-C1 (Clontech), pGBT-9 (Clontech), pcDNAI (FUNAKOSHI), pcDM8 (FUNAKOSHI), pAGE107 (Cytotechnology, 3,133, 1990), pCDM8 (Nature, 329, 840, 1987), pcDNAI/AmP (Invitrogen), pREP4 (Invitrogen), pAGE103 (J. Blochem., 101, 1307, 1987), pAGE210, etc. may be used as plasmids.

This vector containing a viral plasmid replicon is not integrated into the chromosome of the host cell. In some embodiments, the vector may contain a viral early gene, which is a product that activates the viral ori. This allows the episome to be well regulated and localized in the transfected host cell line.

Episomal vectors have the following advantages. The inserted gene of interest is interrupted or subjected to regulatory constraints, which often occur from integration into cellular DNA. The presence of the inserted heterologous gene does not lead to rearrangement or interruption of the cell's own important regions. Episomal vectors persist in multiple copies in the nucleus, resulting in amplification of the gene of interest whenever the necessary viral transacting factors are supplied. Episomal vectors have high transfection efficiency due to a stable transfection process, and since they have a plasmid structure that is replicable in prokaryotic as well as eukaryotic cells, conversion from one cell system to another is easy.

For example, since SV40 is not coupled with the control mechanism of the host cell and shows an independent replication pattern, each viral genome can be replicated several times during the cell cycle, and thus SV40 Ori has a very high copy number of 100 to 1000. Therefore, SV40 Ori can exhibit high-efficiency replication and persistence in the nucleus during cell division. SV40 Ori contains an oncoprotein, T-antigen, which limits its application to humans, and thus SV40 Ori can be used in a form in which the T-antigen has been removed or it can be expressed in trans in a host cell.

BPV Ori has a site of binding to the E1 protein encoded by the virus, and E1 is combined with a helicase with the help of E2 to help nuclear localization of intracellular genes. BPV Ori has a copy number of 50 to 150. Since the E2 protein can bind to the chromosome and exhibit a piggy-back effect, it can maintain gene nuclear localization and maintain stability.

In the case of EBNA Ori, in the latent stage of DNA replication after introduction into host cells, the EBV genome can be cloned into a plasmid having a copy number of 5 to 20 through oriP including FR (family of repeats) and DS (dyad symmetry) and EBNA1 (EBV nuclear antigen 1) capable of binding to each of FR and DS. DS-bound EBNA1 can gather host cell mechanisms necessary for DNA replication, and FR-bound EBNA1 can maintain gene nuclear localization through a piggy-back effect and maintain stability (Current Gene Therapy, 2008, 8, 147-161, Eur. J. Biochem. 267, 5665±5678 (2000)). The above-description of the episomal vector is also applicable to the present invention and delivery thereof and may be incorporated herein by reference.

In some embodiments, as disclosed in International Patent Publication No. WO2018/075235, the episomal vector may include an alphavirus replicon RNA. Alphaviruses have a positive single-stranded RNA genome enclosed in a nucleocapsid surrounded by an envelope containing a spike protein. Alphaviruses may include, for example, Sindbis Virus (SIN), Semliki Forest Virus (SFV), Ross River Virus (RRV), Venezuelan Equine Encephalitis Virus (VEEV), and Eastern Equine Encephalitis Virus (EEEV).

The episomal vector may comprise a modified replicon RNA comprising a modified 5'-UTR and lacking at least a portion of a nucleic acid sequence encoding a viral structural protein. The modified 5'-UTR may comprise one or more nucleotide substitutions. Thus, the episomal vector may not comprise a nucleic acid sequence encoding a viral structural protein. This nucleic acid molecule may be capable of integration into the host genome, episomal replication, or transient expression. The above description is also applicable to the composition according to the present invention and may be incorporated herein by reference.

### (2) Delivery in mRNA form

Compared to delivery via a vector containing DNA, delivery in mRNA form does not require the process of transcription into mRNA, and thus the initiation of gene editing may be quick. The possibility of transient protein expression is high. mRNA can be unstable compared to DNA and has a high probability of being degraded by RNAse.

Each of the nuclease and/or cleavaging agents in the composition according to the present invention may be delivered in mRNA form. The mRNA of the nuclease and/or the cleavaging agents may be chemically modified or delivered in the form of synthetic self-replicative RNA.

International Patent Publication No. WO2011/071931 discloses a modification method for intracellular delivery in the form of a single-stranded mRNA molecule. Modified nucleosides may be included. The following examples of the modified nucleosides are described: m⁵C (5-methylcytidine); m⁵U (5-methyluridine) ; m⁶A (N6-methyladenosine) ; s²U (2-thiouridine); ψ (pseudouridine); Um (2'-O-methyluridine); n¹A (1-methyladenosine); m²A (2-methyladenosine); Am (2'-O-methyladenosine); ms²mGA (2-methylthio-N6-methyladenosine); i^{G}A(~isopentenyladenosine); ms^{z}i6A (2-methyIthio-N⁶isopentenyladenosine); io⁶A (N⁶-(cis-hydroxyisopentenyl)adenosine); ms²io⁶A (2-methylthio-N⁶"(cis-hydroxyisopentenyl)adenosine); g⁶A (N⁶-glycinylcarbamoyladenosine); t⁶A (N⁶-threonylcarbamoyladenosine); ms²t⁶A (2-methylthio-N⁶-threonylcarbamoyladenosine); m⁶t⁵A (N⁶-methyl-N⁶-threonylcarbamoyladenosine) ; hn⁶A (N⁶hydroxynorvalylcarbamoyladenosine) ; ms²hn⁶A (2-methylthio-N⁶-hydroxynorvalyl carbamoyladenosine); Ar(p) (2'-O-ribosyladenosine(phosphate)); I (inosine); m¹I (1-methylinosine); m¹Im (1,2'-O-dimethylinosine); m³C (3-methylcytidine); Cm (2'-O-methylcytidine); S²C (2-thiocytidine); ac⁴C (N⁴-acetylcytidine); f⁵C (5-formylcytidine); m⁵Cm (5,2'-O-dimethylcytidine) ; ac⁴Cm (N⁴-acetyl-2'-O-methylcytidine); k²C (lysidine); m¹G (1-methylguanosine); m²G (N²-methylguanosine); m⁷G (7-methylguanosine); Gm (2'-O-methylguanosine); m²₂G (N²,N²-dimethylguanosine); m²Gm (N², 2'-O-dimethylguanosine); m²₂Gm (N²,N²,2'-O-trimethylguanosine); Gr(p) (2'-O-ribosylguanosine (phosphate)); yW (wybutosine); o₂yW (peroxywybutosine); OH_{y}W (hydroxywybutosine); OHyW* (undermodified hydroxywybutosine); imG (wyosine); mimG (methylwyosine); Q (queuosine); oQ (epoxyqueuosine); galQ (galactosyl-queuosine); manQ (mannosylqueuosine); preQo (7-cyano-7-deazaguanosine); preQ₁ (7-aminomethyl-7-deazaguanosine); G⁺ (archaeosine); D (dihydrouridine); m⁵Um (5,2'-O-dimethyluridine); S⁴U (4-thiouridine); m²s²U (5-methyl-2-thiouridine); s²Um (2-thio-2'-O-methyluridine); acp³U (3-(3-amino-3-carboxypropyl)uridine); ho⁵U (5-hydroxyuridine); mo⁵U (5-methoxyuridine); cmo⁵U (uridine 5-oxyacetic acid); mcmo⁵U (uridine 5-oxyacetic acid methyl ester); cmnm⁵U (5-carboxymethylaminomethyluridine); mchm⁵U (5-(carboxyhydroxymethyl) uridine methyl ester), mcm⁵U (5-methoxycarbonylmethyluridine), mcm⁵Um (5-methoxycarbonylmethyl-2'-O-methyluridine), mcm⁵s²U (5-methoxycarbonylmethyl-2-thiouridine), nm⁵s²U (5-aminomethyl-2-thiouridine), mnm⁵U (5-methylaminomethyluridine), mnm⁵s²U (5-methylaminomethyl-2-thiouridine), mnmse²U (5-methylaminomethyl-2-selenouridine), ncm⁵U (5-carbamoylmethyluridine), ncm⁵Um (5-carbamoylmethyl-2'-O-methyluridine), cmnm⁵U (5-carboxymethylaminomethyluridine), cmnm⁵Um (5-carboxymethylaminomethyl-2'-methyluridine), cmnm⁵s²U (5-carboxymethylaminomethyl-2-thiouridine), m⁶₂A (N⁶,N⁶-dimethyl adenosine), Im (2'-O- methylinosine), m⁴C (N⁴-methylcytidine), m⁴Cm (N⁴,2'-O-dimethylcytidine), hm⁵C (5-hydroxymethylcytidine), m³U (3-methyluridine), cm⁵U (5-carboxymethyluridine), m⁶Am (N⁶,2'-O-dimethyl adenosine), m⁶₂Am (N⁶,N⁶,2'-O-trimethyladenosine), m^{2,7}G (N²,7-dimethylguanosine), m^{2,2,7}G (N²,N²,7-trimethylguanosine), m³Um (3,2'-O-dimethyluridine), m⁵D (5-methyldihydrouridine), f⁵Cm (5-formyl-2'-O-methylcytidine), m¹Gm (1,2'-O-dimethylguanosine), m¹Am (1,2'-O-dimethyl adenosine), τm⁵U (5-taurinomethyluridine), τm5s2U (5- taurinomethyl-2-thiouridine), imG-14 (4-dimethylwyosine), imG2 (isowyosine), or ac⁶A (N⁶-acetyl adenosine). The above disclosure of WO2011/071931 is applicable to the composition of the present invention and delivery thereof.

The nuclease and/or cleavaging agents may be delivered in the form of synthetic self-replicative RNA (Cell Stem Cell. 2013 Aug 1, 13(2): 10). The synthetic self-replicative RNA is unlikely to be packaged due to its non-infectious nature and may contain an RNA replicon of the Venezuelan Equine Encephalitis (VEE) virus that is capable of self-replicating. The synthetic self-replicative RNA may include nsP1 to nsP4 encoding the RNA replicon. In some embodiments, 5'-cap and poly(A) tail may be added to the mRNA.

As disclosed in US Patent No. 10370646, a structure having an arrangement of (VEE RNA replicases)-(promoter)-(GOI)-(IRES or optional promoter)-(optional selectable marker)-(VEE 3'UTR and polyA tail)-(optional selectable marker)-promoter (from 5' to 3') may be prepared, thereby preparing a synthetic self-replicative RNA comprising a self-replicating RNA replicon comprising the self-replicating backbone of alphavirus. The above disclosure is applicable to the composition of the present invention and delivery thereof.

It is possible to use methods capable of delivering mRNA molecules into cells *in vitro* or *in vivo,* including a method of delivering the mRNA into cells or a method of delivering the mRNA *in vivo* into cells in an organism such as a human or animal. For example, mRNA molecules may be delivered to cells by using lipids (e.g., liposomes, micelles, etc.), nanoparticles, nanotubes, or cationic compounds (e.g., polyethyleneimine or PEI). In some embodiments, a biolistic method such as a gene gun or a biolistic particle delivery system may be used to deliver mRNA into cells.

However, when the nuclease is delivered in mRNA form, if the nuclease and the cleavaging agents in mRNA form are present in the same vector, the nuclease must be present in protein form for inducing apoptosis according to the present invention, and thus the delivered nuclease mRNA is translated into a protein, whereas the cleavaging agents can start to degrade during translation.

To solve this problem, it is possible to use a method of delaying the delivery of the cleavaging agents, or inducing chemical modification of the cleavaging agents, or introducing the cleavaging agents or polynucleotides encoding the same into a viral vector.

It is possible to deliver the cleavaging agents or polynucleotides encoding the same within about 6 hours after delivering the nuclease mRNA (Cell Res. 2017,27(3):440-443.). This delivery method may result in improvement in editing efficiency. Through chemical modification of the cleavaging agents or polynucleotides encoding the same, the stability of the cleavaging agents or polynucleotides encoding the same after delivery can be improved (Nat Biotechnol. 2017 Dec, 35(12): 1179-1187.). In some embodiments, the nuclease may be delivered in the form of a nuclease-encoding mRNA, and the cleavaging agents or polynucleotides encoding the same can be cloned into a vector and delivered. In this case, the cleaving factors that are easily degraded can be stably delivered.

Regarding chemical modification of the cleavaging agents, they can be delivered in the form of synthetic modified RNA (Stem Cells Int. 2019 Jun 19,2019:7641767.). Cap structure and/or poly A tail may be added to the mRNA terminus during *in vitro* transcription (IVT). In some embodiments, a modified nucleoside such as Pseudo-UTP (pseudouridine) or 5mCTP (5-methylcytidine) may be added. The stability of the mRNA and the protein translated therefrom can be increased. However, in the case of multiple daily mRNA transfections in mRNA form, the innate immune response is highly likely to be induced, which may induce cellular stress and severe cytotoxicity.

### (3) RNP

In order to induce apoptosis through a vector, the following process must be performed: A plasmid containing a polynucleotide encoding a composition containing a nuclease and cleavaging agents according to the present invention is transfected into a cell, and the plasmid moves to the nucleus in the cell to start transcription, and then the mRNA for the nuclease moves out of the nucleus and is translated into a protein. The cleavaging agent RNAs are transcribed from the plasmid and move out of the nucleus. The nuclease and the cleavaging agent RNAs combine to form ribonucleoproteins (RNPs), which are then transported back to the nucleus.

However, the pre-formed RNPs can be introduced into a cell to initiate targeted genomic DNA editing. This type of RNP delivery can reduce the off-target effect compared to vector delivery, and has the advantage of being able to deliver the nuclease and cleavaging agent RNAs directly to the nucleus in a cell (Scientific Reports volume 8, Article number: 14355 (2018)).

For the formation of RNP (ribonucleoprotein), each of the nuclease and the cleavaging agents may be synthesized and purified to form RNPs. In some embodiments, CL7-tagged nuclease and cleavaging agent RNAs may be directly expressed through a plasmid, and then RNPs assembled in a self-assembly form may be obtained by purification (Curr Protoc Mol Biol 2017 Oct 2,120:31.10.1-31.10.19).

International Patent Publication No. WO2019/089884 proposes, as a method of modifying the expression of TGFBR2 gene in cells, a genome editing system including gRNA and RNA-guided nuclease, in addition to a vector containing gRNA and a RNA-guided nuclease-encoding polynucleotide. According to WO2019/089884, gRNA and RNA-guided nuclease may comprise a ribonucleoprotein (RNP) complex, and may comprise two or more different ribonucleoprotein (RNP) complexes, and may comprise gRNAs having different target domains.

In some embodiments, as disclosed in Korean Patent No. 2107735, a pre-assembled Cas protein-guide RNA RNP (ribonucleoprotein) obtained by mixing guide RNA and Cas protein *in vitro* may be used for one or more intrinsic genetic modifications (deletion or introduction). In KR2107735, a pre-assembled Cas protein-guide RNA RNP was applied to delete or introduce one or more plant endogenous genes in the plant protoplast, but may also be applied for disease treatment in humans as in the present invention.

Specifically, as disclosed in US Patent No. 9637739 that describes a method for modifying a target DNA, comprising assembling Cas9 protein and gRNA *in vitro* under conditions for forming a complex containing Cas9 protein and gRNA, and bringing the complex containing Cas9 protein and gRNA into contact with target DNA, even in the present invention, apoptosis can be induced by assembling a nuclease and cleaving factors *in vitro* and treating cells with the RNP (ribonucleoprotein) complex comprising the nuclease and the cleavaging agents.

As disclosed in U.S. Patent No. 10626419 which describes a method for targeting and binding target DNA, comprising bringing the complex containing Cas9 protein and gRNA into contact with the target DNA, even in the present invention, it is possible to form a complex of a nuclease and cleavaging agents and allow the complex to bind to the target DNA, thereby targeting the target DNA.

### (4) Delivery by carrier

Each of mRNA encoding the nuclease and/or the cleavaging agent RNAs may be prepared individually or in the form of ribonucleoprotein (RNP) and delivered through a carrier delivery system. The carrier may include, for example, cell penetrating peptides (CPPs), nanoparticles, zeolitic imidazole frameworks (ZIFs), RNA aptamer-streptavidin, or a polymer.

CPPs are short peptides that facilitate cellular uptake of a variety of molecular cargoes (from nanoscale particles to small chemical molecules and large fragments of DNA). The cargo may include a nuclease or a polynucleotide encoding the same and/or cleavaging agents or polynucleotides encoding the same. A complex comprising a cell-penetrating peptide and a nuclease or a polynucleotide encoding the same and/or cleavaging agents encoding the same or polynucleotides encoding the same can be prepared. Specifically, it may include a nuclease conjugated with a cell-penetrating peptide or a polynucleotide encoding the same and cleavaging agents complexed with a cell penetrating peptide or polynucleotides encoding the same. A cell-penetrating peptide and a nuclease or a polynucleotide encoding the same and/or cleavaging agents or polynucleotides encoding the same may be assembled together through chemical covalent bonds or non-covalent interactions. A nuclease or a polynucleotide encoding the same is conjugated to CPP via a thioester bond, whereas cleavaging agents or polynucleotides encoding the same are complexed with CPP to form condensed positively charged particles. Simultaneous and sequential treatment of human cells, including embryonic stem cells, dermal fibroblasts, HEK293T cells, HeLa cells, and embryonic carcinoma cells, leads to efficient gene disruption with reduced off-target mutations relative to plasmid transfections (Genome Res. 2014, 24:1020-1027.) .

In US Patent No. 10584322, in addition to the guide polynucleotide and Cas endonuclease, a cell-penetrating peptide (CPP) is used to change the target position, and is applicable to the present invention. The guide polynucleotide, Cas endonuclease, and CPP may be linked covalently or non-covalently to form a guide polynucleotide/Cas endonuclease-CPP complex. Thereby, the complex can pass through the cell membrane or cell wall of a microbial cell, and Cas endonuclease can induce a double-strand break at the target position in the genome of the microbial cell.

Regarding nanoparticles, the composition according to the present invention may be delivered via polymer nanoparticles, metal nanoparticles, metal/inorganic nanoparticles, or lipid nanoparticles.

The polymer nanoparticles may be, for example, DNA nanoclews, yarn-like DNA nanoparticles synthesized by rolling circle amplification. DNA nanoclews, yarn-like DNA nanoparticles, are loaded with Cas9/sgRNA and coated with PEI to improve endosomal escape. This complex binds to the cell membrane, is internalized, and then translocates into the nucleus through endosomal escape, resulting in simultaneous delivery of Cas9 protein and single guide RNA (Angew Chem Int Ed Engl. 2015 Oct 5; 54(41) 12029-12033). This complex is applicable to delivery of the composition according to the present invention and is incorporated herein by reference.

As another example, an RNP of a Cas9 protein and a single guide RNA may be coated with a mixture of cationic and anionic acrylate monomers through electrostatic interactions. An imidazole-containing monomer, a GSH-degradable crosslinker, acrylate mPEG, and acrylate PEG-conjugated ligands can also be attached to the RNP surface. *In situ* free-radical polymerization may be initiated to form a glutathione (GSH)-cleavable nanocapsule around RNP, and the nanocapsule may be crosslinked with a GSH-cleavable linker, N,N'-bis(acryloyl)cystamine, which can be degraded in the cytosol (Nature Nanotechnology volume 14, pages 974-980(2019)). This example s applicable to delivery of the composition according to the present invention and is incorporated herein by reference.

Regarding the metal nanoparticles, Cas9 ribonucleoprotein and donor DNA can be delivered to cells by linking gold particles to DNA and forming a complex with a cationic endosomal disruptive polymer (Nature Biomedical Engineering volume 1, pages 889-901(2017)). The endosomal disruptive polymer may be polyethylene imine, poly(arginine), poly(lysine), poly(histidine), poly-[2-{(2-aminoethyl)amino}-ethyl-aspartamide] (pAsp(DET)), a block copolymer of poly(ethylene glycol) (PEG) and poly(arginine), a block copolymer of PEG and poly(lysine), or a block copolymer of PEG and poly{N-[N-(2-aminoethyl)-2-aminoethyl]aspartamide} (PEG-pAsp(DET)).

In another example, magnetic nanoparticles and a recombinant baculoviral vector can form a complex which may be activated locally through a magnetic field to allow CRISPR-Cas9 mediated gene editing (Nature Biomedical Engineering volume 3, pages126-136 (2019)). This example is applicable to delivery of the composition according to the present invention and is incorporated herein by reference.

In another example, a photolabile semiconductor polymer nanotransducer (pSPN) can be used as a gene vector to deliver the CRISPR/Cas9 plasmid to cells, and can be used as a light regulator to remotely activate gene editing. pSPN may comprise a single oxygen (¹O₂) generating backbone grafted with polyethylenimine brushes via ¹O₂-cleavable linkers. Near-infrared (NIR) light irradiation spontaneously triggers the cleavage of gene vectors from pSPN, resulting in the release of CRISPR/Cas9 plasmids and subsequent gene editing (Angew Chem Int Ed Engl 2019 Dec 9; 58 (50) 18197-18201) . This example is applicable to delivery of the composition according to the present invention and is incorporated herein by reference.

Regarding metal/inorganic nanoparticles, for example, CRISPR/Cas9 can be encapsulated through the zeolitic imidazolate framework-8 (ZIF-8), and the expression of the desired target gene can be altered through efficient endosomal escape (Advanced Therapeutics Volume2, Issue4 April 2019) . This method is applicable to delivery of the composition according to the present invention and is incorporated herein by reference.

In another example, gold particles modified with arginine can be used. Arginine-modified gold particles can be assembled with a nuclease or a polynucleotide encoding the same and/or cleavaging agents or polynucleotides encoding the same. Thereby, the complex fuses with the membrane of the target cell and moves into the cytoplasm (ACS Nano. 2017, 11:2452-2458). This method is applicable to the composition according to the present invention and delivery thereof.

Negatively charged CRISPR RNPs can be encapsulated with positively charged nanoscale ZIFs via zeolitic imidazole frameworks (ZIFs). Endosomal escape of RNPs can be improved by such ZIFs (J Am Chem Soc. 2018, 140:143-146). This method is applicable to the composition according to the present invention and delivery thereof.

RNA aptamer-streptavidin, for example, Simplex (modular RNA aptamer-streptavidin strategy) can be used. For example, a 60-nucleotide S1m aptamer that has a strong non-covalent interaction with streptavidin can be added to the 3' end of sgRNAs. Thereafter, a complex with streptavidin and Cas9 protein may be formed, and Slmplexes can be delivered into cells. In some embodiments, biotinylated single-stranded oligodeoxynucleotides (ssODNs) with high affinity to streptavidin can be bound to a nucleic acid donor template. It was confirmed that nucleic acid could be precisely modified through the complex having this structure. The complex may exhibit increased gene editing efficiency compared to standard sgRNA RNP (Nat Commun. 2017, 8:1711.). This complex is applicable to the composition of the present invention and delivery thereof.

Negatively charged DNA or nucleic acid constituting CRISPR/Cas9 can combine with cationic substances to form nanoparticles, which can penetrate cells through receptor-mediated endocytosis or phagocytosis. For example, US Patent No. 10682410 discloses technology for delivering a nuclease into cells by using a negatively charged Cas9 protein-RNA, obtained by combining a positively charged Cas9 protein and a strongly negatively charged guide RNA together, and a supercharged protein (e.g., a positively supercharged protein), a cationic polymer, or a cationic lipid, and this technology is applicable to the present invention. The nuclease may be introduced into cells in contact with the supercharged protein, cationic polymer or cationic lipid. For delivery to the target cell, the Cas9 protein provides an anionic charge and is associated with a supercharged GFP (e.g., +36 GFP or -30 GFP) as a supercharged fluorescent protein. Endosomes may be favorably collapsed by the supercharged protein and endosomal escape ability can be increased. The above disclosure of US Patent No. 10682410 is applicable to the composition of the present invention and delivery thereof.

In the case of the cationic polymer, a Cas9:gRNA complex can be bound to the cationic polymer. Examples of the cationic polymer include polyallylamine (PAH); polyethyleneimine (PEI); poly(L-lysine) (PLL); poly(L-arginine) (PLA); polyvinylamine homo- or copolymers; poly(vinylbenzyl-tri-C1-C4-alkylammonium salt); polymers of aliphatic or araliphatic dihalides and aliphatic N,N,N',N'-tetra-C1-C4-alkyl-alkylenediamines; poly(vinylpyridine) or poly(vinylpyridinium salt); poly(N,N-diallyl-N,N-di-C1-C4-alkyl-ammonium halide); homo- or copolymers of quaternized di-C1-C4-alkyl-aminoethyl acrylates or methacrylates; POLYQUAD^{™}; polyaminoamide, etc.

The cationic lipids may include a cationic liposome preparation. The lipid bilayer of the liposome may protect the encapsulated nucleic acid from degradation and can prevent specific neutralization by antibodies capable of binding to the nucleic acid. During endosomal maturation, the endosomal membrane and the liposome are fused, allowing efficient endosomal escape of cationic lipid-nuclease. The Cas9:gRNA complex can be encapsulated by the cationic lipid. Examples of the cationic lipid include polyethylenimine, poly(amidoamine) (PAMAM) starburst dendrimers, Lipofectin^{®} (a combination of DOTMA and DOPE), Lipofectase, LIPOFECTAMINE^{®} (e.g., LIPOFECTAMINE^{®} 2000, LIPOFECTAMINE^{®} 3000, LIPOFECTAMINE^{®} RNAiMAX, LIPOFECTAMINE^{®}), SAINT-RED (Synvolux Therapeutics, Groningen Netherlands), DOPE, Cytofectin (Gilead Sciences, Foster City, Calif.), and Eufectins (JBL, San Luis Obispo, Calif.). Exemplary cationic liposomes can be made from N-[1-(2,3-dioleoloxy)-propyl]-N,N,N-trimethylammonium chloride (DOTMA), N-[1-(2,3-dioleoloxy)-propyl]-N,N,N-trimethylammonium methylsulfate (DOTAP), 3β-[N--(N',N'-dimethylaminoethane)carbamoyl]cholesterol (DC-Chol), 2,3,-dioleyloxy-N-[2(sperminecarboxamido)ethyl]-N,N-dimethyl-1-propanaminium trifluoroacetate (DOSPA), 1,2-dimyristyloxypropyl-3-dimethyl-hydroxyethyl ammonium bromide; or dimethyldioctadecylammonium bromide (DDAB).

In the case of the polymer, for example, a flexible dendritic polymer and the CRISPR Cas system can be delivered into cells (Chem Sci. 2017, 8:2923-2930.). In addition, for example, the CRISPR Cas system can be delivered into cells via hybrid polymer microcarriers made of polypeptides and polysaccharides (Nanomedicine. 2018, 14:97-108.). Moreover, for example, the CRISPR Cas system can be delivered into cells via graphene oxide-poly(ethylene glycol)-polyethylenimine (GO-PEG-PEI) nanocarriers (Nanoscale. 2018, 10:1063-1071).

US Patent Publication No. 2019/0099381 discloses nanoparticles including, as a core, an RNP complex containing a Cas9 polypeptide and a guide RNA, and as a shell, a biodegradable crosslinked polymer. The biodegradable crosslinked polymer may include a polymer of imidazolyl acryloyl monomers, bisacryloyl disulfides, optionally PEG acryloyl monomers, cationic acryloyl monomers, anionic acryloyl monomers, cationic and anionic acryloyl monomers, or non-ionic acryloyl monomers. In this case, the mass ratio between the biodegradable crosslinked polymer and the RNP can be adjusted to a ratio of 0.4 to 3.5. Such nanoparticles can be applied to deliver the composition according to the present invention and are incorporated herein by reference.

Regarding lipid nanoparticles, the CRISPR Cas system can be delivered using liposomes as a carrier. Liposomes are spherical vesicular structures composed of a single or multiple lamellar lipid bilayers surrounding internal aqueous compartments and a relatively impermeable outer lipophilic phospholipid bilayer. Liposome preparations may mainly contain natural phospholipids and lipids, such as 1,2-distearolyl-sn-glycero-3-phosphatidyl choline (DSPC), sphingomyelin, phosphatidylcholine or monosialoganglioside. In some cases, cholesterol or 1,2-dioleoyl-sn-glycero-3-phosphoethanolamine (DOPE) may be added to the lipid membrane to resolve instability in plasma. Addition of cholesterol decreases rapid release of encapsulated bioactive compounds into plasma, or 1,2-dioleoyl-sn-glycero-3-phosphoethanolamine (DOPE) increases stability (Semple et al., Nature Biotechnology, Volume 28 Number 2 February 2010, pp. 172-177) .

US Patent No. 10363217 describes that a nanoliposome carrier comprising lecithin, cholesterol and metal chelating lipid can be used for delivery of Cas9 and guide RNA. Examples of the metal chelating lipid include 1,2-dioleoyl-sn-glycero-3-[(N-5-amino-1-carboxypentyl) iminodiacetic acid) succinyl] (nickel salt) ("DOGS-NTA-Ni"); 1,2-dimyristoyl-sn-glycero-3-phosphoethanolamine-N-diethylenetriamine-pentaacetic acid (gadolinium salt) ("DMPE-DTPA-Gd"); or 1,2-dimyristoyl-sn-glycero-3-phosphoethanolamine-N-diethylenetriamine-pentaacetic acid (copper salt) ("DMPE-DTPA-Cu"). Cas9 and guide RNA may form a complex with a cationic polymer, for example, poly-L-lysine, poly(amidoamine), poly[2-(N,N-dimethylamino)ethyl methacrylate], chitosan, poly-L-ornithine, cyclodextrin, histone, collagen, dextran, or polyethyleneimine (PEI), and the nanoliposome carrier may encapsulate and deliver the hybrid CRISPR complex.

In some cases, the CRISPR Cas system can be administered via liposomes, such as nucleic acid-lipid particles (SNALP). SNALP preparations may contain lipid 3-N-[(w-methoxypoly(ethylene glycol) 2000) carbamoyl]-1,2-dimyristyloxy-propylamine (PEG-C-DMA), 1,2-dilinoleyloxy-N,N-dimethyl-3-aminopropane (DLinDMA), 1,2-distearoyl-sn-glycero-3-phosphocholine (DSPC) and cholesterol at a mole percent ratio of 2:40:10: 48 (Zimmerman et al., Nature Letters, Vol. 441, 4 May 2006). In addition, SNALP may comprise synthetic cholesterol, dipalmitoylphosphatidylcholine, 3-N-[(w-methoxy poly(ethylene glycol)2000)carbamoyl]-1,2-dimyrestyloxypropylamine, and cationic 1,2-dilinoleyloxy-3-N,N-dimethylaminopropane (Geisbert et al., Lancet 2010, 375: 1896-905). Furthermore, SNALP can be prepared by dissolving cationic lipid, DSPC, cholesterol and PEG-lipid, for example, at a molar ratio of 40:10:40:10, for example, in ethanol (Semple et al., Nature Biotechnology, Volume 28 Number 2 February 2010, pp. 172-177).

US Patent No. 10626393 discloses a pharmaceutical composition for delivering nucleic acid using lipid nanoparticles. The pharmaceutical composition comprises a first nucleic acid-lipid nanoparticle comprising gRNA and a second nucleic acid-lipid nanoparticle comprising a Cas9 protein-coding mRNA. When such lipid nanoparticles are used, it is possible to induce target-specific mutations by effectively delivering Cas9 protein-coding mRNA and gRNA *in vivo.* Examples of the cationic lipid include 1,2-dilinoleyloxy-N,N-dimethylaminopropane (DLinDMA), 1,2-dilinolenyloxy-N,N-dimethylaminopropane (DLenDMA), 1,2-di-γ-linolenyloxy-N,N-dimethylaminopropane (γ-DLenDMA; Compound (15)), 3-((6Z,9Z,28Z,31Z)-heptatriaconta-6,9,28,31-tetraen-19-yloxy)-N,N-dimethylpropan-1-amine (DLin-MP-DMA; Compound (8)), (6Z,9Z,28Z,31Z)-heptatriaconta-6,9,28,31-tetraen-19-yl 4-(dimethylamino)butanoate) (Compound (7)), (6Z,16Z)-12-((Z)-dec-4-enyl)docosa-6,16-dien-11-yl 5-(dimethylamino)pentanoate (Compound (13)), etc. The non-cationic lipids include phospholipids or cholesterol. Such lipid nanoparticles are applicable to the present invention.

In some cases, an aptamer containing an RNA binding domain target such as MS2 bacteriophage coat protein may be linked to the 3' or 5' end of the guide RNA to control expression of the target nucleic acid (see US Patent No. 10640789). A transcriptional activator or repressor including an RNA binding domain that binds to an RNA binding domain target may be expressed in cells. A transcriptional activator or repressor may be linked to a guide RNA, and expression of the target nucleic acid may be regulated through the transcriptional activator or repressor, thereby increasing the editing efficiency.

### (5) Delivery via exosomes

The term "exosomes" refers to cell-derived small (20-300 nm diameter, more preferably 40-200 nm diameter) vesicles which comprise a membrane surrounding an internal space and are produced from the cells by direct plasma membrane budding or fusion of the late endosome with the plasma membrane. Generally, production of exosomes does not result in the destruction of the producer cell. The exosome comprises lipid or fatty acid and polypeptide, and optionally comprises a payload (e.g. a therapeutic agent), a receiver (e.g. a targeting moiety), a polynucleotide (e.g. a nucleic acid, RNA, or DNA), a sugar (e.g. a simple sugar, polysaccharide, or glycan) or other molecules. The exosome can be derived from a producer cell, and isolated from the producer cell based on its size, density, biochemical parameters, or a combination thereof.

Such exosomes may be obtained by opening an extracellular vesicle or organelle by treatment with an aqueous solution having pH 9 to 14 to obtain a membrane, and removing the content from the extracellular vesicle or organelle, and then re-assembling the membrane to form a membrane vesicle.

The exosome may be mixed with a cargo and reassembled to form a membrane vesicle. The cargo may be a genome editing system. Examples of the genome editing system include, but are not limited to, a meganuclease system, a zinc finger nuclease (ZFN) system, a transcription activator-like effector nuclease (TALEN) system, and a clustered regularly interspaced short palindromic repeats (CRISPR) system. The CRISPR system may be a CRISPR-Cas9 system. The CRISPR-Cas9 system comprises a nucleotide sequence encoding a Cas9 protein, a nucleotide sequence encoding CRISPR RNA (crRNA) hybridized with a target sequence, and a nucleotide sequence encoding a trans-activating CRISPR RNA (tracrRNA). crRNA and tracrRNA may be fused to one guide RNA. The components of the CRISPR-Cas system may be located in the same vector or in different vectors. The CRISPR-Cas9 system may further comprise a nuclear localization signal (NLS). These exosomes are applicable to delivery of the composition according to the present invention, and may be introduced herein by reference.

The CRISPR-Cas9 system can be delivered via functionalized exosomes (Biomater. Sci. 2020 May 19;8(10):2966-2976.). GFP and a GFP nanobody can be bound to the exosome membrane protein CD63, and the Cas9 protein can be fused to the exosome. Thereby, it was confirmed that the target gene could be efficiently edited in recipient cells. The composition according to the present invention may be delivered via these exosomes, which are incorporated herein by reference.

As disclosed in WO2020/101740 and US Patent Publication No. US2020/0155703, therapeutic membrane vesicles mimic extracellular vesicles, such as exosomes, by their ability to interact with a recipient cell via their surface molecules. The therapeutic membrane vesicles are formed from membranes derived from extracellular vesicles or organelles. For instance, said membranes can be derived from extracellular vesicles or organelles by opening of such extracellular vesicles or organelles. The resulting emptied therapeutic membrane vesicles can thus be devoid of detrimental content that natural extracellular vesicles or organelles contain, including harmful endogenous molecules such as DNA or nuclear membrane components, or any unwanted RNA species, enzymes or other proteins, as well as infectious components such as viruses, virus components including virus genomic material and/or prions or similar infectious constituents.

The therapeutic membrane vesicles can be loaded with a therapeutic cargo. Therapeutic membrane vesicles comprising a loaded cargo mimic naturally-occurring extracellular vesicles in that they have an ability to interact with a recipient cell via their surface molecules, and to deliver their cargo to said recipient cell.

The therapeutic membrane vesicles can solve multiple problems with current extracellular vesicle therapeutics, e.g. by improving targeting efficacy, delivery, and increasing therapeutic cargo to a recipient cell/organ/object. Therapeutic membrane-vesicles could be said to mimic exosomes or any other extracellular vesicle by mimicking the latter in their ability to interact with a recipient cell via their surface molecules, and/or to deliver a therapeutic cargo to a recipient cell. At the same time, the therapeutic membrane vesicles differ from exosomes and other naturally-occurring extracellular vesicles in that the former can mitigate possible contamination with unwanted extracellular vesicles with possible negative side effects, as well as any detrimental content these can naturally contain.

As disclosed in European Patent Application Publication No. 3706796 and European Patent No. 3463465, the composition according to the present invention can be delivered through an exosome polypeptide, which is incorporated herein by reference.

"Exosomal polypeptide" comprises any polypeptide that enables transporting, trafficking or shuttling of any polypeptide that enables transporting, trafficking or shuttling of a polypeptide construct (which as abovementioned typically comprises at least one POI and at least one multimerization domain, but which may also include other types of polypeptide domains) to a vesicular structure, such as an exosome.

Examples of such exosomal sorting domains include CD9, CD53, CD63, CD81, CD54, CD50, FLOT1, FLOT2, CD49d, CD71, CD133, CD138, CD235a, ALIX, Syntenin-1, Syntenin-2, Lamp2b, TSPAN8, TSPAN14, CD37, CD82, CD151, CD231, CD102, NOTCH1, NOTCH2, NOTCH3, NOTCH4, DLL1, DLL4, JAG1, JAG2, CD49d/ITGA4, ITGB5, ITGB6, ITGB7, CD11a, CD11b, CD11c, CD18/ITGB2, CD41, CD49b, CD49c, CD49e, CD51, CD61, CD104, Fc receptors, interleukin receptors, immunoglobulins, MHC-I or MHC-II components, CD2, CD3 epsilon, CD3 zeta, CD13, CD18, CD19, CD30, CD34, CD36, CD40, CD40L, CD44, CD45, CD45RA, CD47, CD86, CD110, CD111, CD115, CD117, CD125, CD135, CD184, CD200, CD279, CD273, CD274, CD362, COL6A1, AGRN, EGFR, GAPDH, GLUR2, GLUR3, HLA-DM, HSPG2, L1CAM, LAMB1, LAMC1, LFA-1, LGALS3BP, Mac-1 alpha, Mac-1 beta, MFGE8, SLIT2, STX3, TCRA, TCRB, TCRD, TCRG, VTI1A, or VTI1B.

The exosome may include a nucleic acid and comprise single-stranded RNA or DNA, double-stranded RNA or DNA, or a chemically synthesized and/or chemically modified oligonucleotide.

The exosome includes a polypeptide of interest (PoI), wherein the polypeptide of interest is releasably attached to the exosomal polypeptide. The attachment of the polypeptide of interest (PoI) to the exosomal polypeptide is releasable, in order to enable efficient, non-obstructed loading and endogenously triggered release of the therapeutic polypeptide of interest into the EV. The releasable attachment between the Pol and the exosomal polypeptide is a feature mediated by an inventive release system which enables endogenously activatable release of the Pol inside the EV and/or subsequently inside a target cell or target tissue, to optimize loading and therapeutic activity. The release system is a polypeptide-based system that may be selected from the group comprising various releasable polypeptide interaction systems which may be activated or triggered without the need for exogenous stimuli (i.e. the release systems are typically triggered by endogenous activity within a cell or an EV, or essentially within any biological system), for instance a cis-cleaving polypeptide-based release system (e.g. based on inteins), a nuclear localization signal (NLS) - NLS binding protein (NLSBP)-based release system or release systems based on other protein domains. A monomeric light-induced cleavage-based release system may be utilized, where only a very short boost of light is utilized to start an endogenous proteolytic cleavage of a monomeric protein domain and release the PoI.

Korean Patent No. 1900465 describes an exosome containing a Cas9 protein using a photospecific binding protein. It discloses a technique of preparing exosomes so that a protein of interest is included therein by temporarily binding the protein of interest with a marker protein using a photospecific binding protein. For example, CIBN and CRY2, which are photospecific binding proteins, may be used. Specifically, genes encoding fusion proteins are introduced into exosome-producing cells so that the CIBN is expressed in a form fused with CD9, which is one of the marker proteins, and the CRY2 is expressed in a form fused with the protein of interest. The CIBN-CD9 fusion protein expressed in the exosome-producing cells is introduced into the exosome due to CD9. In this case, when the cell is irradiated with blue LED light, the CRY2 domain of the protein of interest-CRY2 fusion protein expressed in the exosome-producing cells is bound to the CIBN domain fused to CD9, and thus a reversibly induced fusion protein in the form of protein of interest-CRY2-CIBN-CD9 is formed, and this fusion protein is introduced into the exosome due to CD9. If the cell is no longer irradiated with the blue LED light after the exosome containing the protein of interest therein is produced as described above, the CIBN-CRY2 binding is released, and the protein of interest is present in a state in which it is not bound to the cell membrane of the exosome. As a result, the exosome containing the protein of interest may be produced.

As the photospecific protein, in addition to CRY (cryptochrome), CIB (cryptochrome-interacting basic-helix-loop-helix protein), CIBN (N-terminal domain of CIB), PhyB (phytochrome B), PIF (phytochrome interacting factor), FKF1 (Flavinbinding, Kelch repeat, F-box 1), GIGANTEA, or PHR (photolyase homologous region) may be used.

Since the protein of interest remains attached to the exosomal membrane and cannot be separated from the exosomal membrane, the protein of interest can be delivered to the target cell only when the exosome is fused to the cell membrane of the target cell. Even after the exosome is fused to the target cell as described above, the protein of interest remains bound to the exosome fused to the exosomal membrane, and thus there is a limitation in that the probability that the target protein exhibits its effect in the target cell is very low.

However, if the protein of interest is trapped inside the exosome without being bound to the membrane, when the exosome is endocytosed by the target cell and enters the cytoplasm, the protein of interest remains unattached to the exosome membrane. Thus, when the exosome is degraded, the protein of interest can be delivered to the cytoplasm of the target cell and can freely move within the cytoplasm of the target cell, so that the protein of interest can sufficiently exhibit physiological activity in the target cytoplasm.

As a result of expressing a fusion protein of CRY2 and mCherry and a fusion protein of CIB and CD9 in HEK293T cells, an immortal cell line that produces a large amount of exosomes, it was observed that the mCherry protein uniformly distributed in the cytoplasm was present in the cell membrane and endosome-like structures when irradiated with blue light. Similar results could be observed even when the fusion protein of FKF1 and mCherry and the fusion protein of GIGANTEA and CD9 were expressed in HEK293T cells. In addition, as a result of expressing the fusion protein of CRY2 and mCherry and the fusion protein of CIBN and CD9 in HEK293T and controlling the intensity of blue light, it was confirmed that, when the cells were irradiated with 20 to 50 µW of light, the mCherry protein captured in the exosomes exhibited the highest level. In addition, as a result of treating HT1080 cells, a different type of cells, with about 250 µg/ml of the exosomes produced and isolated from the cells, it was confirmed that the exosomes showed no particular cytotoxicity to the HT1080 cells, and the mCherry protein was delivered into the cytoplasm of the HT1080 cells. According to these results, exosomes can be applied for delivery of the composition according to the present invention and may be incorporated herein by reference.

### 6. Delivery method

### A. Cargoes

The composition according to the present invention comprises: a plurality of cleavaging agents that specifically recognize a plurality of nucleic acid sequences comprising specific sequences, which are not present in normal cells, among variant region sequences specific for cells having genomic sequence variation, or polynucleotides encoding the cleavaging agents; and a nuclease or a polynucleotide encoding the nuclease. To deliver the composition, a single delivery vehicle or different delivery vehicles may be used.

International Patent Publication No. WO2015/191693 discloses a delivery system in which gRNA is provided in a first delivery vehicle and a nucleic acid editing system is provided in a second delivery vehicle. It discloses the delivery system in which the delivery vehicles are simultaneously viral delivery vehicles, or one delivery vehicle is a viral delivery vehicle and the other is a non-viral delivery vehicle, or the delivery vehicles are simultaneously non-viral delivery vehicles. The delivery system is applicable to delivery of the composition according to the present invention and is incorporated herein by reference.

The polynucleotide sequences encoding the plurality of cleavaging agents and/or the polynucleotide sequence encoding the nuclease may be an RNA sequence, a DNA sequence, or a combination thereof (RNA-DNA combination sequence).

In the composition according to the present invention, the cleavaging agent or a polynucleotide encoding the same may comprise the RNA sequence or DNA sequence of the cleavaging agent. In the composition according to the present invention, the nuclease or a polynucleotide encoding the same may comprise a nuclease protein or an RNA sequence or DNA sequence encoding the nuclease protein.

### B. Delivery by vector

A method for delivering the composition according to the present invention may include the DNA sequence of the cleavaging agent and a DNA sequence encoding the nuclease. The DNA sequence of the cleavaging agent and the DNA sequence encoding the nuclease may be provided through a delivery vehicle such as a vector.

In one aspect, the present invention is directed to a vector for inducing apoptosis of cells having genomic sequence variation, the vector comprising: (i) a polynucleotide encoding a nuclease; and (ii) cleavaging agents that respectively recognize at least 4 variant regions specific for the cells having genomic sequence variation in the cells having genomic sequence variation, or polynucleotides encoding the cleavaging agents.

In another aspect, the present invention is directed to a composition for inducing apoptosis of cells having genomic sequence variation, the composition containing: (i) a polynucleotide encoding a nuclease; and (ii) cleavaging agents that respectively recognize at least 4 variant regions specific for the cells having genomic sequence variation in the cells having genomic sequence variation, or polynucleotides encoding the cleavaging agents, wherein (i) and (ii) are contained in the same vector or different vectors.

In one embodiment, (i) a polynucleotide encoding a nuclease; and (ii) cleavaging agents that respectively recognize at least 4 variant regions specific for the cells having genomic sequence variation in the cells having genomic sequence variation, or polynucleotides encoding the cleavaging agents, may be contained in the same vector or different vectors.

### (1) Delivery by individual vectors

Vectors can be designed for expression of nucleic acid transcripts, proteins or enzymes of a plurality of cleavaging agents and nuclease in prokaryotic or eukaryotic cells. The DNA sequence of the cleavaging agent and the DNA sequence encoding the nuclease may be placed in different vectors, respectively, and delivered.

According to the present invention, the DNA sequences may be delivered using viral vectors, for example, Adeno-Associated Viral Vector (AAV), Adenoviral Vector (AdV), Lentiviral Vector (LV) or Retroviral Vector (RV), or other viral vectors, for example, episomal vectors, Simian virus 40 (SV40) ori, bovine papilloma virus (BPV) ori or Epstein-Barr nuclear antigen (EBV) ori.

### (2) Delivery by single vector

The DNA sequences of the cleavaging agents and the DNA sequence encoding the nuclease protein may be located in the same vector and simultaneously delivered by the single vector.

According to the present invention, the DNA sequences may be delivered using viral vectors, for example, Adeno-Associated Viral Vector (AAV), Adenoviral Vector (AdV), Lentiviral Vector (LV) or Retroviral Vector (RV), or other viral vectors, for example, episomal vectors, Simian virus 40 (SV40) ori, bovine papilloma virus (BPV) ori or Epstein-Barr nuclear antigen (EBV) ori.

Editing of multiple genes including multiple cleavaging agents is associated with a crRNA-processing mechanism (Nature Communications volume 11, Article number: 1281 (2020), Nature Biotechnology volume 35, pages31-34 (2017)).

With respect to the type of nuclease, Casl2a/Casl3a can process crRNA without tracrRNA. Since Casl2a/Casl3a possesses nuclease properties, it can remove DRs (direct repeats) that are repetitive sequences required for precursor crRNA (pre-crRNA) processing.

Cas6 requires an accessory protein to form a final effector complex, and the crRNA processed by Cas6 is introduced into a target recognition module consisting of Cas5, Cas6, Cas7 and Cas8, called a cascade. This cascade complex edits DNA by recognizing the PAM of the foreign gene.

Cas9, Cas12b and Cas12c process precursor crRNA (pre-crRNA) through tracrRNA (transactivating crRNA) and RNase III.

In complex gene editing, when Cas9, for example, is applied, the gRNA of Cas9 includes a crRNA:tracrRNA complex, and crRNA:tracrRNA is fused to form sgRNA. Thus, for complex gene editing, a direct repeat, a repetitive sequence required for precursor crRNA (pre-crRNA) processing, is inserted between crRNAs, and tracrRNA is expressed separately, so that RNase III can work according to tracrRNA to edit a plurality of genes while removing the direct repeats.

In some cases, a self-cleaving sequence such as ribozymes may be bound between RNAs, or a Csy4 recognition site, an enzyme that recognizes a 28-nt stem-loop sequence in RNA and removes the nucleotide at position 20, may be bound between RNAs. Alternatively, tRNA that can be processed by endogenous RNase P/RNase Z may be inserted between RNAs, whereby precursor crRNA (pre-crRNA) can be processed into crRNA.

A self-cleaving sequence such as ribozymes may be bound between a plurality of gRNAs. Pol II and Pol III promoters may be applied to process pre-crRNA through transcription mediated by the Pol II and Pol III promoters.

In addition, a Csy4 recognition site, an enzyme that recognizes a 28-nt stem-loop sequence in RNA and removes the nucleotide at position 20, may be bound between gRNAs. When a gRNA containing a Csy4 recognition sequence is co-expressed with Csy4, multiple gRNAs can be expressed and processed by a single promoter.

Furthermore, pre-crRNA can be processed by inserting tRNA that can be processed by endogenous RNase P/RNase Z. RNase P/RNase Z cleaves the 5' and 3' ends of pre-tRNA, and multiple gRNAs can be processed from tRNA-gRNA.

### C. Combination of vector and mRNA, carrier or exosome

A method for delivering the composition according to the present invention may include the DNA sequences of cleavaging agents and a nuclease protein or an RNA sequence encoding the nuclease protein.

The present invention is directed to a composition for inducing apoptosis of cells having genomic sequence variation, the composition comprising: (i) a nuclease or a polynucleotide encoding the same; and (ii) cleavaging agents that respectively recognize at least 4 variant regions specific for the cells having genomic sequence variation in the cells having genomic sequence variation, or polynucleotides encoding the cleavaging agents, wherein (ii) is contained in a vector.

The DNA sequences of the cleavaging agents may be delivered by a vector. The DNA sequences may be delivered using any of the vectors described herein. For example, the DNA sequences may be delivered using viral vectors, for example, Adeno-Associated Viral Vector (AAV), Adenoviral Vector (AdV), Lentiviral Vector (LV) or Retroviral Vector (RV), or other viral vectors, for example, episomal vectors, Simian virus 40 (SV40) ori, bovine papilloma virus (BPV) ori or Epstein-Barr nuclear antigen (EBV) ori.

The RNA sequence encoding the nuclease protein may be delivered in the form of mRNA. Delivery in the form of mRNA may include synthetic modified mRNA or self-replicating RNA (srRNA).

The nuclease protein or the RNA sequence encoding the nuclease protein may comprise a modified nucleoside, or comprise synthetic self-replicative RNA, or may be delivered by the carrier or exosome described herein. For example, the nuclease protein or the RNA sequence encoding the nuclease protein may be delivered by carriers or exosomes, such as nanoparticles, CPP or a polymer.

### D. Delivery by mRNA or carrier

A method for delivering the composition according to the present invention may include the RNA sequences of cleavaging agents and a nuclease protein or an RNA sequence encoding a nuclease protein. The cleavaging agents may be delivered in mRNA form. The nuclease may be delivered in the form of a protein or an mRNA encoding the nuclease protein.

The present invention is directed to a composition for inducing apoptosis of cells having genomic sequence variation, the composition containing: (i) a nuclease or a polynucleotide encoding the same; and (ii) cleavaging agents that respectively recognize at least 4 variant regions specific for the cells having genomic sequence variation in the cells having genomic sequence variation, or polynucleotides encoding the cleavaging agents, wherein (i) and (ii) are contained in a carrier.

The present invention is directed to an exosome for inducing apoptosis of cells having genomic sequence variation, the exosome comprising: (i) a nuclease or a polynucleotide encoding the same; and (ii) cleavaging agents that respectively recognize at least 4 variant regions specific for the cells having genomic sequence variation in the cells having genomic sequence variation, or polynucleotides encoding the cleavaging agents.

The present invention is directed to a composition for inducing apoptosis of cells having genomic sequence variation, the composition containing: (i) a nuclease or a polynucleotide encoding the same; and (ii) cleavaging agents that respectively recognize at least 4 variant regions specific for the cells having genomic sequence variation in the cells having genomic sequence variation, or polynucleotides encoding the cleavaging agents, wherein (i) and (ii) are contained in an exosome.

Delivery in mRNA form may include synthetic modified mRNA or self-replicating RNA (srRNA).

The cleavaging agents may be delivered in mRNA form, and the nuclease protein or the mRNA sequence encoding the nuclease protein may be delivered in a nuclease protein form or in the form of an mRNA encoding the nuclease protein, or delivered by the carrier or exosome described herein, for example, nanoparticles, CPP or a polymer.

### E. Delivery by RNP

mRNAs of the cleavaging agents and the nuclease protein may be delivered in an RNP form so that they can form a complex *in vitro.*

The present invention is directed to a ribonucleoprotein (RNP) complex for inducing apoptosis of cells having genomic sequence variation, the ribonucleoprotein (RNP) complex comprising: (i) a nuclease; and (ii) cleavaging agents that respectively recognize at least 4 variant regions specific for the cells having genomic sequence variation in the cells having genomic sequence variation, or polynucleotides encoding the cleavaging agents.

The present invention is directed to a composition for inducing apoptosis of cells having genomic sequence variation, the composition containing: (i) a nuclease; and (ii) cleavaging agents that respectively recognize at least 4 variant regions specific for the cells having genomic sequence variation in the cells having genomic sequence variation, or polynucleotides encoding the cleavaging agents, wherein (i) and (ii) forms a ribonucleoprotein (RNP) complex.

In some embodiments, the RNP may be delivered by the carrier or exosome described herein, for example, nanoparticles, CPP or a polymer.

### 7. Exonuclease

In a specific example of the present invention, it was confirmed that, when a nuclease in which endonuclease and exonuclease I are fused together was used, the number of cleavaging agents required to induce apoptosis could be reduced.

In another aspect, the present invention is directed to a composition for inducing apoptosis of cells having genomic sequence variation, the composition containing: (i) a nuclease in which endonuclease and exonuclease I are fused together, or a polynucleotide encoding the nuclease; and (ii) cleavaging agents that respectively recognize at least 2 variant regions specific for the cells having genomic sequence variation in the cells having genomic sequence variation, or polynucleotides encoding the cleavaging agents.

The exonuclease may have the ability to cleave nucleotides in the 5' to 3' direction. According to the present invention, it was confirmed that, when Cas9 was used, five gRNAs did not cause apoptosis, but when Exo-Cas9 was used, even 5 gRNAs sufficiently caused apoptosis. Without being bound by theory, it is believed that the reason why even 5 gRNAs sufficiently cause apoptosis is that they inhibit repair after DNA double-strand breaks caused by Cas9.

The exonuclease I may be derived from a microorganism, an enzyme, an insect, a mouse, or a human. Specifically, the exonuclease I may include the sequence of SEQ ID NO: 31.

MMNDGKQQSTFLFHDYETFGTHPALDRPAQFAAIRTDSEFNVI
GEPEVFYCKPADDYLPQPGAVLITGITPQEARAKGENEAAFAA
RIHSLFTVPKTCILGYNNVRFDDEVTRNIFYRNFYDPYAWSWQ
HDNSRWDLLDVMRACYALRPEGINWPENDDGLPSFRLEHLTKA
NGIEHSNAHDAMADVYATIAMAKLVKTRQPRLFDYLFTHRNKH
KLMALIDVPQMKPLVHVSGMFGAWRGNTSWVAPLAWHPENRNA
VIMVDLAGDISPLLELDSDTLRERLYTAKTDLGDNAAVPVKL
VHINKCPVLAQANTLRPEDADRLGINRQHCLDNLKILRENPQV
REKVVAIFAEAEPFTPSDNVDAQLYNGFFSDADRAAMKIVLET
EPRNLPALDITFVDKRIEKLLFNYRARNFPGTLDYAEQQRWLE
HRRQVFTPEFLQGYADELQMLVQQYADDKEKVALLKALWQYAE
EIV

When the nuclease in which endonuclease and exonuclease I are fused together is used, the variant regions specific for the cells having genomic sequence variation may be at least 2 variant regions corresponding to a target sufficient to induce apoptosis. The variant regions specific for the cells having genomic sequence variation may be, for example, 20 or fewer different variant regions. The variant regions specific for the cells having genomic sequence variation may be, for example, 2 to 20 different variant regions.

Specifically, the variant regions specific for the cells having genomic sequence variation may be 2 to 20, 2 to 19, 2 to 18, 2 to 17, 2 to 16, 2 to 15, 2 to 14, 2 to 13, 2 to 12, 2 to 11, 2 to 10, 2 to 9, 2 to 8, 2 to 7, 2 to 6, 2 to 5, 2 to 4, or 2 to 3 different variant regions.

Specifically, the variant regions specific for the cells having genomic sequence variation may be 2, 3, 4, 5, 6, 7, 8, 9, 10, 11, 12, 13, 14, 15, 16, 17, 18, 19, or 20 (also including ranges between the numbers) different variant regions.

Since the genome including the locus where variation exists may be diploid and/or triploid in the cells, in consideration of 2, 3, 4, 5, 6, 7, 8, 9, 10, 11, 12, 13, 14, 15, 16, 17, 18, 19, or 20 (also including ranges between the numbers) pairs or 2, 3, 4, 5, 6, 7, 8, 9, 10, 11, 12, 13, 14, 15, 16, 17, 18, 19, or 20 pairs of variant regions, apoptosis of the cells may be induced by inducing 2 to 40, for example, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3 or 2 (also including ranges between the numbers) double-strand breaks (DSBs).

The variant regions specific for the cells having genomic sequence variation may be nucleic acid sequences, for example, coding or non-coding nucleic acids. The nucleic acids of the variant regions specific for the cells having genomic sequence variation may be coding nucleic acids and may be transcribed from the genome and translated into proteins. The nucleic acids of the variant regions specific for the cells having genomic sequence variation may be non-coding nucleic acids that are not transcribed from the genome and translated into proteins.

Variation in the cells having genomic sequence variation according to the present invention may not be found in normal cells. This variation may be the sequence of a variant region specific for cells having genomic sequence variation, obtained by WGS (whole genome sequencing) of the cells having genomic sequence variation and normal cells. For example, this variation may be at least one selected from the group consisting of insertion, deletion and substitution of a sequence.

Mapping of the sequences specific to the cell having genomic sequence variation may be performed through WGS (whole genome sequencing), or subtractive hybridization and sequencing methods. In the case of detected indels, when insertions are found in cancer, guide RNAs may be prepared immediately, and when deletions are found in cancer, break points may be mapped and then guide RNAs including the break points may be prepared and used.

WGS (whole genome sequencing) refers to a method of reading the genome in various multiples (10X, 20X and 40X formats) by next generation sequencing. "Next-generation sequencing" refers to technology of fragmenting the whole genome in a chip-based and PCR-based paired end format and performing high-throughput sequencing of the fragments on the basis of chemical reaction (hybridization).

Subtractive hybridization is a method used for cloning a gene whose expression differs between various tissues or cells. A DNA sample-specific gene in the cell tested can be detected. The DNA in the cell tested is denatured into single-stranded DNA and then annealed. By controlling the annealing conditions, the DNA sequence specific for the cell tested can be separated as double-stranded DNA.

In the present invention, the plurality of cleavaging agents may comprise a plurality of different sequences, respectively.

The number of the plurality of cleavaging agents having different sequences should be sufficient to induce apoptosis compared to the nucleotide sequences of normal cells, and may be 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, or 20 or more. The number of the plurality of cleavaging agents having different sequences may be 20 or less, 19 or less, 18 or less, 17 or less, 16 or less, 15 or less, 14 or less, 13 or less, 12 or less, 11 or less, 10 or less, 9 or less, 8 or less, 7 or less, 6 or less, 5 or less, 4 or less, 3 or less, or 2 or less.

As the number of the cleavaging agents increases, apoptosis is better achieved, but when the number of the cleavaging agents is about 10, a sufficient apoptotic effect is exhibited. Accordingly, the number of the cleavaging agents may be 2 to 10, preferably 2 to 8, more preferably 3 to 5.

The number of the cleavaging agents having different sequences may be 4 to 20, 4 to 19, 4 to 18, 4 to 17, 4 to 16, 4 to 15, 4 to 14, 4 to 13, 4 to 12, 4 to 11, or 4 to 10.

In the present invention, the number of the cleavaging agents having different sequences may be, for example, 6 to 20, 6 to 19, 6 to 18, 6 to 17, 6 to 16, 6 to 15, 6 to 14, 6 to 13, 6 to 12, 6 to 11, or 6 to 10.

In the present invention, the number of the cleavaging agents having different sequences may be, for example, 8 to 20, 8 to 19, 8 to 18, 8 to 17, 8 to 16, 8 to 15, 8 to 14, 8 to 13, 8 to 12, 8 to 11, or 8 to 10.

In the present invention, the number of the cleavaging agents having different sequences may be, for example, 10 to 20, 10 to 19, 10 to 18, 10 to 17, 10 to 16, 10 to 15, 10 to 14, 10 to 13, or 10 to 12.

In the present invention, the number of the cleavaging agents having different sequences may be, for example, 12 to 20, 12 to 19, 12 to 18, 12 to 17, 12 to 16, 12 to 15, 12 to 14, or 12 to 13.

In the present invention, the number of the cleavaging agents having different sequences may be, for example, 14 to 20, 14 to 19, 14 to 18, 14 to 17, 14 to 16, or 14 to 15.

In the present invention, the number of the cleavaging agents having different sequences may be, for example, 16 to 20, 16 to 19, 16 to 18, or 16 to 17.

In the present invention, the number of the cleavaging agents having different sequences may be, for example, 18 to 20, or 18 to 19.

In the present invention, the number of the cleavaging agents having different sequences may be, for example, 20, 19, 18, 17, 16, 15, 14, 13, 12, 11, 10, 9, 8, 7, 6, 5, or 4 (also including ranges between the numbers). In the present invention, the number of the cleavaging agents having different sequences may be, for example, 20, 19, 18, 17, 16, 15, 14, 13, 12, 11, 10, 9, 8, 7, 6, 5, 4, 3 or 2 (also including ranges between the numbers).

The number of the cleavaging agents having different sequences should be sufficient to induce apoptosis compared to the nucleotide sequences of normal cells, and may be 2 to 20. The number of the cleavaging agents may be 2 to 20, 2 to 19, 2 to 18, 2 to 17, 2 to 16, 2 to 15, 2 to 14, 2 to 13, 2 to 12, 2 to 11, 2 to 10, 2 to 9, 2 to 8, 2 to 7, 2 to 6, 2 to 5, 2 to 4, or 2 to 3.

Depending on whether the chromosome to be targeted by the cleavaging agents is diploid and/or triploid, it may include 2 and/or 3 sequences to be targeted by each cleavaging agent. Thus, the number of double-strand breaks (DSBs) induced by the cleavaging agents may be double and/or triple the number of the cleavaging agents capable of inducing apoptosis. For example, when 5 cleavaging agents having different sequences are contained in the composition, 4 to 6 double-strand breaks (DSBs) are required for apoptosis.

In some embodiments, to increase the efficiency of apoptosis by DNA double-stranded breaks and inhibit DNA double-strand break repair, the composition may further contain, for example, at least one ATM (Ataxia telangiectasia mutated) inhibitor selected from the group consisting of Caffeine, Wortmannin, CP-466722, KU-55933, KU-60019, and KU-559403, at least one ATR (Ataxia telangiectasia and Rad-3 mutated) inhibitor selected from the group consisting of Schisandrin B, NU6027, NVP-BEZ235, VE-821, VE-822 (VX-970), AZ20, and AZD6738, or a DNA double-strand break repair inhibitor of DNA-PKcs (DNA-dependent protein kinase catalytic subunit).

### A. Cargoes

The composition according to the present invention comprises: a plurality of cleavaging agents that specifically recognize at least 2 cell-specific variant regions comprising specific sequences, which are not present in normal cells, among cell-specific variant region sequences in cells having genomic sequence variation, or polynucleotides encoding the cleavaging agents; and a nuclease in which endonuclease and exonuclease I are fused together, or a polynucleotide encoding the nuclease. To deliver the composition, a single delivery vehicle or different delivery vehicles having different configurations may be used.

The polynucleotide sequences encoding the plurality of cleavaging agents and/or the polynucleotide sequence encoding the nuclease may be an RNA sequence, a DNA sequence, or a combination thereof (RNA-DNA combination sequence).

In the composition according to the present invention, the cleavaging agent or a polynucleotide encoding the same may comprise the RNA sequence or DNA sequence of the cleavaging agent. In the composition according to the present invention, the polynucleotide encoding the nuclease in which endonuclease and exonuclease I are fused together may comprise a protein or an RNA sequence or DNA sequence encoding the protein.

### B. Delivery by vector

A method for delivering the composition according to the present invention may include the DNA sequence of the cleavaging agent and a DNA sequence encoding the nuclease in which endonuclease and exonuclease I are fused together. The DNA sequence of the cleavaging agent and the DNA sequence encoding the nuclease in which endonuclease and exonuclease I are fused together may be provided through a delivery vehicle such as a vector.

In one aspect, the present invention is directed to a vector for inducing apoptosis of cells having genomic sequence variation, the vector comprising: (i) a polynucleotide encoding a nuclease in which endonuclease and exonuclease I are fused together; and (ii) cleavaging agents that respectively recognize at least 2 variant regions specific for the cells having genomic sequence variation in the cells having genomic sequence variation, or polynucleotides encoding the cleavaging agents.

In another aspect, the present invention is directed to a composition for inducing apoptosis of cells having genomic sequence variation, the composition containing: (i) a polynucleotide encoding a nuclease in which endonuclease and exonuclease I are fused together; and (ii) polynucleotides encoding cleavaging agents that respectively recognize at least 2 variant regions specific for the cells having genomic sequence variation in the cells having genomic sequence variation, wherein (i) and (ii) are contained in the same vector or different vectors.

In one embodiment, (i) a polynucleotide encoding a nuclease in which endonuclease and exonuclease I are fused together; and (ii) one or more cleavaging agents that recognize at least 2 variant regions specific for the cells having genomic sequence variation, may be contained in the same vector or different vectors.

### (1) Delivery by individual vectors

Vectors can be designed for expression of nucleic acid transcripts, proteins or enzymes of a plurality of cleavaging agents and a nuclease in prokaryotic or eukaryotic cells. The DNA sequence of the cleavaging agent and the DNA sequence encoding the nuclease protein may be placed in different vectors, respectively, and delivered.

According to the present invention, the DNA sequences may be delivered using viral vectors, for example, Adeno-Associated Viral Vector (AAV), Adenoviral Vector (AdV), Lentiviral Vector (LV) or Retroviral Vector (RV), or other viral vectors, for example, episomal vectors, Simian virus 40 (SV40) ori, bovine papilloma virus (BPV) ori or Epstein-Barr nuclear antigen (EBV) ori.

### (2) Delivery by single vector

The DNA sequences of the cleavaging agents and the DNA sequence encoding the nuclease in which endonuclease and exonuclease I are fused together may be located in the same vector and simultaneously delivered by the single vector.

According to the present invention, the DNA sequences may be delivered using viral vectors, for example, Adeno-Associated Viral Vector (AAV), Adenoviral Vector (AdV), Lentiviral Vector (LV) or Retroviral Vector (RV), or other viral vectors, for example, episomal vectors, Simian virus 40 (SV40) ori, bovine papilloma virus (BPV) ori or Epstein-Barr nuclear antigen (EBV) ori.

Editing of multiple genes including a plurality of cleavaging agents is associated with a crRNA-processing mechanism (Nature Communications volume 11, Article number: 1281 (2020), Nature Biotechnology volume 35, pages 31-34 (2017)).

With respect to the type of nuclease, Casl2a/Casl3a can process crRNA without tracrRNA. Since Casl2a/Casl3a possesses nuclease properties, it can remove DRs (direct repeats) that are repetitive sequences required for precursor crRNA (pre-crRNA) processing.

Cas6 requires an accessory protein to form a final effector complex, and the crRNA processed by Cas6 is introduced into a target recognition module consisting of Cas5, Cas6, Cas7 and Cas8, called a cascade. This cascade complex edits DNA by recognizing the PAM of the foreign gene.

Cas9, Cas12b and Cas12c process precursor crRNA (pre-crRNA) through tracrRNA (transactivating crRNA) and RNase III.

In complex gene editing, when Cas9, for example, is applied, the gRNA of Cas9 includes a crRNA:tracrRNA complex, and crRNA:tracrRNA is fused to form sgRNA. Thus, for complex gene editing, a direct repeat, a repetitive sequence required for precursor crRNA (pre-crRNA) processing, is inserted between crRNAs, and tracrRNA is expressed separately, so that RNase III can function according to tracrRNA to edit a plurality of genes while removing the direct repeats.

In some cases, a self-cleaving sequence such as ribozymes may be bound between RNAs, or a Csy4 recognition site, an enzyme that recognizes a 28-nt stem-loop sequence in RNA and removes the nucleotide at position 20, may be bound between RNAs. Alternatively, tRNA that can be processed by endogenous RNase P/RNase Z may be inserted between RNAs, whereby precursor crRNA (pre-crRNA) can be processed into crRNA.

A self-cleaving sequence such as ribozymes may be bound between a plurality of gRNAs. Pol II and Pol III promoters may be applied to process pre-crRNA through transcription mediated by the Pol II and Pol III promoters.

In addition, a Csy4 recognition site, an enzyme that recognizes a 28-nt stem-loop sequence in RNA and removes the nucleotide at position 20, may be bound between gRNAs. When a gRNA containing a Csy4 recognition sequence is co-expressed with Csy4, multiple gRNAs can be expressed and processed by a single promoter.

Furthermore, pre-crRNA can be processed by inserting tRNA that can be processed by endogenous RNase P/RNase Z. RNase P/RNase Z cleaves the 5' and 3' ends of pre-tRNA, and multiple gRNAs can be processed from tRNA-gRNA.

### C. Combination of vector and mRNA, carrier or exosome

A method for delivering the composition according to the present invention may include the DNA sequences of cleavaging agents and a nuclease in which endonuclease and exonuclease I are fused together, or an RNA sequence encoding the nuclease.

The present invention is directed to a composition for inducing apoptosis of cells having genomic sequence variation, the composition comprising: (i) a nuclease in which endonuclease and exonuclease I are fused together, or an RNA sequence encoding the nuclease; and (ii) polynucleotides encoding cleavaging agents that respectively recognize at least 2 variant regions specific for the cells having genomic sequence variation in the cells having genomic sequence variation, wherein (ii) is contained in a vector.

The DNA sequences of the cleavaging agents may be delivered by a vector. The DNA sequences may be delivered using any of the vectors described herein. For example, the DNA sequences may be delivered using viral vectors, for example, Adeno-Associated Viral Vector (AAV), Adenoviral Vector (AdV), Lentiviral Vector (LV) or Retroviral Vector (RV), or other viral vectors, for example, episomal vectors, Simian virus 40 (SV40) ori, bovine papilloma virus (BPV) ori or Epstein-Barr nuclear antigen (EBV) ori.

The RNA sequence encoding the nuclease in which endonuclease and exonuclease I are fused together may be delivered in the form of mRNA. Delivery in the form of mRNA may include synthetic modified mRNA or self-replicating RNA (srRNA).

The nuclease in which endonuclease and exonuclease I are fused together, or the RNA sequence encoding the nuclease, may comprise a modified nucleoside, or comprise synthetic self-replicative RNA, or may be delivered by the carrier or exosome described herein. For example, the nuclease or the RNA sequence may be delivered by carriers or exosomes, such as nanoparticles, CPP or a polymer.

### D. Delivery by mRNA or carrier

A method for delivering the composition according to the present invention may include the RNA sequences of cleavaging agents and a nuclease in which endonuclease and exonuclease I are fused together, or an RNA sequence encoding the nuclease. The cleavaging agents may be delivered in mRNA form. The nuclease in which endonuclease and exonuclease I are fused together may be delivered in the form of a protein or an mRNA encoding the nuclease.

The present invention is directed to a carrier for inducing apoptosis of cells having genomic sequence variation, the carrier containing: (i) a nuclease in which endonuclease and exonuclease I are fused together, or a polynucleotide encoding the nuclease; and (ii) polynucleotides encoding cleavaging agents that respectively recognize at least 2 variant regions specific for the cells having genomic sequence variation in the cells having genomic sequence variation.

The present invention is directed to a composition for inducing apoptosis of cells having genomic sequence variation, the composition containing: (i) a polynucleotide encoding a nuclease in which endonuclease and exonuclease I are fused together; and (ii) polynucleotides encoding cleavaging agents that respectively recognize at least 2 variant regions specific for the cells having genomic sequence variation in the cells having genomic sequence variation, wherein (i) and (ii) are contained in a carrier.

The present invention is directed to an exosome for inducing apoptosis of cells having genomic sequence variation, the exosome containing: (i) a polynucleotide encoding a nuclease in which endonuclease and exonuclease I are fused together; and (ii) polynucleotides encoding cleavaging agents that respectively recognize at least 2 variant regions specific for the cells having genomic sequence variation in the cells having genomic sequence variation.

The present invention is directed to a composition for inducing apoptosis of cells having genomic sequence variation, the composition containing: (i) a polynucleotide encoding a nuclease in which endonuclease and exonuclease I are fused together; and (ii) polynucleotides encoding cleavaging agents that respectively recognize at least 2 variant regions specific for the cells having genomic sequence variation in the cells having genomic sequence variation, wherein (i) and (ii) are contained in an exosome.

Delivery in mRNA form may include synthetic modified mRNA or self-replicating RNA (srRNA).

The cleavaging agents may be delivered in mRNA form, and the nuclease protein or the mRNA sequence encoding the nuclease protein may be delivered in a nuclease protein form or in the form of an mRNA encoding the nuclease protein, or delivered by the carrier or exosome described herein, for example, nanoparticles, CPP or a polymer.

### E. Delivery by RNP

mRNAs of the cleavaging agents and the nuclease protein may be delivered in an RNP form so that they can form a complex *in vitro.*

The present invention is directed to a ribonucleoprotein (RNP) complex for inducing apoptosis of cells having genomic sequence variation, the ribonucleoprotein (RNP) complex comprising: (i) a nuclease in which endonuclease and exonuclease I are fused together; and (ii) polynucleotides encoding cleavaging agents that respectively recognize at least 2 variant regions specific for the cells having genomic sequence variation in the cells having genomic sequence variation.

The present invention is directed to a ribonucleoprotein (RNP) complex for inducing apoptosis of cells having genomic sequence variation, the ribonucleoprotein (RNP) complex comprising: (i) a nuclease in which endonuclease and exonuclease I are fused together; and (ii) polynucleotides encoding cleavaging agents that respectively recognize 2 to 10 variant regions among variant regions in cells having genomic sequence variation, wherein (i) and (ii) form a ribonucleoprotein (RNP) complex.

In some embodiments, the RNP may be delivered by the carrier or exosome described herein, for example, nanoparticles, CPP or a polymer.

According to the present invention, it is possible to manufacture a patient-specific therapeutic drug. As used herein, the term "patient-specific" means that the patient's disease can be effectively treated by sufficiently reflecting the patient's unique characteristics or the characteristics of the patient's disease. The patient-specific composition for treating disease can be effectively used in the selection of a therapeutic agent and a treatment method.

Accordingly, the present invention is directed to a patient-specific composition for treating disease, the composition containing: (i) a nuclease or a polynucleotide encoding the nuclease; and (ii) cleavaging agents that respectively recognize at least 4 variant regions specific for cells having genomic sequence variation in the cells having genomic sequence variation, or polynucleotides encoding the cleavaging agents. The present invention is directed to a patient-specific method for treating disease, the method comprising treating a patient with: (i) a nuclease or a polynucleotide encoding the nuclease; and (ii) cleavaging agents that respectively recognize at least 4 variant regions specific for cells having genomic sequence variation in the cells having genomic sequence variation, or polynucleotides encoding the cleavaging agents.

The present invention is directed to a patient-specific method for treating disease, the method comprising steps of: selecting a plurality of genomic variant sequences different from those in normal cells; and treating a patient with a composition for inducing apoptosis of cells having genomic sequence variation, the composition containing: a nuclease or a polynucleotide encoding the same; and a plurality of cleavaging agents that specifically recognize specifically a plurality of variant regions, respectively, in the cells having genomic sequence variation, or polynucleotides encoding the cleavaging agents.

The present invention is directed to a patient-specific composition for treating disease, the composition containing: (i) a nuclease in which endonuclease and exonuclease I are fused together, or a polynucleotide encoding the nuclease; and (ii) polynucleotides encoding cleavaging agents that respectively recognize at least 2 variant regions specific for cells having genomic sequence variation in the cells having genomic sequence variation. The present invention is directed to a patient-specific method for treating disease, the method comprising treating a patient with: (i) a nuclease in which endonuclease and exonuclease I are fused together, or a polynucleotide encoding the nuclease; and (ii) polynucleotides encoding cleavaging agents that respectively recognize at least 2 variant regions specific for cells having genomic sequence variation in the cells having genomic sequence variation.

The present invention is directed to a patient-specific method for treating disease, the method comprising steps of: selecting a plurality of genomic variant sequences different from those in normal cells; and treating a patient with a composition for inducing apoptosis of cells having genomic sequence variation, the composition containing: a nuclease in which endonuclease and exonuclease I are fused together, or a polynucleotide encoding the nuclease; and a plurality of cleavaging agents that specifically recognize a plurality of variant regions, respectively, in the cells having genomic sequence variation, or polynucleotides encoding the cleavaging agents.

The compositions, methods, and uses of the present invention may be administered to a subject in need thereof in a sufficient or effective amount. The term "effective amount" or "sufficient amount" refers to an amount that provides, in single or multiple doses, alone or in combination, with one or more other therapeutic compositions, or therapeutic regimens agents, an expected or desired outcome in or a benefit to a subject for any duration of time. The effective amount or "sufficient amount" may vary depending on factors such as formulation method, administration mode, the patient's age, weight, sex and pathological condition, administration time, administration route, excretion rate, and responsiveness.

### Examples

Hereinafter, the present invention will be described in more detail with reference to examples. These examples are only for illustrating the present invention, and it will be apparent to those of ordinary skill in the art that the scope of the present invention is not to be construed as being limited by these examples.

### Example 1. Induced apoptosis based on multiple simultaneous DNA DSBs

The present inventors hypothesized that targeting indels would produce high quantities of cancer cell-specific DNA DSBs to kill cancer cells selectively (FIG. 1A). The present inventors tested whether enzymatically induced DSBs would be sufficient to kill proliferating cancer cells, using ER-AsiSI U2OS cells.

U2OS cells with ER-AsiSI (17) were kindly provided as a gift by Dr. Tanya Paull (University of Austin, Texas, USA). ER-AsiSI U2OS cells were cultured in DMEM (GE Healthcare) containing 10% fetal bovine serum. Next, 1 × 10⁵ cells were seeded in a 6-well plate and 4-hydroxytamoxifen (4-OHT) (Cat#. H7904; Merck, St. Louis, MO, USA) was added thereto to a final treatment concentration of 300 nM. 4 hours after 4-OHT treatment, the medium was regenerated and the cells were incubated for an additional 6 days. Cell viability was analyzed through colony formation assay (Cat# M9140; Merck) or Celltiter-Glo assay by methylene blue staining according to the manufacturer's protocol (G9241; Promega, Madison, WI, USA).

ER-AsiSI U2OS cells express the AsiSI restriction enzyme that translocates into the nucleus in the presence of tamoxifen (4OHT) (FIG. 1B). The current version of the human genome (GRCh38) contains 1,225 AsiSI recognition sites. Therefore, it was predicted that about 100 to 1,000 DSBs were likely to occur in this cell type. When AsiSI was translocated to the nucleus after 4OHT treatment, almost all ER-AsiSI U2OS cells were dead compared to U2OS cells treated with 4OHT (FIG. 1B). These data show that many of the DSBs produced by the enzyme can kill proliferating cancer cells.

The present inventors tested whether the DNA DSBs induced by CRISPR-Cas9 could also kill cancer cells.

To construct sgRNAs targeting multiple loci in the human genome, sgRNAs were designed and the number of their targets was validated using the Cas-OFFinder against the human GRCh38 reference genome. Each sgRNA sequence was cloned into an sgRNA expression vector containing a modified mouse U6 promoter from the pMJ179 plasmid (Cat# 85996; Addgene, Watertown, MA, USA). One day before transfection, 1.5 × 104 HEK293T cells were seeded into each well of a 96-well plate. Next, 200 ng of SpCas9 (Cat # 43945; Addgene) and sgRNA expression plasmids were transfected into cells using Lipofectamine 3000 (Cat # L3000015; Thermo Fisher Scientific, Waltham, MA, USA). Three days after transfection, cell viability was measured using CellTiter-Glo Luminescent Cell Viability Assay (Cat # G7570; Promega) according to the manufacturer's instructions. Luminescence was measured using a microplate reader and Gen5 software (BioTek, Winooski, VT, USA) .

Known CRISPR-Cas9 target sequences that appear repetitively present in the human genome were selected (FIGS. 1C and 2). It was observed that, when the number of sgRNAs increased to 10 or more and 20 DSBs were generated in the genome of aneuploid HEK293T cells, the cells began to die. 30 gRNAs were selected as starting points for testing CINDELA-driven cell death in cancer cell lines.

**[Table] Guide RNA targeting multiple loci in the human genome**

| **sgRNA_name** | **sgRNA sequence** | **SEQ ID No.** | **Number of found targets** |
|---|---|---|---|
| NTC_1 | GAAGCCGACGTACACTGAAA | 32 | 0 |
| NTC_4 | GCTCCGATCGGGTTACAACA | 33 | 0 |
| CCR5 | TGACATCAATTATTATACAT | 34 | 1 |
| EMX1-2 | AGGTGTGGTTCCAGAACCGG | 35 | 2 |
| MT10_1 | AACTACACGGAGAGCAAGAG | 36 | 10 |
| MT10_2 | ACGCCATTGTTAATGCACGG | 37 | 10 |
| MT10_3 | GCAACTGGAGCCTCAGATGA | 38 | 10 |
| MT20_1 | ACTCAGGAGGCTGATGTGGA | 39 | 20 |
| MT20_2 | GAACATGTCCGAACATCAGA | 40 | 20 |
| MT20_3 | GGAGACTCATCTAACACATA | 41 | 20 |
| MT30_1 | CCTAGCTACTCGGTAGGCTG | 42 | 30 |
| MT30_2 | TAAGTAGGAATGCCTAGAGT | 43 | 30 |
| MT30_3 | GCAGGCAAGAGAACATGTGC | 44 | 30 |
| MT40_1 | GAACACTTACACACTGCTGG | 45 | 40 |
| MT40_2 | GAGGTTACAGTGAGCTATGA | 46 | 40 |
| MT40_3 | TGTCTTGTGCAGTTGAGATA | 47 | 40 |
| MT50_1 | GCAGAGGCAGGTGGATCATG | 48 | 50 |
| MT50_2 | GCAACCTCTAGAGTCGGCAT | 49 | 50 |
| MT50_3 | GAGAATTGCTTGAATGCAGG | 50 | 50 |
| MT100_1 | AGGTTGCCTGTTGACTCTGA | 51 | 100 |
| MT100_2 | GGAAGAACATTCCATGCTCG | 52 | 100 |
| MT100_3 | GAATAGTGAGTTGTGGAGGA | 53 | 101 |
| MT330 | TGAGACCAGCATGGCCAACA | 54 | 330 |
| MT376 | TACTCAAGCCTCAGTAATGG | 55 | 376 |
| MT1006 | AGAGTGCAGTGGCGCGATCT | 56 | 1006 |
| MT1070 | GAAGTCAAGAATTGAGGTTT | 57 | 1070 |
| MT20K | CAGGAGAATCACTTGAACCT | 58 | 19723 |

To test whether DSBs of specific indels generated by CRISPR-Cas9 selectively kill cancer cells, whole-genome nucleotide sequences of U2OS and HCT116 cells derived from osteosarcoma and colon cancer, respectively, were determined. For comparison, the whole genome of RPE1, a non-tumor cell line immortalized by telomerase expression, was also sequenced. For the indels identified in each cell line, sgRNAs targeting indels were designed. For indels identified in each cell line, sgRNAs targeting indels were designed.

Whole genome sequencing of cell lines and xenograft tissues was performed using an Illumina NovaSeq 6000, which targeted 30× coverage with 150-bp paired-end reads according to the manufacturer's instructions (Illumina, San Diego, CA, USA). After trimming the adapter sequences with Trimmomatic (version 0.39) (31), the reads were mapped against the human genome (GRCh38) using BMA-MEM (version 0.7.17-r1188). The reads were mapped against databases for the human (GRch38) genome and the mouse (mm10) genome. The filtered reads were then preferentially mapped to the human genome for further analysis on xenograft samples. After removing duplicate hits with Samtools (version 1.10), Strelka2 (version 2.9.10) was used with the default setting for calling germline variations.

To design the sgRNAs for cancer cell line-specific indels, the present inventors first filtered the indels that had lengths equal to 3 to 8 bps and designed sgRNAs with those indels for either SpCas9 or SaCas9. The *heterogeneity, allele depth,* and indel availability in either the gnomAD database (version 2.1.1) or other cancer cells the present inventors had previously tested *were also considered when sgRNAs were* prioritized. The present inventors checked the *possibility of off targets using Exonerate* (version 2.4.0) (42) with a two mismatch allowance, and all final candidate targets were visually inspected. All code used in this analysis is available on Github (https://github.com/taejoon/CRISPR-toolbox) .

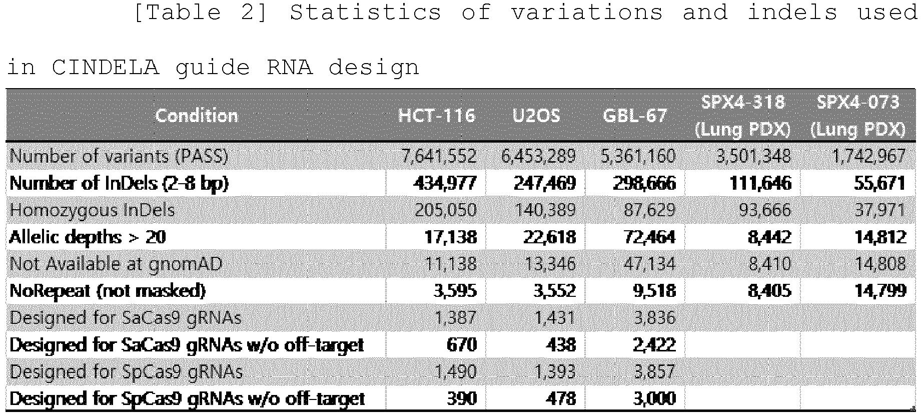

Guide RNA and tracer RNA (Integrated DNA Technologies, Inc., Coralville, IA, USA) targeting cancer-specific indels were used. RNP (ribonucleoprotein) complexes were formed by combining 20 µg equimolar mixture of tracer and gRNAs with 15 µg SpCas9. The RNPs were delivered using Lipofectamine^{™} CRISPRMAX^{™} Cas9 Transfection Reagent (Cat# CMAX00001, Thermo Fisher Scientific) according to the manufacture's protocol.

30 CRISPR-SpCas9RNP complexes were transfected into U2OS cells to determine whether these sgRNAs could induce cell-specific DNA DSBs. Apoptosis of U2OS cells containing the CRISPR-RNP complex targeting U2OS-specific indels was observed 72 hours after transfection. Apoptosis was not observed in U2OS cells transfected with the CRISPR-RNP complex targeting HCT116-specific indels (FIG. 1D). When the number of gRNAs for RNP delivery was reduced from 30 to 18, 12 or 6, apoptosis was observed in a dose-dependent manner. (FIG. 2).

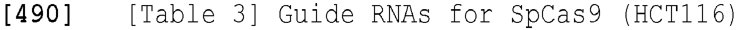

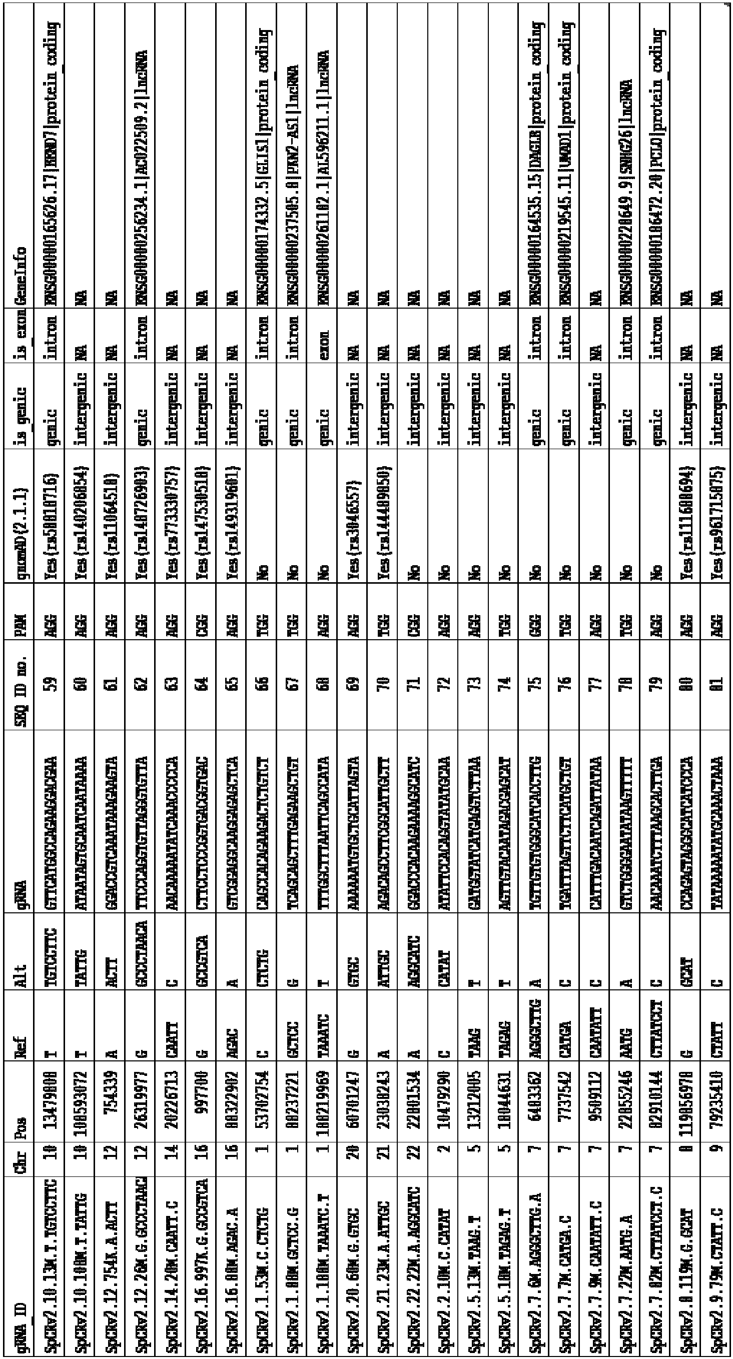

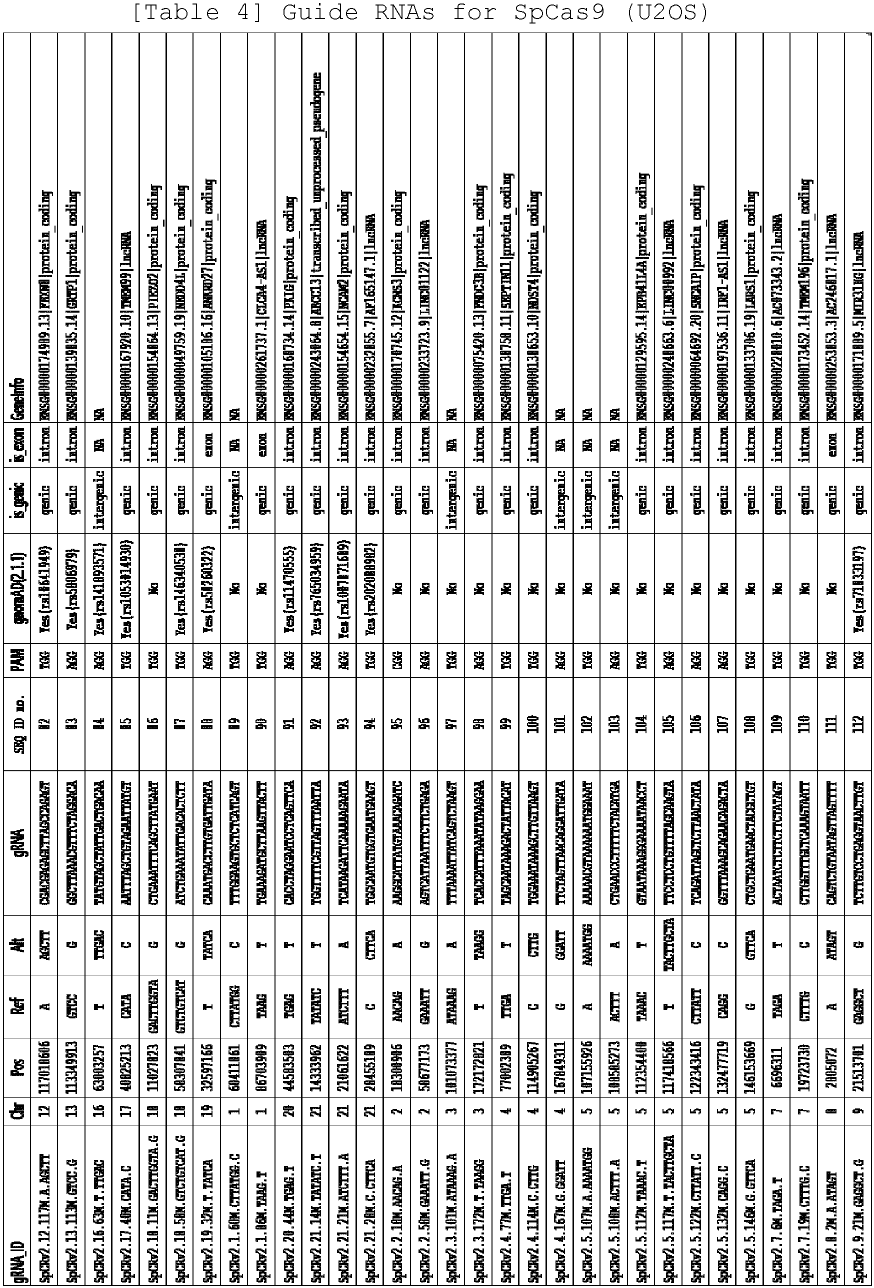

### Induced cancer-specific apoptosis (cell death) upon treatment with CINDELA treatment with AAV-SaCas9

The AAV plasmid Addgene (#61591) expressing CRISPR/SaCas9 under the CMV promoter was prepared, and AAV virus particles were harvested day 3 after transfection into HEK293T cells and concentrated (Addgene, AAV Production in HEK293T Cells). AAV serotype 2 was used for viral packaging and 2.81 × 10¹⁰ viral particles were transduced into target cells for CINDELA.

SaCas9 used for AAV delivery was tagged with 3xHA and detected with a monoclonal anti-HA antibody (Cat# H3663; Sigma-Aldrich, St. Louis, MO). Cells with DNA damage were analyzed using an anti-phosphorylation-histone H2A.X antibody that detects γH2AX (Cat# 05-636; Sigma-Aldrich). Apoptotic cell death was quantified using a BD FACSVerse instrument equipped with Annexin V Alexa Fluor^{™} 488 conjugate (Cat#. A13201; Thermo Fisher Scientific) and Flow-Jo software (version 10) according to the manufacturer's instructions.

As delivery of RNP complexes to the tumor environment *in vivo* is not straightforward, the present inventors tested whether Adeno-Associated Virus (AAV)-based SaCas9 could be used for CINDELA treatment. Through plasmid transfection, the present inventors transfected a plasmid containing two different sets of CINDELA sgRNAs expressing the SaCas9 protein, one targeting U2OS indels and the other targeting HCT116 indels, and observed the effect of U2OSCINDELA. Similar to RNP complex delivery, U2OS cells were dead only when sgRNA targeting U2OS indels was co-expressed with SaCas9 (FIG. 3A). The increase in annexin V signal after CRISPR-RNP transfection showed that cells were killed by apoptosis (FIG. 3B). For *in vivo* CINDELA testing, the present inventors produced AAVs that coexpressed both sgRNAs targeting cancerspecific indels and SaCas9. A total of *30 AAVs coexpressing* different sgRNAs and SaCas9 were transduced into U2OS cells and RPE1 cells. Transduction of AAV was confirmed by Western blot for SaCas9 expression.

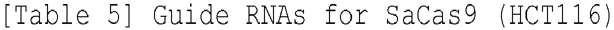

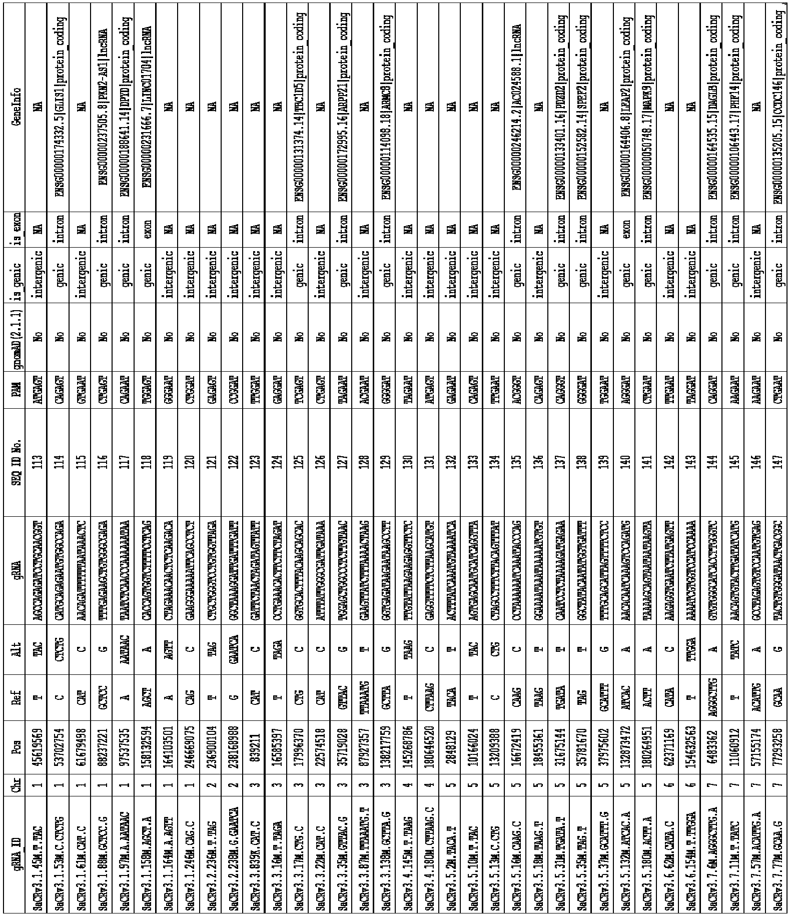

Selective U2OS cell death caused by AAVs targeting *U2OS-specific indels* started 2 days after transduction (FIGS. 3C, 4A and 4B). In contrast, RPE1 cells were not affected. A strong γH2AX signal was observed only in U2OS cells targeted by CRISPR-Cas9, and thus DSBs were present selectively in the target cell line (FIG. 4C). Similar to the transfection approach, AAVs transduced with CINDELA also caused apoptosis in a time-dependent manner (FIG. 4D).

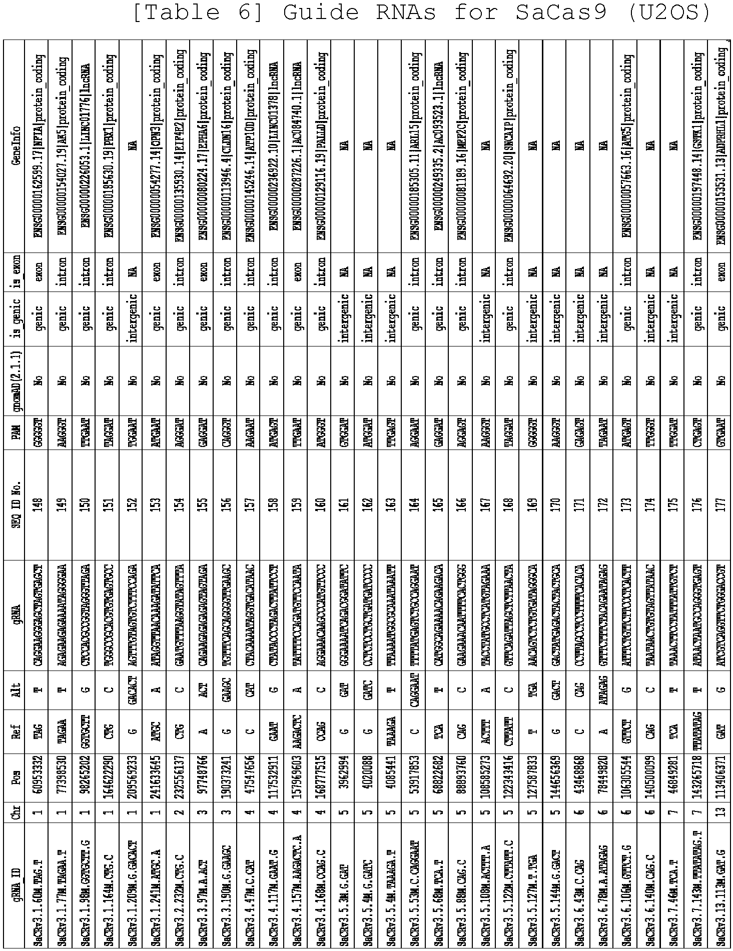

To identify the minimum number of CINDELA targets required for each cell, the number of sgRNAs delivered by AAV was systematically reduced. U2OS cell death induced by CINDELA required 5 or more sgRNAs along with SaCas9 protein expression (FIG. 4D). Since some chromosomes in U2OS cells are diploid or triploid, the U2OS genome carries two or more DNA sequences targeted by each guide RNA, and 10 to 15 DSBs are required for cell death. It was consistently observed that CINDELA-induced cell death increased with an increase in the number of targeting sgRNAs (FIG. 4E). No apparent cell death occurred when one or three AAVs were transduced into U2OS cells.

### Example 3. Inhibited growth of in vivo xenograft model tumor upon CINDELA treatment

NOD-SCID mice were bred under standard conditions. All animal experiments were conducted using protocols approved by the Institutional Animal Care and Use Committee of the UNIST (IACUC-19-05). Briefly, 6-week-old mice were subcutaneously injected in one flank with 2 × 10⁶ cancer cells. When the size of the tumor reached about 1 cm, AAV particles were injected 4 times at 3-day intervals. 2 weeks after the first AAV injection, tumors were collected and analyzed. TUNEL assay was performed using Promega DeadEnd^{™} Colorimetric TUNEL System (Cat#G7360, Promega). PDX experiments were conducted by DNAlink, Inc. using the method known in PDX models of human lung squamous cell carcinoma: consideration of factors in preclinical and co-clinical applications. J. Transl. Med 18, 307 (2020).

The present inventors tested whether CINDELA could inhibit tumor growth *in vivo.* U2OS cell xenograft model was prepared in NOD-SCID mice and monitored until the tumor size reached approximately 1 cm. Next, U2OS-specific CINDELA sgRNAs and AAVs expressing SaCas9 or AAVs expressing only SaCas9 were injected into mice 5 times at 3-day intervals for 2 weeks. As shown in FIG. 5A, it was observed that tumor growth was selectively inhibited by injection of U2OS-specific CINDELA sgRNAs and SaCas9-expressing AAVs. The HA tag of SaCas9 was detected in SaCas9-injected mice and SaCas9 tumor sections containing sgRNA (FIG. 6A). Mice did not show any significant symptoms during the AAV treatment period, suggesting that CINDELA delivered by AAV did not affect normal tissues and cells (FIG. 6B). To confirm the specific apoptosis pathway induced by CINDELA treatment, the remaining tumors were collected and stained with γH2AX or TUNEL. As expected, CINDELA-targeted tumors exhibited significant induction of γH2AX and TUNEL signals in addition to apoptotic cell death (FIG. 5C) compared to control tumors (FIGS. 5A and 5B).

### Example 4. CINDELA Treatment selectively kills patient-derived primary tumors and PDX

The present inventors used the Korean glioblastoma patient-derived tumor cell line GBL-67 to prove whether CINDELA treatment can be applied to actual tumor samples. Based on whole genome sequence data, the present inventors designed 26 SaCas9 CINDELA guide RNAs that specifically targeted GBL-67 InDels. Since the present inventors did not have access to normal cells from the same individual, NSC-10 neural stem cells without targeted InDels were used as a control. AAVs expressing sgRNA and SaCas9 were generated and simultaneously transduced into primary glioblastoma GBL-67 and NSC-10 cells. It was confirmed that about 50 cellspecific DSBs were induced by CINDELA treatment and could sufficiently kill glioblastoma cells (FIG. 7A). In contrast, the growth of NSC-10 cells after transduction was not inhibited.

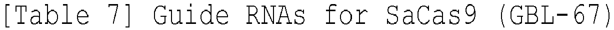

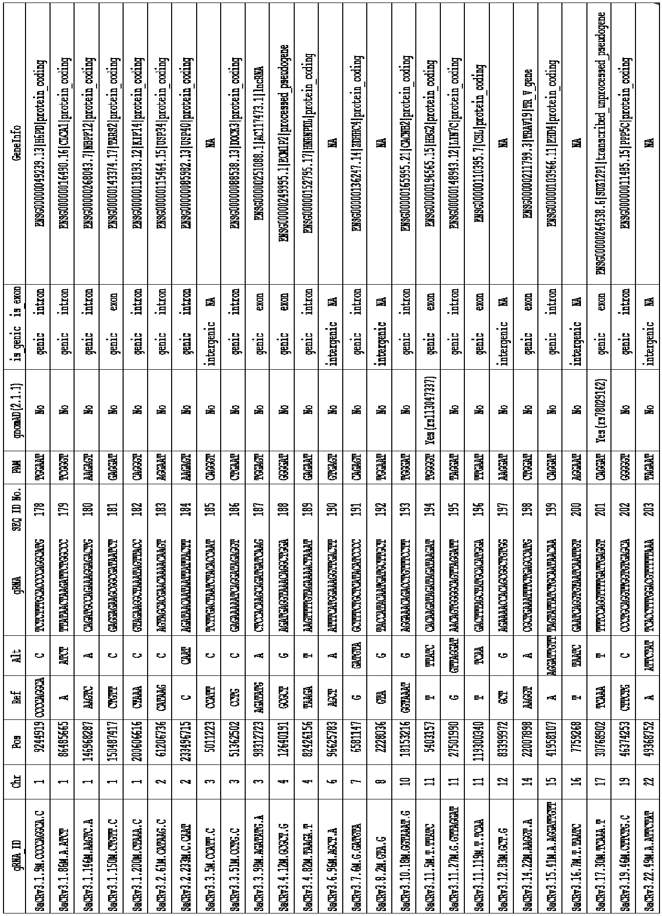

Finally, the present inventors tested the effectiveness of CINDELA in a patient-derived xenografts (PDX), a predictive preclinical model for cancer treatment. After sequencing tissue from an established lung cancer PDX, the present inventors identified about 30 CINDELA targets (29 gRNAs for SPX4-073, and 22 gRNAs for SPX4-318). After injecting AAVs expressing SaCas9 together with the targeting sgRNA 5 times at 3-day intervals, the tumor size of the xenograft model was monitored for 2 weeks. PDX tumor growth was inhibited by the AAV expressing sgRNA and SaCas9. In contrast, the AAV expressing only SaCas9 did not inhibit tumor growth (FIGS. 7B, 8A and 8B). Mice showed no significant symptoms during the AAV treatment period (FIG. 8C) .

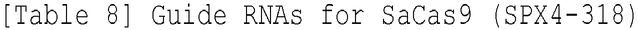

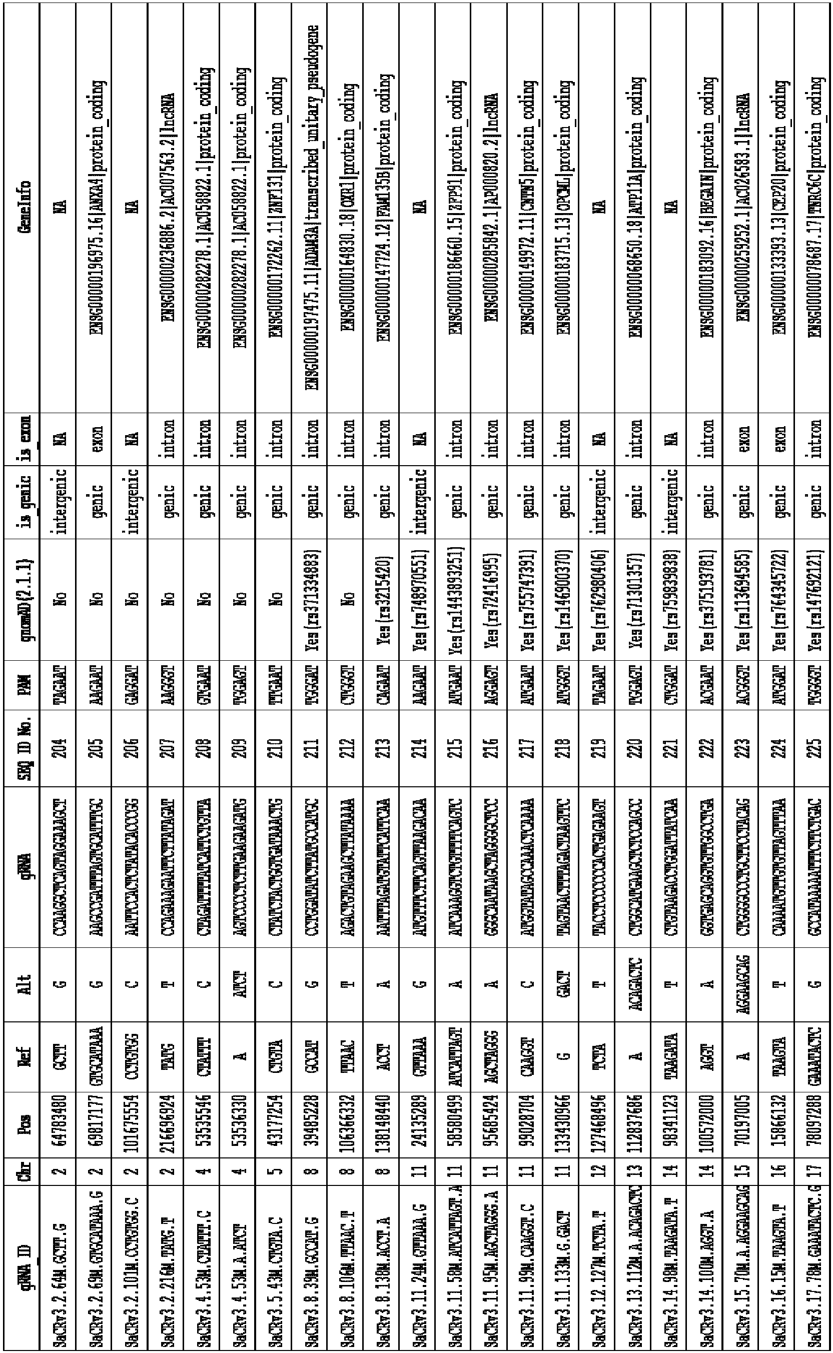

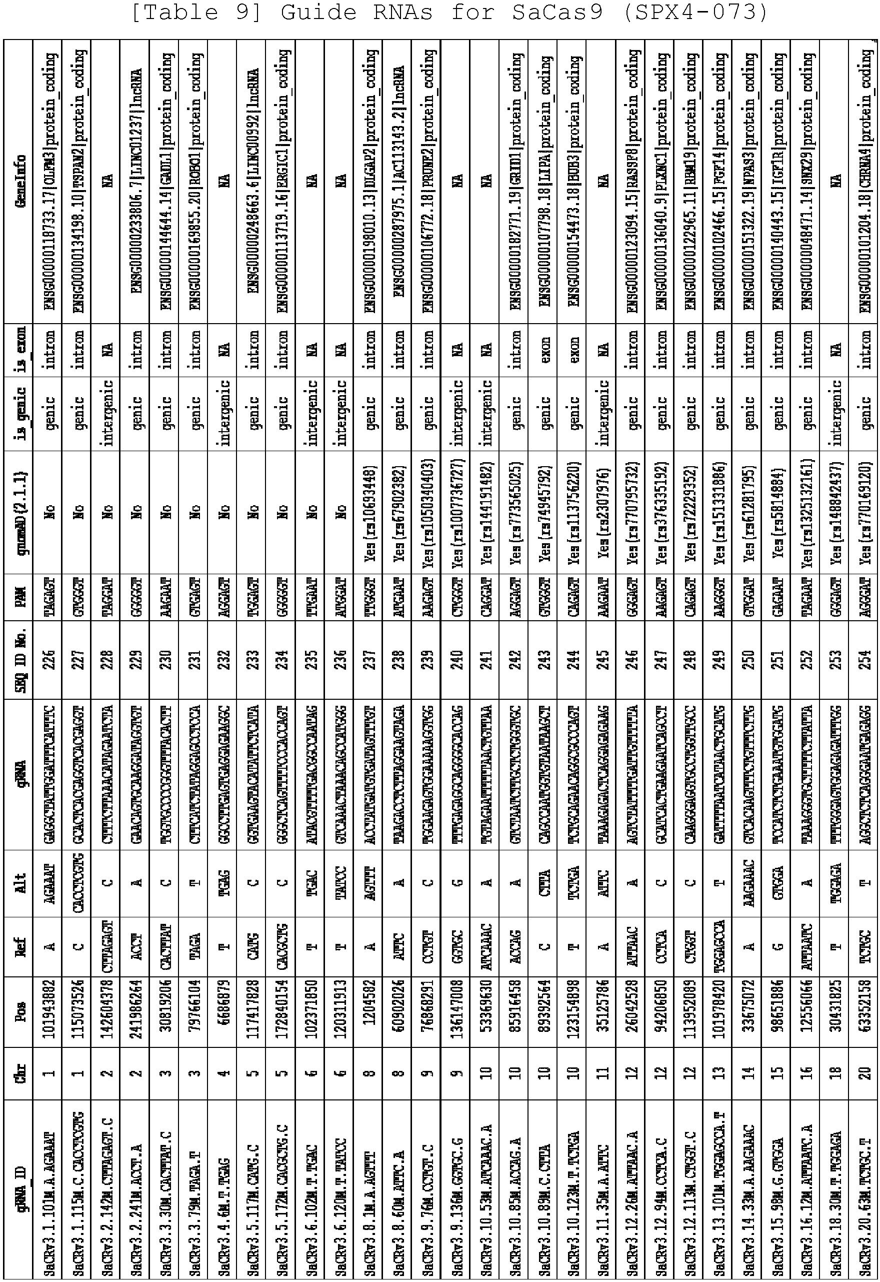

### Example 5. Induction of selective cell death by targeting chromosome 5 through AAV transduction

### 5-1. 30 CINDELA with saCas9

After identifying 30 sequences that can selectively target osteosarcoma cells by WGS (whole genome sequencing), each sequence was added to an AAV-saCAS9 vector (pX601-AAV-CMV::NLS-SaCas9-NLS-3xHA), and then a total of 6 AAV particle groups, each targeting 5 sequences, were prepared using HEC293T cells. The prepared AAV particles were quantified by qPCR and then transduced into normal RPE1 cells and U2OS osteosarcoma cells. It was confirmed that cell death occurred selectively performed only in osteosarcoma cells after 96 hours.

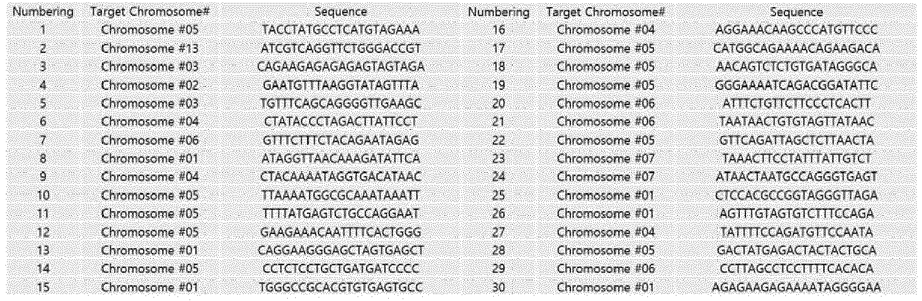

It was confirmed that it was possible to selectively kill osteosarcoma cells by targeting 30 DNA sequences in U2OS cells (FIG. 9).

### 5-2. 10 CINDELA with saCas9

The present inventors examined whether the use of 10 sequences capable of being the target regions of chromosome 5, among 30 sequences capable of being the target regions of osteosarcoma cells, could induce cell death, in the same manner as in Example 5-1, except that two AAV particle groups, each targeting 5 sequences, were prepared.

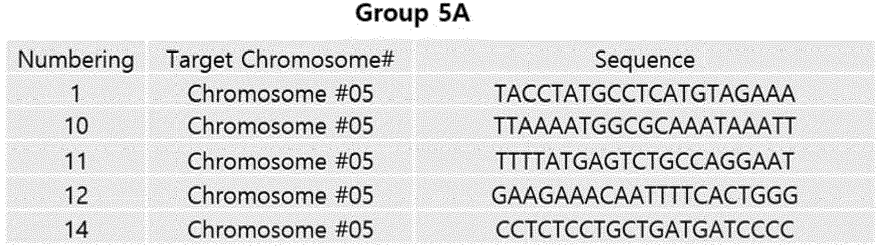

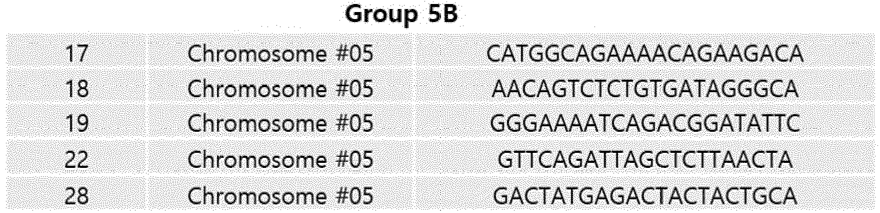

It was confirmed that it was possible to selectively kill osteosarcoma cells by targeting 10 DNA sequences in U2OS cells. It was confirmed that targeting the 10 DNA sequences could induce cell death in the same manner as when targeting the 30 sequences (FIG. 10).

### 5-3. 5 CINDELA with saCas9

The present inventors examined whether cell death could be induced by only one group (targeting five DNA sequences; group 5A).

It was confirmed that it was possible to selectively kill osteosarcoma cells by targeting 5 DNA sequences in U2OS cells. It was confirmed that, compared to when the 30 or 10 sequences were targeted, the proportion of living cells slightly increased, but most of the cells were killed (FIG. 11) .

### 5-4. 5 CINDELA with saCas9

The present inventors examined whether cell death could be induced by one group (group 5B), which differs from that of Example 5-3 and is capable of targeting chromosome 5.

It was confirmed that it was possible to selectively kill osteosarcoma cells by targeting 5 DNA sequences, which differ from those of Example 5-3, in U2OS cells. When compared to the time of targeting 30 or 10 sequences, it was confirmed that the ratio of living cells slightly increased, but most of the cells were killed (FIG. 12). It was confirmed that, compared to when the 30 or 10 sequences were targeted, the proportion of living cells slightly increased, but most of the cells were killed (FIG. 12).

### 5-5. 3 CINDELA with saCas9

The present inventors randomly selected 3 sequences from the 10 sequences capable of being the target regions of chromosome 5, and prepared one AAV particle group targeting the selected sequences, and then examined whether cell death could be induced by the prepared AAV group.

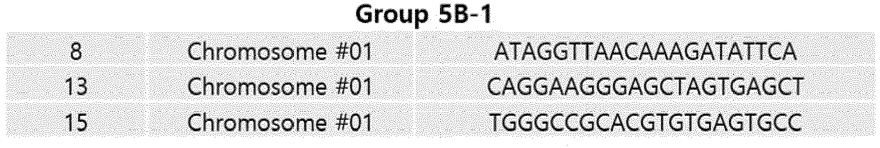

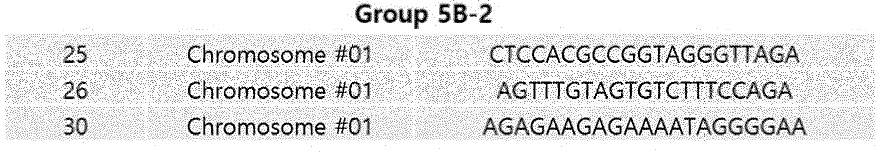

It was confirmed that cell death was not achieved even though an attempt was made to selectively kill osteosarcoma cells by targeting the 3 DNA sequences in U2OS cells (FIG. 13).

### Example 6. Induction of selective cell death by targeting chromosome 5

### 6-1. HCT116 transfection with five gRNAs targeting chromosome 3

Exo-saCas9 recombinant protein was produced by binding *E. coli* exonuclease to saCas9 protein. 12 hours before transfection, HCT116 cells (6 × 10⁵/6-well plate) were prepared. The cells were transfected with 5 gRNAs targeting choromosome3 and saCas9 or Exo-Cas9 using lipofectamine3000, and after 48 hours, cell death was observed using a microscope.

The experiment was conducted by transfecting the cells with five gRNAs targeting chromosome 3. It was confirmed that, when saCas9 was used alone, cell death was not induced by the five gRNAs, but when Exo-saCas9 was used, even the five gRNAs sufficiently induced cell death (FIG. 15) .

### 6-2. U2OS transfection with five gRNA targeting chromosome 1

12 hours before transfection, U2OS cells (3 × 10⁵/6-well plate) were prepared. The cells were transfected with 5 gRNAs targeting U2OS choromosome1 and saCas9 or Exo-Cas9 using lipofectamine3000, and after 48 hours, cell death was observed using a microscope.

The experiment was conducted by transfecting the cells with five gRNAs targeting chromosome 1. It was confirmed that, when saCas9 was used alone, cell death was not induced by the five gRNAs, but when Exo-saCas9 was used, even the five gRNAs sufficiently induced cell death (FIG. 16) .

### 6-3. Examination of repair inhibition

Cells transfected as described in Examples 6-1 and 6-2 were harvested using PBS after 48 hours. The cells were centrifuged at 5,000 rpm for 5 minutes, the supernatant was discarded, and the pellet was treated with 200 µl of buffer A (buffer for removal of cell membrane and nuclear membrane) . The resulting cell solution was centrifuged at 5,000 rpm for 5 min, and the supernatant was discarded. The pellet was resuspended in 50 µl of RIPA buffer and then sonicated at 50 amp for 1 min. The resulting suspension was centrifuged at 13,200 rpm for 25 min, and then the supernatant was quantified, separated by SDS-PAGE gel electrophoresis and subjected to Western blotting using g-H2AX antibody.

It was confirmed that, when the Exo-Cas9 shown in FIG. 14 was used, the expression level of g-H2AX, a DNA damage marker, was high (FIG. 17). It is believed that the reason why cell death was better induced upon the use of Exo-Cas9 is that it inhibited the repair of DNA double-strand breaks caused by saCas9.

### 6-4. DR-GFP assay

U2OS cells expressing DR-GFP were transfected with either saCas9 or exo-saCas9 and DR-GFP-specific gRNAs by using lipofectamine3000 and harvested after 72 hours. The GFP expression level in the harvested cells was measured using FACS (fluorescence-activated cell sorting).

As a result of analyzing the level of homologous recombination (HR) by DR-GFP assay, it was confirmed that HR significantly decreased when Exo-Cas9 was used. This demonstrated that Exo-Cas9 could inhibit HR (FIG. 18).

Although the present invention has been described in detail with reference to specific features, it will be apparent to those skilled in the art that this description is only of a preferred embodiment thereof, and does not limit the scope of the present invention. Thus, the substantial scope of the present invention will be defined by the appended claims and equivalents thereto.

### Sequence List Free Text

Electronic file attached

## Claims

1. A composition for inducing apoptosis of cells having genomic sequence variation, the composition comprising:
(i) a nuclease or a polynucleotide encoding the nuclease; and
(ii) cleavaging agents or polynucleotides encoding the cleavaging agents that recognize at least 4 variant regions respectively specific for the cells having genomic sequence variation in the cells having genomic sequence variation.

2. The composition of claim 1, wherein the cells having genomic sequence variation are cancer cells, senescent cells, immune disease-causing cells, or cardiovascular disease-causing cells.

3. A vector for inducing apoptosis of cells having genomic sequence variation, the vector containing:
(i) a polynucleotide encoding a nuclease; and
(ii) cleavaging agents or polynucleotides encoding the cleavaging agents that recognize at least 4 variant regions respectively specific for the cells having genomic sequence variation in the cells having genomic sequence variation.

4. The vector of claim 3, wherein (i) and (ii) are contained in the same vector or different vectors.

5. The vector of claim 3, wherein the vector comprises:
a viral vector selected from the group consisting of an adeno-associated viral vector (AAV), an adenoviral vector (AdV), a lentiviral vector (LV), and a retroviral vector (RV);
or an episomal vector containing a viral replicon.

6. The vector of claim 5, wherein the episomal vector comprises a Simian virus 40 (SV40), bovine papilloma virus (BPV) or Epstein-Barr nuclear antigen (EBV) replication origin (Ori).

7. A ribonucleoprotein (RNP) complex for inducing apoptosis of cells having genomic sequence variation, the complex comprising:
(i) a nuclease; and
(ii) polynucleotides encoding cleavaging agents that respectively recognize at least 4 variant regions specific for the cells having genomic sequence variation in the cells having genomic sequence variation.

8. A carrier for inducing apoptosis of cells having genomic sequence variation, the carrier containing:
(i) a nuclease or a polynucleotide encoding the nuclease; and
(ii) polynucleotides encoding cleavaging agents that respectively recognize at least 4 variant regions specific for the cells having genomic sequence variation in the cells having genomic sequence variation.

9. An exosome for inducing apoptosis of cells having genomic sequence variation, the exosome containing:
(i) a nuclease or a polynucleotide encoding the nuclease; and
(ii) polynucleotides encoding cleavaging agents that respectively recognize at least 4 variant regions specific for the cells having genomic sequence variation in the cells having genomic sequence variation, or polynucleotides encoding the cleavaging agents.

10. A composition for inducing apoptosis of cells having genomic sequence variation, the composition containing:
(i) a polynucleotide encoding a nuclease; and
(ii) polynucleotides encoding cleavaging agents that respectively recognize at least 4 variant regions specific for the cells having genomic sequence variation in the cells having genomic sequence variation,
wherein (i) and (ii) are contained in the same vector or different vectors.

11. A composition for inducing apoptosis of cells having genomic sequence variation, the composition containing:
(i) a nuclease or a polynucleotide encoding the nuclease; and
(ii) polynucleotides encoding cleavaging agents that respectively recognize at least 4 variant regions specific for the cells having genomic sequence variation in the cells having genomic sequence variation,
wherein (ii) is contained in a vector.

12. The composition of claim 11, wherein the polynucleotide encoding the nuclease (i) is an RNA encoding the nuclease, and optionally comprises a modified nucleoside or comprises synthetic self-replicative RNA; or
the polynucleotide encoding the nuclease (i) is contained in a carrier or an exosome.

13. A composition for inducing apoptosis of cells having genomic sequence variation, the composition containing:
(i) a nuclease; and
(ii) polynucleotides encoding cleavaging agents that respectively recognize at least 4 variant regions specific for the cells having genomic sequence variation in the cells having genomic sequence variation,
wherein (i) and (ii) form a ribonucleoprotein (RNP) complex.

14. A composition for inducing apoptosis of cells having genomic sequence variation, the composition containing:
(i) a nuclease or a polynucleotide encoding the nuclease; and
(ii) polynucleotides encoding cleavaging agents that respectively recognize at least 4 variant regions specific for the cells having genomic sequence variation in the cells having genomic sequence variation,
wherein (i) and (ii) are contained in a carrier.

15. The composition of claim 14, wherein the carrier comprises a cell penetrating peptide (CPP), nanoparticles, zeolitic imidazole frameworks (ZIFs), RNA aptamerstreptavidin, or a polymer.

16. A composition for inducing apoptosis of cells having genomic sequence variation, the composition containing:
(i) a nuclease or a polynucleotide encoding the nuclease; and
(ii) polynucleotides encoding cleavaging agents that respectively recognize at least 4 variant regions specific for the cells having genomic sequence variation in the cells having genomic sequence variation,
wherein (i) and (ii) are contained in an exosome.

17. The composition of any one of claims 9 to 16, containing at least 4 cleavaging agents having different sequences.

18. The composition of any one of claims 9 to 16, containing cleavaging agents that specifically recognize 4 to 30 variant regions in the cells having genomic sequence variation.

19. The composition of any one of claims 9 to 16, containing 4 to 30 cleavaging agents having different sequences.

20. The composition of any one of claims 9 to 16, which kills the cells by inducing double-stranded breaks (DSBs) in 4 to 60 nucleic acid regions comprising an insertion/deletion specific for the cells having genomic sequence variation.

21. The composition of any one of claims 9 to 16, wherein the nuclease is Cas1, Cas1B, Cas2, Cas3, Cas4, Cas5, Cas6, Cas7, Cas8, Cas9, Cas10, Cas12a, Cas12b, Cas12c, Cas12d, Cas12e, Cas12g, Cas12h, Cas12i, Cas12j, Cas13a, Cas13b, Cas13c, Cas13d, Cas14, Csy1, Csy2, Csy3, Cse1, Cse2, Csc1, Csc2, Csa5, Csn2, CsMT2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3 or Csf4.

22. A patient-specific composition for treating disease, the composition containing:
(i) a nuclease or a polynucleotide encoding the nuclease; and
(ii) polynucleotides encoding cleavaging agents that respectively recognize at least 4 variant regions specific for the cells having genomic sequence variation in the cells having genomic sequence variation.

23. A composition for inducing apoptosis of cells having genomic sequence variation, the composition containing:
(i) a nuclease in which endonuclease and exonuclease I are fused together, or a polynucleotide encoding the nuclease; and
(ii) cleavaging agents or polynucleotides encoding the cleavaging agents that recognize at least 2 variant regions respectively specific for the cells having genomic sequence variation in the cells having genomic sequence variation.

24. A vector for inducing apoptosis of cells having genomic sequence variation, the vector containing:
(i) a polynucleotide encoding a nuclease in which endonuclease and exonuclease I are fused together; and
(ii) polynucleotides encoding cleavaging agents that recognize at least 2 variant regions respectively specific for the cells having genomic sequence variation in the cells having genomic sequence variation.

25. The vector of claim 24, wherein (i) and (ii) are contained in the same vector or different vectors.

26. The vector of claim 25, which comprises: a viral vector selected from the group consisting of an adeno-associated viral vector (AAV), an adenoviral vector (AdV), a lentiviral vector (LV), and a retroviral vector (RV); or an episomal vector containing a viral replicon.

27. The vector of claim 26, wherein the episomal vector comprises a Simian virus 40 (SV40), bovine papilloma virus (BPV) or Epstein-Barr nuclear antigen (EBV) replication origin (Ori).

28. A ribonucleoprotein (RNP) complex for inducing apoptosis of cells having genomic sequence variation, the complex comprising:
(i) a nuclease in which endonuclease and exonuclease I are fused together; and
(ii) polynucleotides encoding cleavaging agents that respectively recognize at least 2 variant regions specific for the cells having genomic sequence variation in the cells having genomic sequence variation.

29. A carrier for inducing apoptosis of cells having genomic sequence variation, the carrier containing:
(i) a nuclease in which endonuclease and exonuclease I are fused together, or a polynucleotide encoding the nuclease; and
(ii) polynucleotides encoding cleavaging agents that respectively recognize at least 2 variant regions specific for the cells having genomic sequence variation in the cells having genomic sequence variation.

30. An exosome for inducing apoptosis of cells having genomic sequence variation, the exosome containing:
(i) a nuclease in which endonuclease and exonuclease I are fused together, or a polynucleotide encoding the nuclease; and
(ii) polynucleotides encoding cleavaging agents that respectively recognize at least 2 variant regions specific for the cells having genomic sequence variation in the cells having genomic sequence variation.

31. A composition for inducing apoptosis of cells having genomic sequence variation, the composition containing:
(i) a polynucleotide encoding a nuclease in which endonuclease and exonuclease I are fused together; and
(ii) polynucleotides encoding cleavaging agents that respectively recognize at least 2 variant regions specific for the cells having genomic sequence variation in the cells having genomic sequence variation,
wherein (i) and (ii) are contained in the same vector or different vectors.

32. A composition for inducing apoptosis of cells having genomic sequence variation, the composition containing:
(i) a nuclease in which endonuclease and exonuclease I are fused together, or a polynucleotide encoding the nuclease; and
(ii) polynucleotides encoding cleavaging agents that respectively recognize at least 2 variant regions specific for the cells having genomic sequence variation in the cells having genomic sequence variation,
wherein (ii) is contained in a vector.

33. The composition of claim 32, wherein the nuclease or the polynucleotide encoding the nuclease (i) is an RNA encoding the nuclease, and optionally comprises a modified nucleoside or comprises synthetic self-replicative RNA; or
the nuclease or the polynucleotide encoding the nuclease (i) is contained in a carrier or an exosome.

34. A composition for inducing apoptosis of cells having genomic sequence variation, the composition containing:
(i) a nuclease in which endonuclease and exonuclease I are fused together; and
(ii) polynucleotides encoding cleavaging agents that respectively recognize at least 2 variant regions specific for the cells having genomic sequence variation in the cells having genomic sequence variation,
wherein (i) and (ii) form a ribonucleoprotein (RNP) complex.

35. A composition for inducing apoptosis of cells having genomic sequence variation, the composition containing:
(i) a nuclease in which endonuclease and exonuclease I are fused together, or a polynucleotide encoding the nuclease; and
(ii) polynucleotides encoding cleavaging agents that respectively recognize at least 2 variant regions specific for the cells having genomic sequence variation in the cells having genomic sequence variation, or polynucleotides encoding the cleavaging agents,
wherein (i) and (ii) are contained in a carrier.

36. The composition of claim 35, wherein the carrier comprises a cell penetrating peptide (CPP), nanoparticles, zeolitic imidazole frameworks (ZIFs), RNA aptamerstreptavidin, or a polymer.

37. A composition for inducing apoptosis of cells having genomic sequence variation, the composition containing:
(i) a nuclease in which endonuclease and exonuclease I are fused together, or a polynucleotide encoding the nuclease; and
(ii) polynucleotides encoding cleavaging agents that respectively recognize at least 2 variant regions specific for the cells having genomic sequence variation in the cells having genomic sequence variation,
wherein (i) and (ii) is contained in an exosome.

38. The composition of any one of claims 28 to 37, containing cleavaging agents that specifically recognize 2 to 10 variant regions in the cells having genomic sequence variation.

39. The composition of claim 38, containing at least 2 cleavaging agents having different sequences.

40. The composition of claim 38, containing 2 to 10 cleavaging agents having different sequences.

41. The composition of any one of claims 28 to 37, wherein the exonuclease I is derived from a microorganism, an enzyme, an insect, a mouse, or a human.

42. The composition of claim 41, wherein the exonuclease I comprises the sequence of SEQ ID NO: 31.

43. A patient-specific composition for treating disease, the composition containing:
(i) a nuclease in which endonuclease and exonuclease I are fused together, or a polynucleotide encoding the nuclease; and
(ii) cleavaging agents or polynucleotides encoding the cleavaging agents that recognize at least 2 variant regions respectively specific for the cells having genomic sequence variation in the cells having genomic sequence variation.
